# EUROPEAN PATENT APPLICATION

(11) **EP 4 434 980 A1**
(43) Date of publication of application: **25.09.2024**
(21) Application number: 22895006.9
(22) Date of filing: 22.11.2022
(51) Int. Cl.: C07D 401/14, C07D 413/14, C07D 417/14, C07D 471/04, C07D 471/10, C07D 487/04, C07D 487/10, A61K 31/497, A61K 31/5377, A61K 31/4427, A61P 35/00, A61P 37/00

(54) **MULTIFUNCTIONAL COMPOUND CAPABLE OF DEGRADING BTK KINASE, AND COMPOSITION AND USE**

(30) Priority: 22.11.2021 CN 202111383481
(71) Applicant: Hangzhou HealZen Therapeutics Co., Ltd., Hangzhou, Zhejiang 310018 (CN); Shanghai Institute of Materia Medica, Chinese Academy of Sciences, Pudong, Shanghai 201203 (CN)
(72) Inventor: ZHOU, Xinglu, Hangzhou, Zhejiang 310018 (CN); LI, Jia, Shanghai 201203 (CN); LIU, Xingguo, Hangzhou, Zhejiang 310018 (CN); ZHOU, Yubo, Shanghai 201203 (CN); HU, Miao, Hangzhou, Zhejiang 310018 (CN); LUO, Xiaomin, Baise, Guangxi 533000 (CN); XIE, Jiangfeng, Hangzhou, Zhejiang 310018 (CN); KAN, Weijuan, Shanghai 201203 (CN); WU, Yizhe, Hangzhou, Zhejiang 310018 (CN); HU, Xiaobei, Wenzhou, Zhejiang 325000 (CN); SU, Mingbo, Shanghai 201203 (CN)
(74) Representative: Gong, Jinping
(86) International application number: PCT/CN2022/133404
(87) International publication number: WO 2023/088477

(57) **Abstract**

Disclosed in the present invention are a Bruton's tyrosine kinase degrading agent, which has a structure represented by general formula 1, or a stereoisomer, a mixture of stereoisomers, or a pharmaceutically acceptable salt and a prodrug thereof, Further disclosed are the uses of the compound and a pharmaceutical composition containing same in the preparation of a drug for treating Bruton's tyrosine kinase-related diseases such as B cell or plasma cell proliferative diseases, The compound of the present invention has potent cell proliferation inhibitory activity, effectively degrades Bruton's tyrosine kinase, and has a good liver particle metabolism stability and good oral absorption properties. The compound can be used alone in drugs or in combination with other drugs for treating a disease, disorder or condition that benefits from the inhibition of the Bruton's tyrosine kinase activity or degradation of the Bruton's tyrosine kinase.

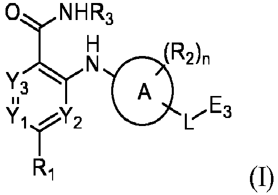

## Description

### TECHNICAL FIELD

The invention belongs to the technical field of pharmaceutical synthesis, and specifically relates to a multifunctional compound that can degrade BTK kinases, and its composition and application thereof.

### BACKGROUND

Bruton's tyrosine kinase (BTK), a member of the Tec protein family (BTK, Tec, Txk, Itk & Bmx), is a key signaling enzyme expressed in all hematopoietic cell types except T lymphocytes cells, BTK plays a critical role in the B cell signaling pathway linking cell surface B-cell receptor (BCR) stimulation to downstream intracellular responses, it has been clinically confirmed as an important drug target. BTK is a key regulator of B cell development, activation, signaling and survival. In addition, BTK plays a role in numerous other hematopoietic cells signaling pathways, such as Toll like receptor (TLR) and cytokine receptor-mediated TNF-α production in macrophages, and immunoglobulin E receptor (FcεR1) signaling in mast cells, inhibition of Fas/APO-1 cell apoptosis signaling and collagen-stimulated platelet aggregation in B-lineage lymphoid cells. See, for example, C.A. Jeffries et al., J. Bio. Chem. (2003) 278: 26258-26264, N. J. Horwood et al., J. Exp. Med. (2003) 197: 1603-1611. Research in recent years has shown that the BTK signaling pathway is a new hot spot in the clinical treatment research of non-Hodgkin lymphoma (NHL), especially chronic lymphocytic leukemia (CLL), B-cell lymphoma and autoimmune diseases. By acting on the BCR signaling pathway, small molecule BTK inhibitors bind to BTK to inhibit BTK autophosphorylation, prevent BTK activation, and thereby block cell conduction and induce apoptosis.

As BTK inhibition is used in the clinical setting, some CLL patients have developed acquired resistance to covalent BTK drugs caused by BTK C481 mutations. A new generation of BTK drugs is urgently needed to solve this acquired resistance.

Significant progress has been made in the targeted protein degradation technology (PROTAC), which achieves target protein degradation by inducing ubiquitination of target proteins. Drugs using this technology do not need to continuously occupy the target proteins, and the catalytic concentration can achieve target degradation. Therefore, the development of targeting degradation molecules for BTK is expected to solve the problem of acquired resistance to BTK.

### SUMMARY OF THE INVENTION

The purpose of the present invention is to provide a novel, unreported BTK degradation molecule with excellent oral PK property or its optical isomers, stereoisomers, or its stereoisomers mixture, or its pharmacologically acceptable salt or solvent compounds, prodrugs, and in BTK-related diseases B cell or plasma cell proliferative diseases and autoimmune diseases.

A first aspect of the present invention provides a compound as shown in the formula I, or its pharmaceutically acceptable salt thereof, prodrugs: 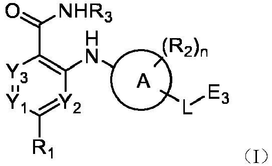 wherein:
L is selected from: a chemical group or a chemical bond connecting the ring A and the E3;
E3 is selected from: E3 ubiquitin ligase ligand;
ring A is selected from :3-12 membered heterocyclic ring, 6-10 membered aromatic ring, 5-12 membered heteroaromatic ring, 3-12 membered cycloalkane, 4-12 membered cycloalkyne, 3-12 membered cycloalkene, wherein the heterocyclic ring, heteroaromatic ring have 1-3 heteroatoms independently selected from N, O or S;
R₂ is selected from: H, halogen, cyano, C₁-C₆ alkyl, Ci-Ce haloalkyl, C₃-C₈ cycloalkyl, C₁-C₄ alkoxy, C₃-C₈ cycloalkoxy, C₃-C₈ heterocyclyl, C₁-C₄ alkylamino, -COOH, -NH₂, OH, NOz;
n is selected from: 0, 1, 2, 3, 4;
Y₁ is selected from: N, CR₄;
Y₂ is selected from: N, CR₅;
Y₃ is selected from: N, CR₅;
R₄ is selected from: halogen, C₁-C₄ alkyl, C₃-C₈ cycloalkyl, C₁-C₄ haloalkyl, C₃-C₈ halocycloalkyl, C₁-C₄ alkoxy, amino, carboxy, hydroxyl, cyano;
R₅ is selected from: H, halogen, C₁-C₄ alkyl, C₃-C₈ cycloalkyl, C₁-C₄ haloalkyl, C₃-C₈ halocycloalkyl, C₁-C₄ alkoxy, amino, carboxy, hydroxyl, cyano;
R₃ is selected from: H, C₁-C₈ alkyl, Ci-Ce haloalkyl, C₃-C₈ alkene, C₃-C₈ alkyne, cycloalkyl, heterocyclic, aryl;
R₁ is selected from: NR₆R₇, OR₆, SR₆, -C(O)R₆, -C(O)OR₆, -C(O)NR₆R₇, OC(O)R₆, OC(O)NR₆R₇, S(O)R₆, S(O)=NH)R₆, S(O)₂NR₆R₇, N(R₆)C(O)R₇, N(R₆)C(O)OR₇, C₁-C₈ alkyl, 3-12 membered heterocyclic ring, 5-6 membered heteroaromatic ring, C₃-C₈ cycloalkyl, aryl, where in the alkyl, heterocyclyl, heteroaromatic ring, cycloalky, aryl can be further substituted with one or more substituents selected from Rs;
R₈ is selected from: H, halogen, hydroxyl, amino, cyano, oxo, carboxy, Ci-Cs alkyl, Ci-Cs haloalkyl, Ci-Cs alkoxy, C₃-C₈ cycloalkyl, -C(O)Ra, -C(O)ORa, -C(O)NRaRb, ORa, OC(O)Ra, OC(O)NRaRb, SRa, S(O)Ra, S(O)(=NH)Ra, S(O)₂NRaRb, NRaRb, N(Ra)C(O)Rb, N(Ra)C(O)ORb, N(Rc )C(O)NRaRb, -N(Ra)S(O) ₂Rb, -N(Ra)S(O)₂NRbRc;wherein the alkyl, alkoxy and cycloalkyl can be further substituted with one or more substituents selected from: H, halogen, hydroxyl, amino, cyano, oxo, carboxyl, Ci-Cs alkyl, Ci-Cs haloalkyl, Ci-Cs alkoxy, C3-C8 cycloalkyl;
R₆ and R₇ are each independently selected from: H, Ci-Cs alkyl, aminoalky, hydroxyalkyl, C₃-C₈ cycloalkyl, heterocyclyl, aryl, heteroaryl; wherein the alkyl, aminoalky, hydroxyalkyl, cycloalkyl, heterocyclyl, aryl, heteroaryl can be further substituted with one or more substituents selected from: H, halogen, hydroxyl, amino, cyano, oxo, carboxyl, Ci-Cs alkyl, Ci-Cs haloalkyl, Ci-Cs alkoxy, C₃-C₈ cycloalkyl, -C(O)Ra, -C(O)ORa, -C(O)NRaRb, ORa, OC(O)Ra, OC(O)NRaRb, SRa, S(O)Ra, S(O)(=NH)Ra, S(O)₂NRaRb, NRaRb, N(Ra)C(O)Rb, N(Ra)C(O)ORb, N(Rc )C(O)NRaRb, -N(Ra)S(O) ₂Rb, -N(Ra)S(O)₂NRbRc;
Ra, Rb and Rc are each independently selected from: H, halogen, alkyl, amino, hydroxyl, cyano, C₃-C₈ cycloalkyl, alkylamino, alkoxy.
When Y₂ and Y₃ are selected from CR₅; CR₅ is independent of each other in both substitutions and may or may not be the same.

As preferred, Y₁, Y₂ and Y₃ are N at the same time.

Still further, a preferred compound of the present invention;
ring A is selected from: 6-10 membered aromatic ring, 5-12 membered heteroaromatic ring, wherein the heteroaromatic ring have 1-3 heteroatoms independently selected from N, O or S, wherein:
ring A is selected from:

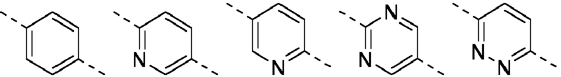

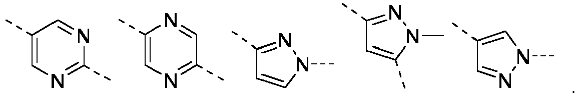

Still further, a preferred compound of the present invention has the structure of the general formula II(a)~II(i): 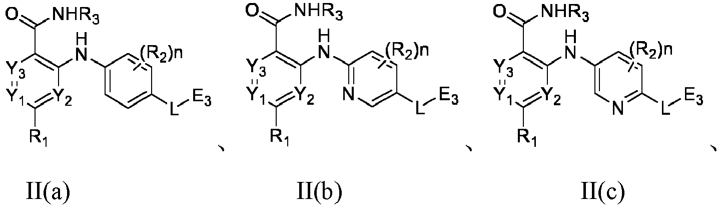 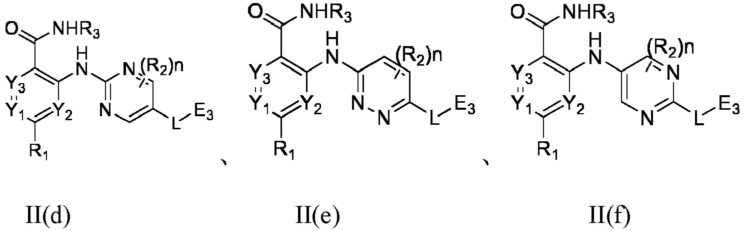 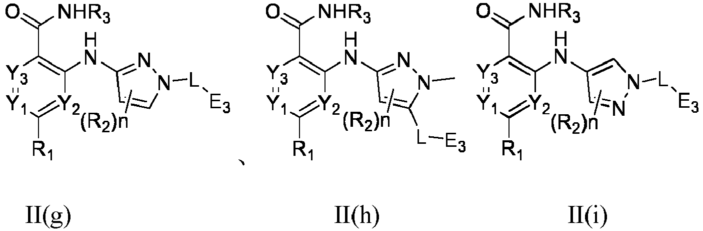 or its stereoisomer thereof, or its stereoisomer mixture thereof, or its pharmaceutically acceptable salt thereof, prodrugs;

Still further, a preferred compound of the present invention has the structure of the general formula III(a)-III(c): 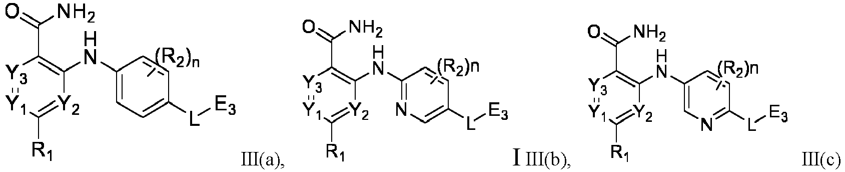 or its stereoisomer thereof, or its stereoisomer mixture thereof, or its pharmaceutically acceptable salt thereof, prodrugs;
R₁ is selected from: NR₆R₇, OR₆, C₁-C₈ alkyl, 3-12 membered heterocyclic ring, 5-6 membered heteroaromatic ring, C₃-C₈ cycloalkyl, aryl, where in the alkyl, heterocyclyl, heteroaromatic ring, cycloalky, aryl can be further substituted with one or more substituents selected from Rs;
R₈ is selected from: H, halogen, hydroxyl, amino, cyano, oxo, carboxyl, Ci-Cs alkyl, Ci-Cs haloalkyl, Ci-Cs alkoxy, C₃-C₈ cycloalkyl, -C(O)Ra, -C(O)ORa, -C(O)NRaRb, ORa, OC(O)Ra, OC(O)NRaRb, SRa, S(O)Ra, S(O)(=NH)Ra, S(O)₂NRaRb, NRaRb, N(Ra)C(O)Rb, N(Ra)C(O)ORb, N(Rc )C(O)NRaRb, -N(Ra)S(O) ₂Rb, -N(Ra)S(O)₂NRbRc; wherein the alkyl, alkoxy and cycloalkyl can be further substituted with one or more substituents selected from: H, halogen, hydroxyl, amino, cyano, oxo, carboxyl, Ci-Cs alkyl, Ci-Cs haloalkyl, Ci-Cs alkoxy, C₃-C₈ cycloalkyl;
R₆ and R₇ are each independently selected from: H, C₁-C₈ alkyl, aminoalky, hydroxyalkyl, C₃-C₈ cycloalkyl, heterocyclyl, aryl, heteroaryl; wherein the alkyl, aminoalky, hydroxyalkyl, cycloalkyl, heterocyclyl, aryl, heteroaryl can be further substituted with one or more substituents selected from: H, halogen, hydroxyl, amino, cyano, oxo, carboxyl, Ci-Cs alkyl, Ci-Cs haloalkyl, C₁-C₈ alkoxy, C₃-C₈ cycloalkyl, -C(O)Ra, -C(O)ORa, -C(O)NRaRb, ORa, OC(O)Ra, OC(O)NRaRb, SRa, S(O)Ra, S(O)(=NH)Ra, S(O)₂NRaRb, NRaRb, N(Ra)C(O)Rb, N(Ra)C(O)ORb, N(Rc )C(O)NRaRb, -N(Ra)S(O) ₂Rb, -N(Ra)S(O)₂NRbRc;
Ra, Rb and Rc are each independently selected from: H, halogen, alkyl, amino, hydroxyl, cyano, C₃-C₈ cycloalkyl, alkylamino, alkoxy.

In some embodiments, R₂ is preferably selected from: H, F.

In some embodiments, fragments in the general formula (I), II (a)~II (i), III (a), III (b), or III (c) 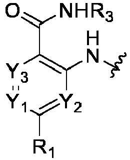 preferably selected from: 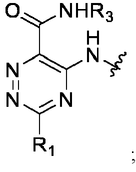 Further preferably selected from:

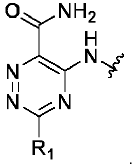

In some embodiments, R₁ is preferably selected from: 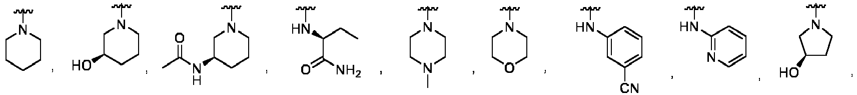 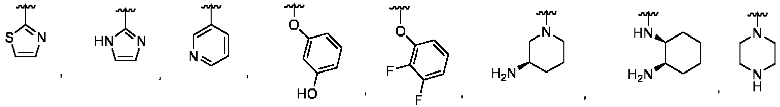 still further, as a preferred embodiment:
L is selected from: 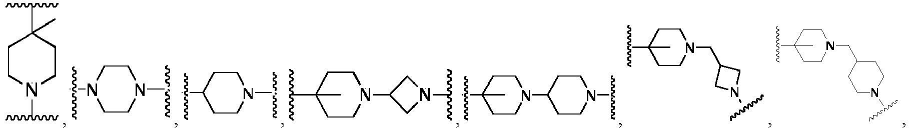 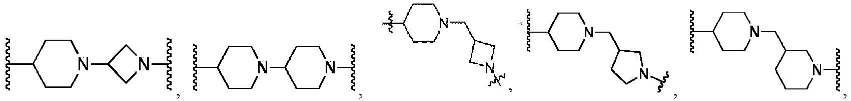 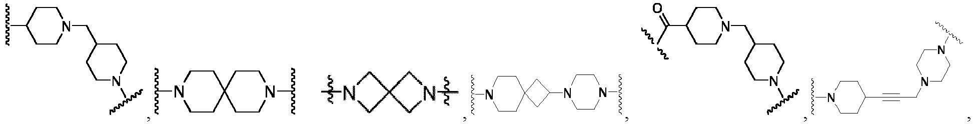 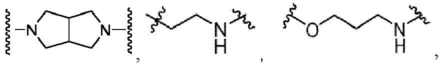 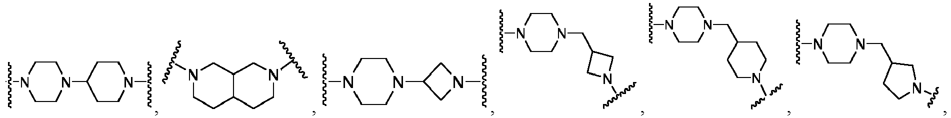 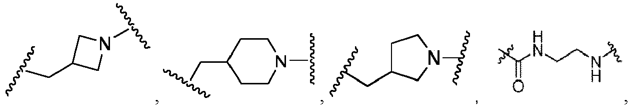 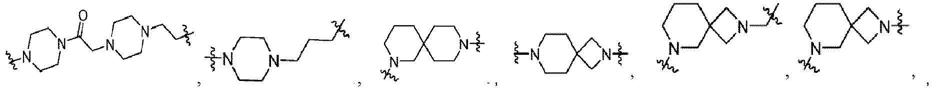 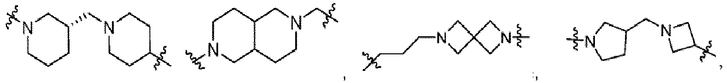 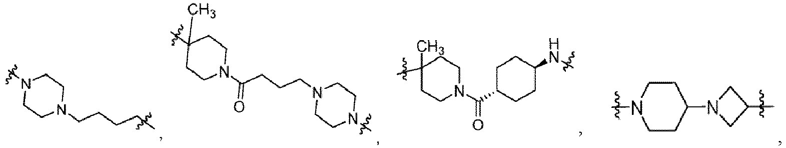 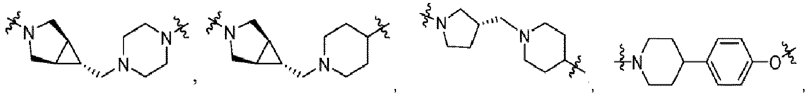 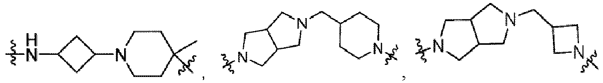 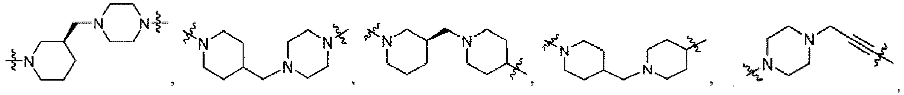 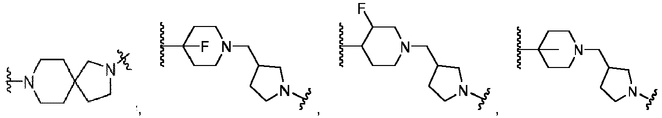 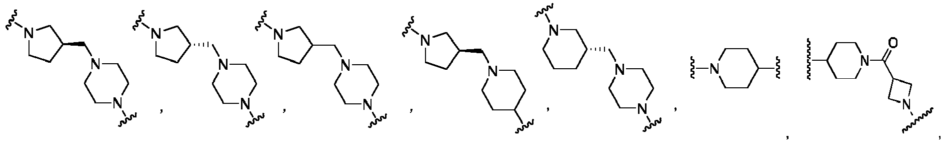 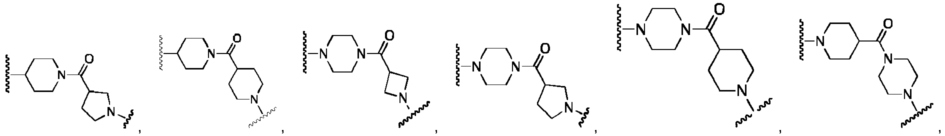 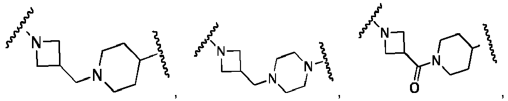 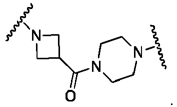
E3 is selected from: 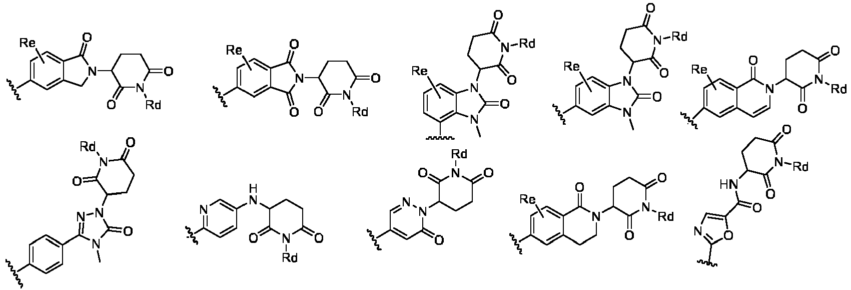 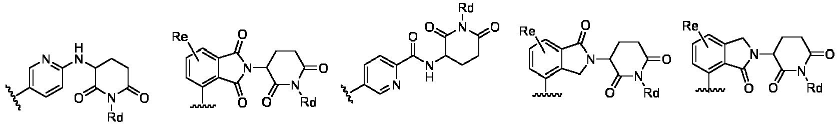 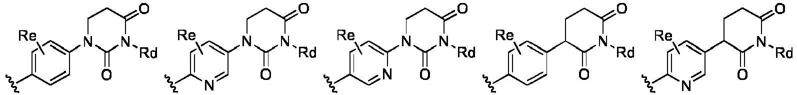 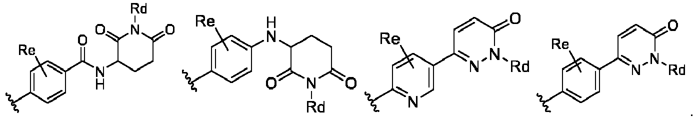
Re is selected from: H, F, Cl, -GH₃, -OMe, -CN, -CF₃;
Rd is selected from: H, L₂R_{g};
L₂ is selected from: 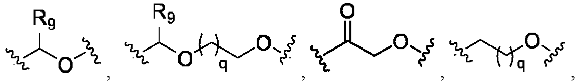 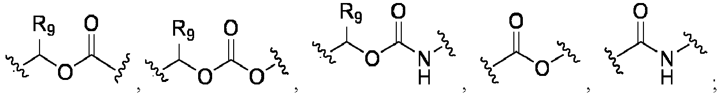
R₉ is selected from: H, C₁-C₅ alkyl (preferably methyl);
q is selected from: 1, 2, 3, 4 or 5;
R_{g} is selected from: H, C₁-C₈ alkyl, substituted or not substituted C₁-C₈ alkyl (preferably tert-butyl, isobutyl, etc); substituents selected from: amino, hydroxyl, cyano, halogen.
still further, E3 is preferably selected from:

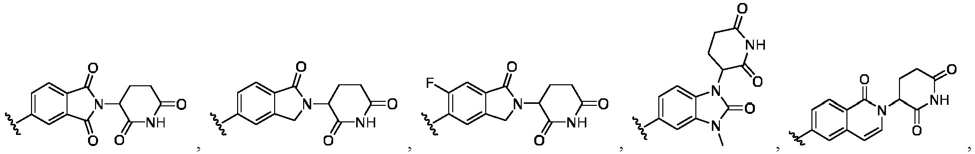

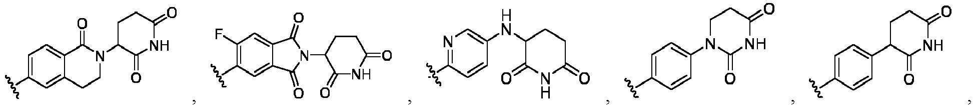

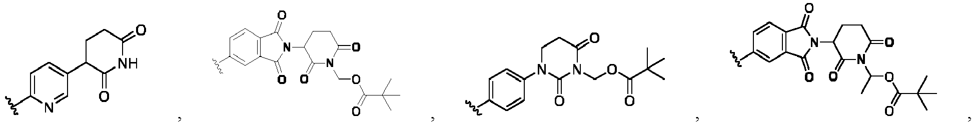

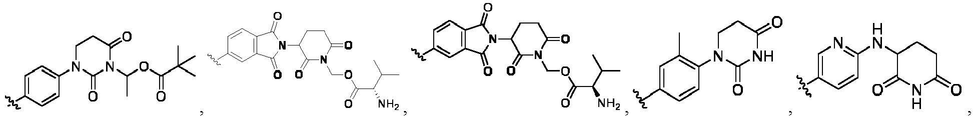

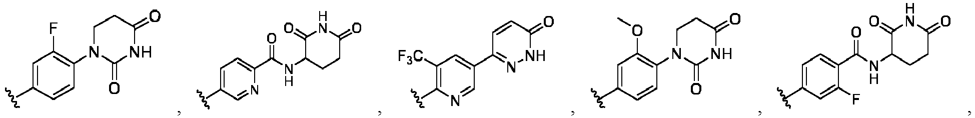

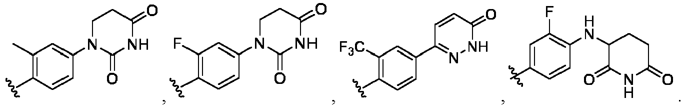

Preferably, the compound or the stereoisomer thereof, or the stereoisomer mixture thereof, or the pharmaceutically acceptable salt thereof, prodrugs is selected from the following compounds:

| No. | Structure | No. | Structure |
|---|---|---|---|
| 001 | 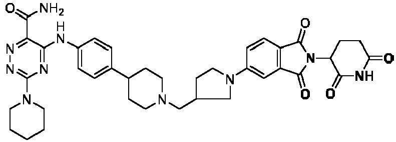 | 002 | 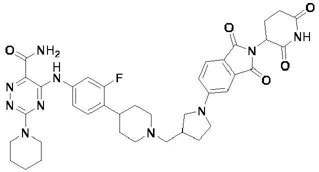 |
| 003 | 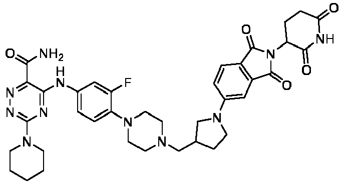 | 004 | 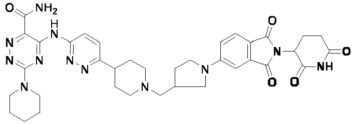 |
| 005 | 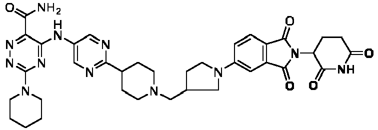 | 006 | 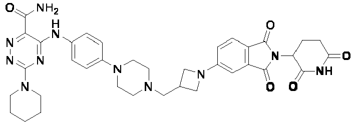 |
| | | | |
| 007 | 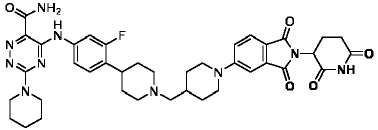 | 008 | 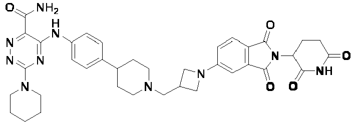 |
| 009 | 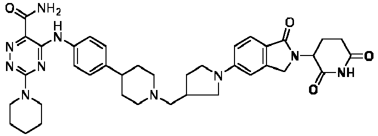 | 010 | 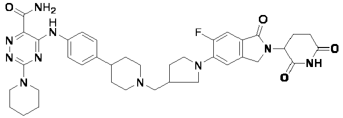 |
| 011 | 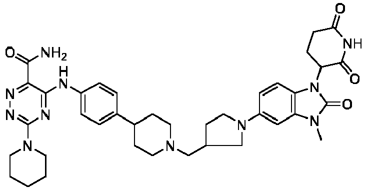 | 012 | 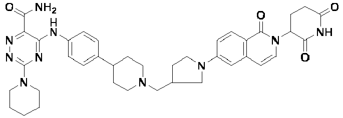 |
| 013 | 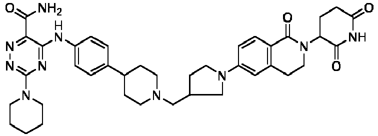 | 014 | 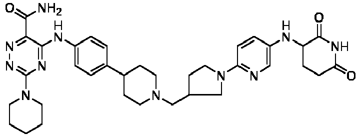 |
| 015 | 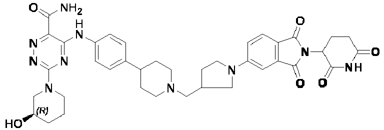 | 016 | 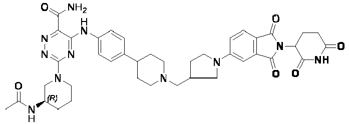 |
| 017 | 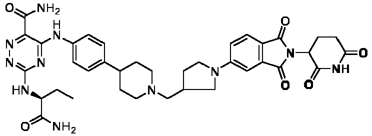 | 018 | 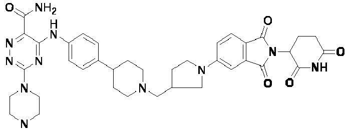 |
| 019 | 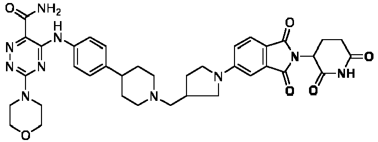 | 020 | 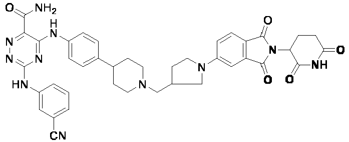 |
| 021 | 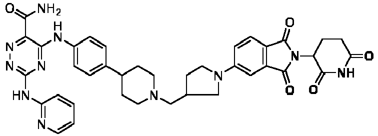 | 022 | 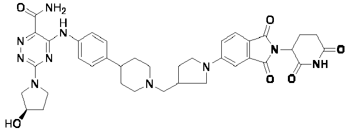 |
| 023 | 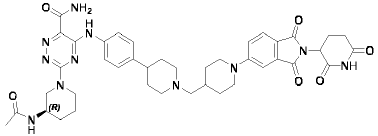 | 024 | 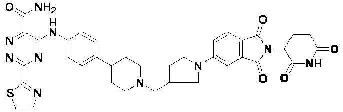 |
| 025 | 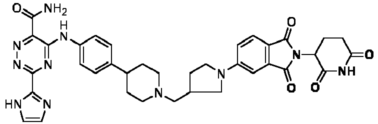 | 026 | 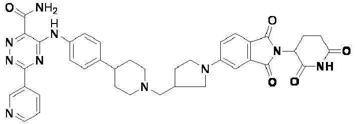 |
| 027 | 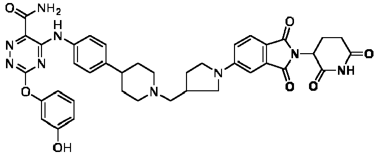 | 028 | 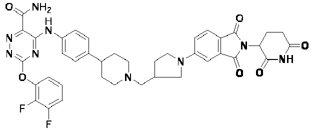 |
| 029 | 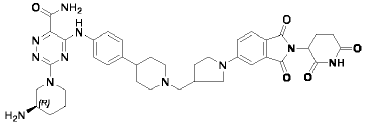 | 030 | 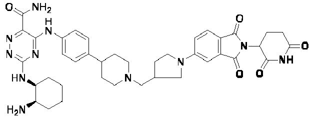 |
| 031 | 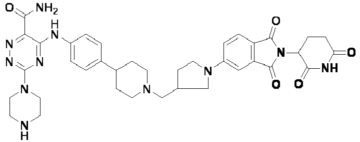 | 032 | 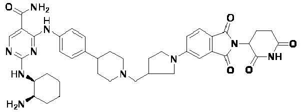 |
| 033 | 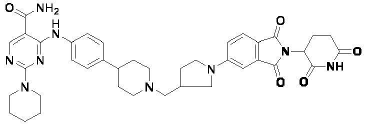 | 034 | 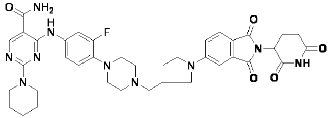 |
| 035 | 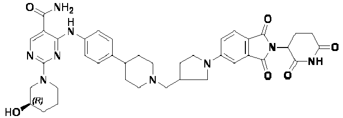 | 036 | 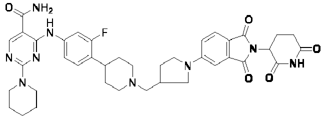 |
| 037 | 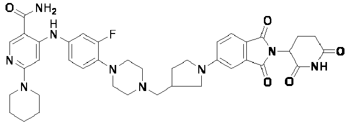 | 038 | 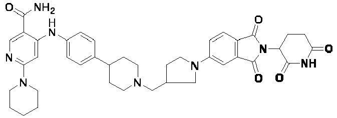 |
| 039 | 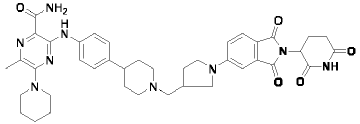 | 040 | 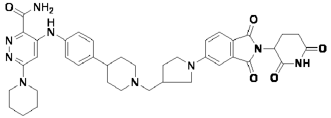 |
| 041 | 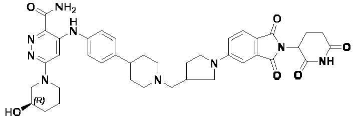 | 042 | 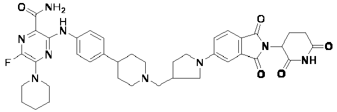 |
| 043 | 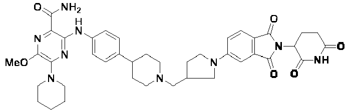 | 044 | 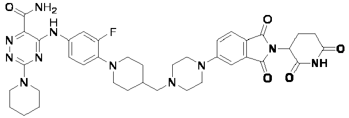 |
| 045 | 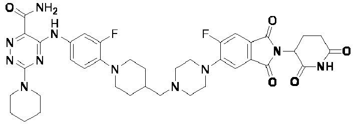 | 046 | 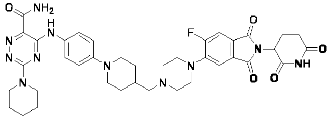 |
| 047 | 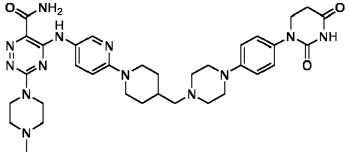 | 048 | 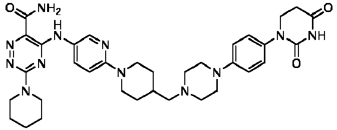 |
| 049 | 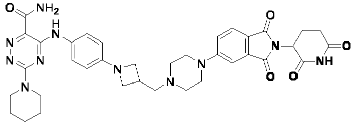 | 050 | 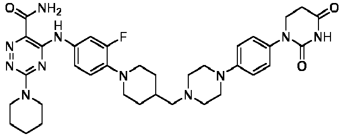 |
| 051 | 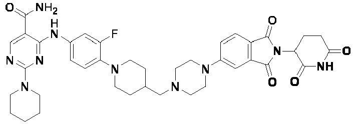 | 052 | 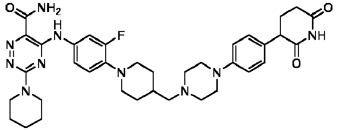 |
| 053 | 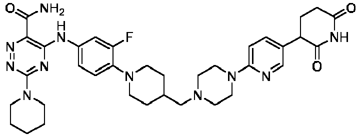 | 054 | 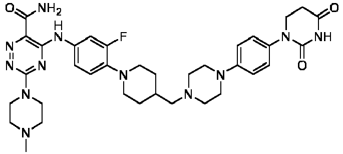 |
| 055 | 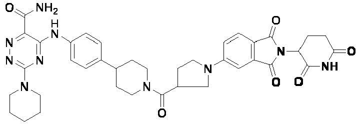 | 056 | 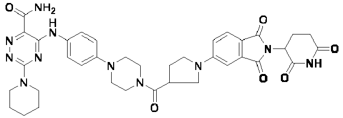 |
| 057 | 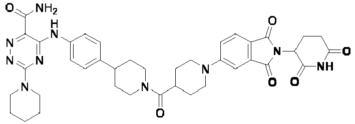 | 058 | 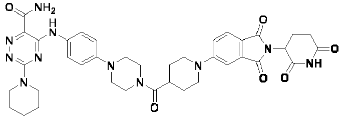 |
| 059 | 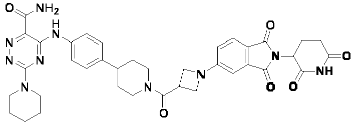 | 060 | 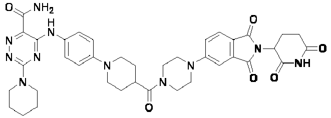 |
| 061 | 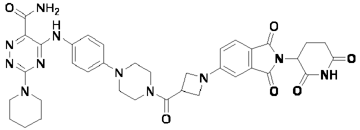 | 062 | 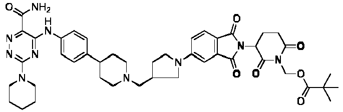 |
| 063 | 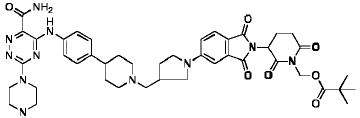 | 064 | 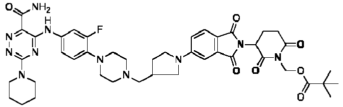 |
| 065 | 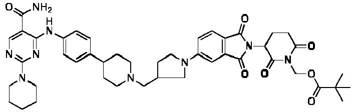 | 066 | 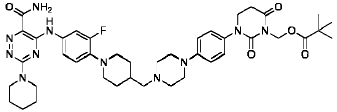 |
| 067 | 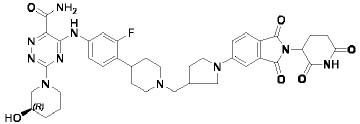 | 068 | 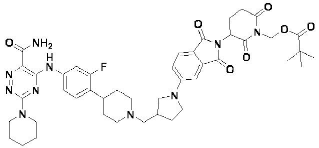 |
| 069 | 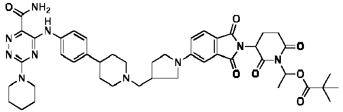 | 070 | 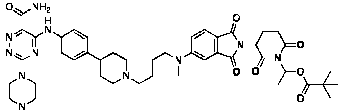 |
| 071 | 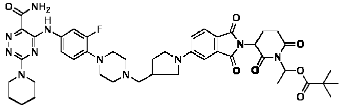 | 072 | 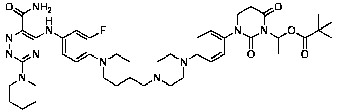 |
| 073 | 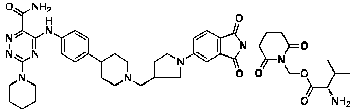 | 074 | 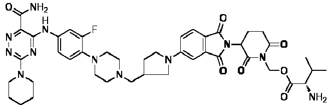 |
| 075 | 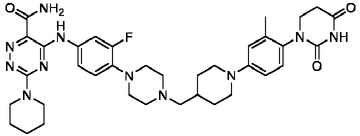 | 076 | 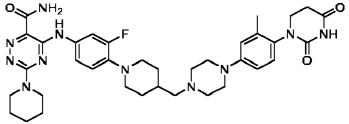 |
| 077 | 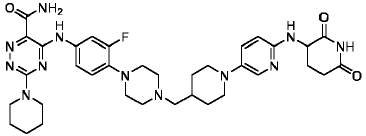 | 078 | 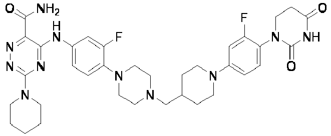 |
| 079 | 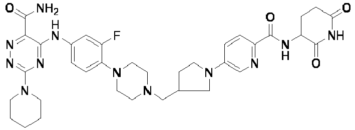 | 080 | 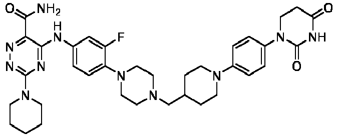 |
| 081 | 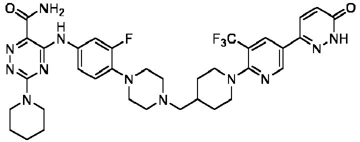 | 082 | 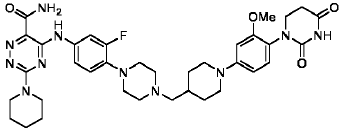 |
| 083 | 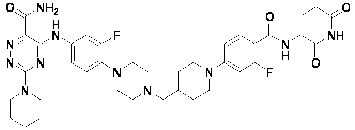 | 084 | 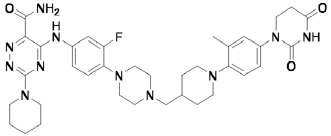 |
| 085 | 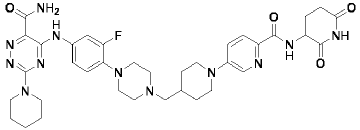 | 086 | 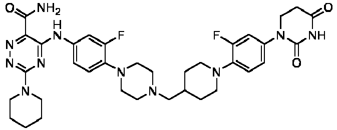 |
| 087 | 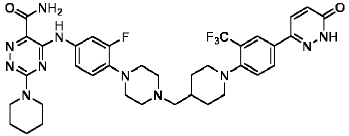 | 088 | 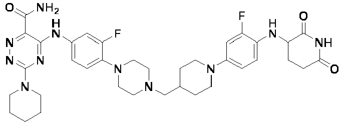 |
| 089 | 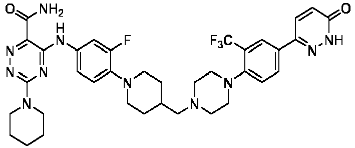 | 090 | 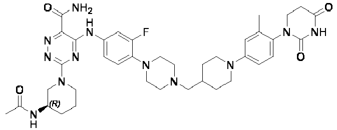 |
| 091 | 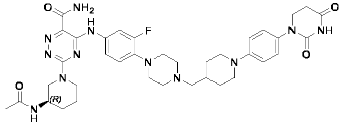 | 092 | 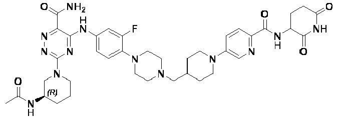 |
| 093 | 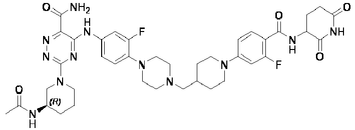 | 094 | 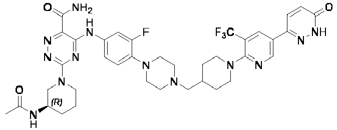 |
| 095 | 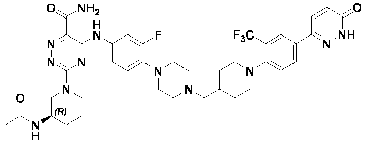 | 096 | 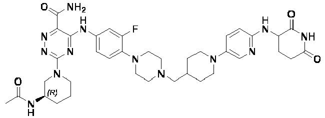 |
| 097 | 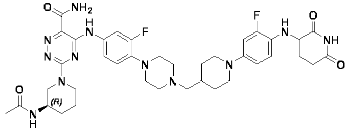 | 098 | 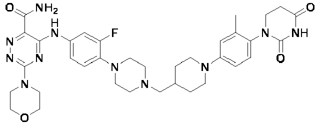 |
| 099 | 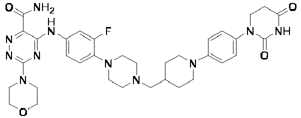 | 100 | 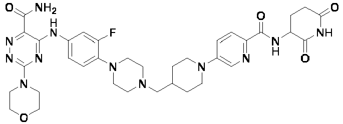 |
| 101 | 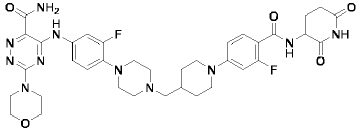 | 102 | 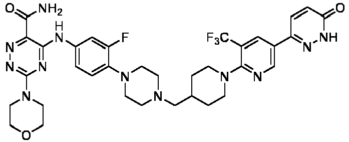 |
| 103 | 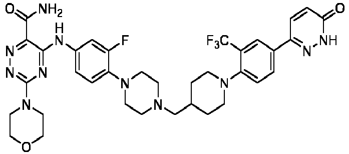 | 104 | 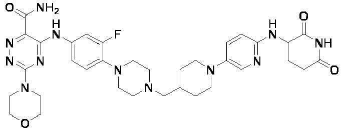 |
| 105 | 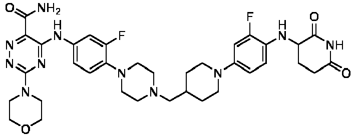 | | |

or its stereoisomer thereof, or its stereoisomer mixture thereof, or its pharmaceutically acceptable salt thereof, prodrugs. Preferably, the compound is the following compound:
5-((4-(1-((1-(2-(2,6-dioxopiperidin-3-yl)-1,3-dioxoisoindolin-5-yl) pyrrolidin-3-yl) methyl) piperidin-4-yl) phenyl) amino)-3-(piperidin-1-yl)-1,2,4-triazine-6-carboxamide **001**
5-((4-(1-((1-(2-(2,6-dioxopiperidin-3-yl)-1,3-dioxoisoindolin-5-yl) pyrrolidin-3-yl) methyl) piperidin-4- yl)-3-fluorophenyl) amino)-3-(piperidin-1-yl)-1,2,4-triazine-6-carboxamide **002**
5-((4-(4-((1-(2-(2,6-dioxopiperidin-3-yl)-1,3-dioxoisoindolin-5-yl) pyrrolidin-3-yl) methyl) piperazin-1-yl) -3-fluorophenyl) amino)-3-(piperidin-1-yl)-1,2,4-triazine-6-carboxamide **003**
5-((6-(1-((1-(2-(2,6-dioxopiperidin-3-yl)-1,3-dioxoisoindolin-5-yl) pyrrolidin-3-yl) methyl) piperidin-4-yl) pyridazin-3-yl) amino)-3-(piperidin-1-yl)-1,2,4-triazine-6-carboxamide **004**
5-((2-(1-((1-(2-(2,6-dioxopiperidin-3-yl)-1,3-dioxoisoindolin-5-yl) pyrrolidin-3-yl) methyl) piperidin-4-yl) pyrimidin-5-yl) amino)-3-(piperidin-1-yl)-1,2,4-triazine-6-carboxamide **005**
5-((4-(4-((1-(2-(2,6-dioxopiperidin-3-yl)-1,3-dioxoisoindolin-5-yl)azetidin-3-yl) methyl) piperazin-1-yl) phenyl) amino)-3-(piperidin-1-yl)-1,2,4-triazine-6-carboxamide **006**
5-((4-(1-((1-(2-(2,6-dioxopiperidin-3-yl)-1,3-dioxoisoindolin-5-yl) piperidin-4-yl) methyl) piperidin-4-yl)-3-fluorophenyl) amino)-3-(piperidin-1-yl)-1,2,4-triazine-6-carboxamide **007**
5-((4-(1-((1-(2-(2,6-dioxopiperidin-3-yl)-1,3-dioxoisoindolin-5-yl) azetidin-3-yl) methyl) piperidin-4-yl) phenyl) amino)-3-(piperidin-1-yl)-1,2,4-triazine-6-carboxamide **008**
5-((4-(1-((1-(2-(2,6-dioxopiperidin-3-yl)-1-oxoisoindolin-5-yl)pyrrolidin-3-yl) methyl) piperidin-4-yl) phenyl) amino)-3-(piperidin-1-yl)-1,2,4-triazine-6-carboxamide **009**
5-((4-(1-((1-(2-(2,6-dioxopiperidin-3-yl)-6-fluoro-1-oxoisoindolin-5-yl) pyrrolidin-3-yl) methyl) piperidin-4-yl) phenyl) amino)-3-(piperidin-1-yl)-1,2,4-triazine-6-carboxamide **010**
5-((4-(1-((1-(1-(2,6-dioxopiperidin-3-yl)-3-methyl-2-oxo-2,3-dihydro-1H-benzo[d]imidazol-5-yl) pyrrolidine -3-yl) methyl) piperidin-4-yl) phenyl) amino)-3-(piperidin-1-yl)-1,2,4-triazine-6-carboxamide **011**
5-((4-(1-((1-(2-(2,6-dioxopiperidin-3-yl)-1-oxo-1,2-dihydroisoquinolin-6-yl)pyrrolidin-3-yl) methyl) piperidin-4-yl) phenyl) amino)-3-(piperidin-1-yl)-1,2,4-triazine-6-carboxamide **012**
5-((4-(1-((1-(2-(2,6-dioxopiperidin-3-yl)-1-oxo-1,2,3,4-tetrahydroisoquinolin-6-yl)pyrrolidin-3-yl) methyl) piperidin-4-yl) phenyl) amino)-3-(piperidin-1-yl)-1,2,4-triazine-6-carboxamide **013**
5-((4-(1-((1-(5-((2,6-dioxopiperidin-3-yl) amino) pyridin-2-yl) pyrrolidin-3-yl) methyl) piperidin-4-yl) phenyl) amino)-3-(piperidin-1-yl)-1,2,4-triazine-6-carboxamide **014**
5-((4-(1-((1-(2-(2,6-dioxopiperidin-3-yl)-1,3-dioxoisoindolin-5-yl) pyrrolidin-3-yl) methyl) piperidin-4-yl) phenyl) amino)-3-((R)-3-hydroxypiperidin-1-yl)-1,2,4-triazine-6-carboxamide **015**
3-((R)-3-acetamidopiperidin-1-yl)-5-((4-(1-((1-(2-(2,6-dioxopiperidin-3-yl)-1,3-dioxoisoindolin-5-yl) pyrrolidin-3-yl) methyl) piperidin-4-yl) phenyl) amino)-1,2,4-triazine-6-carboxamide **016**
3-(((S)-1-amino-1-oxobutan-2-yl) amino)-5-((4-(1-((1-(2-(2,6-dioxopiperidin-3-yl)-1,3-dioxoisoindolin-5-yl) pyrrolidin-3-yl) methyl) piperidin-4-yl) phenyl) amino)-1,2,4-triazine-6-carboxamide **017**
5-((4-(1-((1-(2-(2,6-dioxopiperidin-3-yl)-1,3-dioxoisoindolin-5-yl) pyrrolidin-3-yl) methyl) piperidin-4-yl) phenyl) amino)-3-(4-methylpiperazin-1-yl)-1,2,4-triazine-6-carboxamide **018**
5-((4-(1-((1-(2-(2,6-dioxopiperidin-3-yl)-1,3-dioxoisoindolin-5-yl) pyrrolidin-3-yl) methyl) piperidin-4-yl) phenyl) amino)-3-morpholino-1,2,4-triazine-6-carboxamide **019**
3-((3-cyanophenyl) amino)-5-((4-(1-((1-(2-(2,6-dioxopiperidin-3-yl)-1,3-dioxoisoindolin-5-yl) pyrrolidin-3-yl) methyl) piperidin-4-yl) phenyl) amino)-1,2,4-triazine-6-carboxamide **020**
5-((4-(1-((1-(2-(2,6-dioxopiperidin-3-yl)-1,3-dioxoisoindolin-5-yl) pyrrolidin-3-yl) methyl) piperidin-4-yl) phenyl) amino)-3-(pyridin-2-ylamino)-1,2,4-triazine-6-carboxamide **021**
5-((4-(1-((1-(2-(2,6-dioxopiperidin-3-yl)-1,3-dioxoisoindolin-5-yl) pyrrolidin-3-yl) methyl) piperidin-4-yl) phenyl) amino)-3-((R)-3-hydroxypyrrolidin-1-yl)-1,2,4-triazine-6-carboxamide **022**
3-((R)-3-acetamidopiperidin-1-yl)-5-((4-(1-((1-(2-(2,6-dioxopiperidin-3-yl)-1,3-dioxoisoindolin-5-yl)piperidin-4-yl) methyl) piperidin-4-yl) phenyl) amino)-1,2,4-triazine-6-carboxamide **023**
5-((4-(1-((1-(2-(2,6-dioxopiperidin-3-yl)-1,3-dioxoisoindolin-5-yl) pyrrolidin-3-yl) methyl) piperidin-4-yl) phenyl) amino)-3-(thiazol-2-yl)-1,2,4-triazine-6-carboxamide **024**
5-((4-(1-((1-(2-(2,6-dioxopiperidin-3-yl)-1,3-dioxoisoindolin-5-yl) pyrrolidin-3-yl) methyl) piperidin-4-yl) phenyl) amino)-3-(1H-imidazol-2-yl)-1,2,4-triazine-6-carboxamide **025**
5-((4-(1-((1-(2-(2,6-dioxopiperidin-3-yl)-1,3-dioxoisoindolin-5-yl) pyrrolidin-3-yl) methyl) piperidin-4-yl) phenyl) amino)-3-(pyridin-3-yl)-1,2,4-triazine-6-carboxamide **026**
5-((4-(1-((1-(2-(2,6-dioxopiperidin-3-yl)-1,3-dioxoisoindolin-5-yl) pyrrolidin-3-yl) methyl) piperidin-4-yl) phenyl) amino)-3-(3-hydroxyphenoxy)-1,2,4-triazine-6-carboxamide **027**
3-(2,3-difluorophenoxy)-5-((4-(1-((1-(2-(2,6-dioxopiperidin-3-yl)-1,3-dioxoisoindolin-5-yl)pyrrolidin-3-yl) methyl) piperidin-4-yl) phenyl) amino)-1,2,4-triazine-6-carboxamide **028**
3-((R)-3-aminopiperidin-1-yl)-5-((4-(1-((1-(2-(2,6-dioxopiperidin-3-yl)-1,3-dioxoisoindolin-5-yl) pyrrolidin-3-yl) methyl) piperidin-4-yl) phenyl) amino)-1,2,4-triazine-6-carboxamide **029**
3-(((1S,2R)-2-aminocyclohexyl)amino)-5-((4-(1-((1-(2-(2,6-dioxopiperidin-3-yl)-1,3-dioxoisoindolin-5-yl)pyrrolidin-3-yl) methyl) piperidin-4-yl) phenyl) amino)-1,2,4-triazine-6-carboxamide **030**
5-((4-(1-((1-(2-(2,6-dioxopiperidin-3-yl)-1,3-dioxoisoindolin-5-yl) pyrrolidin-3-yl) methyl) piperidin-4-yl) phenyl) amino)-3-(piperazin-1-yl)-1,2,4-triazine-6-carboxamide **031**
2-(((1S,2R)-2-aminocyclohexyl)amino)-4-((4-(1-((1-(2-(2,6-dioxopiperidin-3-yl)-1,3-dioxoisoindolin-5-yl)pyrrolidin-3-yl) methyl) piperidin-4-yl) phenyl) amino) pyrimidine-5-carboxamide **032**
4-((4-(1-((1-(2-(2,6-dioxopiperidin-3-yl)-1,3-dioxoisoindolin-5-yl) pyrrolidin-3-yl) methyl) piperidin-4-yl) phenyl) amino)-2-(piperidin-1-yl) pyrimidine-5-carboxamide **033**
4-((4-(4-((1-(2-(2,6-dioxopiperidin-3-yl)-1,3-dioxoisoindolin-5-yl) pyrrolidin-3-yl) methyl) piperazin-1-yl)-3-fluorophenyl) amino)-2-(piperidin-1-yl) pyrimidine-5-carboxamide **034**
4-((4-(1-((1-(2-(2,6-dioxopiperidin-3-yl)-1,3-dioxoisoindolin-5-yl) pyrrolidin-3-yl) methyl) piperidin-4-yl) phenyl) amino)-2-((R)-3-hydroxypiperidin-1-yl)pyrimidine-5-carboxamide **035**
4-((4-(1-((1-(2-(2,6-dioxopiperidin-3-yl)-1,3-dioxoisoindolin-5-yl) pyrrolidin-3-yl) methyl) piperidin-4-yl)-3-fluorophenyl) amino)-2-(piperidin-1-yl) pyrimidine-5-carboxamide **036**
4-((4-(4-((1-(2-(2,6-dioxopiperidin-3-yl)-1,3-dioxoisoindolin-5-yl) pyrrolidin-3-yl) methyl) piperazin-1-yl)-3-fluorophenyl) amino)-6-(piperidin-1-yl) nicotinamide **037**
4-((4-(1-((1-(2-(2,6-dioxopiperidin-3-yl)-1,3-dioxoisoindolin-5-yl) pyrrolidin-3-yl) methyl) piperidin-4-yl) phenyl) amino)-6-(piperidin-1-yl) nicotinamide **038**
3-((4-(1-((1-(2-(2,6-dioxopiperidin-3-yl)-1,3-dioxoisoindolin-5-yl) pyrrolidin-3-yl) methyl) piperidin-4-yl) phenyl) amino)-6-methyl-5-(piperidin-1-yl) pyrazine-2-carboxamide **039**
4-((4-(1-((1-(2-(2,6-dioxopiperidin-3-yl)-1,3-dioxoisoindolin-5-yl) pyrrolidin-3-yl) methyl) piperidin-4-yl) phenyl) amino)-6-(piperidin-1-yl) pyridazine-3-carboxamide **040**
4-((4-(1-((1-(2-(2,6-dioxopiperidin-3-yl)-1,3-dioxoisoindolin-5-yl) pyrrolidin-3-yl) methyl) piperidin-4-yl) phenyl) amino)-6-((R)-3-hydroxypiperidin-1-yl) pyridazine-3-carboxamide **041**
3-((4-(1-((1-(2-(2,6-dioxopiperidin-3-yl)-1,3-dioxoisoindolin-5-yl)pyrrolidin-3-yl) methyl) piperidin-4-yl) phenyl) amino)-6-fluoro-5-(piperidin-1-yl) pyrazine-2-carboxamide **042**
3-((4-(1-((1-(2-(2,6-dioxopiperidin-3-yl)-1,3-dioxoisoindolin-5-yl) pyrrolidin-3-yl) methyl) piperidin-4-yl) phenyl) amino)-6-methoxy-5-(piperidin-1-yl) pyrazine-2-carboxamide **043**
5-((4-(4-((4-(2-(2,6-dioxopiperidin-3-yl)-1,3-dioxoisoindolin-5-yl) perazin-1-yl) ethyl) iperidin-1-yl)-3- fluorophenyl) amino)-3-(piperidin-1-yl)-1,2,4-triazine-6-carboxamide **044**
5-((4-(4-((4-(2-(2,6-dioxopiperidin-3-yl)-6-fluoro-1,3-dioxoisoindolin-5-yl) piperazin-1-yl) methyl) piperidin-1-yl)-3-fluorophenyl)amino)-3-(piperidin-1-yl)-1,2,4-triazine-6-carboxamide **045**
5-((4-(4-((4-(2-(2,6-dioxopiperidin-3-yl)-6-fluoro-1,3-dioxoisoindolin-5-yl) piperazin-1-yl) methyl) piperidin-1-yl) phenyl) amino)-3-(piperidin-1-yl)-1,2,4-triazine-6-carboxamide **046**
5-((6-(4-((4-(4-(2,4-dioxotetrahydropyrimidin-1(2H)-yl) phenyl) piperazin-1-yl) methyl) piperidin-1-yl) pyridin-3-yl) amino)-3-(4-methylpiperazin-1-yl)-1,2,4-triazine-6-carboxamide **047**
5-((6-(4-((4-(4-(2,4-dioxotetrahydropyrimidin-1(2H)-yl) phenyl) piperazin-1-yl) methyl) piperidin-1-yl) pyridin-3-yl) amino)-3-(piperidin-1-yl)-1,2,4-triazine-6-carboxamide **048**
5-((4-(3-((4-(2-(2,6-dioxopiperidin-3-yl)-1,3-dioxoisoindolin-5-yl) piperazin-1-yl) methyl) azetidin-1-yl) phenyl) amino)-3-(piperidin-1-yl)-1,2,4-triazine-6-carboxamide **049**
5-((4-(4-((4-(4-(2,4-dioxotetrahydropyrimidin-1(2H)-yl) phenyl) piperazin-1-yl) methyl) piperidin-1-yl)-3-fluorophenyl) amino)-3-(piperidin-1-yl)-1,2,4-triazine-6-carboxamide **050**
4-((4-(4-((4-(2-(2,6-dioxopiperidin-3-yl)-1,3-dioxoisoindolin-5-yl) piperazin-1-yl) methyl) piperidin-1-yl)-3-fluorophenyl) amino)-2-(piperidin-1-yl) pyrimidine-5-carboxamide **51**
5-((4-(4-((4-(4-(2,6-dioxopiperidin-3-yl) phenyl) piperazin-1-yl) methyl) piperidin-1-yl)-3-fluorophenyl) amino)-3-(piperidin-1-yl)-1,2,4-triazine-6-carboxamide **052**
5-((4-(4-((4-(5-(2,6-dioxopiperidin-3-yl) pyridin-2-yl) piperazin-1-yl) methyl) piperidin-1-yl)-3-fluorophenyl) amino)-3-(piperidin-1-yl)-1,2,4-triazine-6-carboxamide **053**
5-((4-(4-((4-(4-(2,4-dioxotetrahydropyrimidin-1(2H)-yl)phenyl) piperazin-1-yl) methyl) piperidin-1-yl)-3-fluorophenyl) amino)-3-(4-methylpiperazin-1-yl)-1,2,4-triazine-6-carboxamide **054**
5-((4-(1-(1-(2-(2,6-dioxopiperidin-3-yl)-1,3-dioxoisoindolin-5-yl)pyrrolidine-3-carbonyl) piperidin-4-yl) phenyl) amino)-3-(piperidin-1-yl)-1,2,4-triazine-6-carboxamide **055**
5-((4-(4-(1-(2-(2,6-dioxopiperidin-3-yl)-1,3-dioxoisoindolin-5-yl) pyrrolidine-3-carbonyl) piperazin-1-yl) phenyl) amino)-3-(piperidin-1-yl)-1,2,4-triazine-6-carboxamide **056**
5-((4-(1-(1-(2-(2,6-dioxopiperidin-3-yl)-1,3-dioxoisoindolin-5-yl) piperidine-4-carbonyl) piperidin-4-yl) phenyl) amino)-3-(piperidin-1-yl)-1,2,4-triazine-6-carboxamide **057**
5-((4-(4-(1-(2-(2,6-dioxopiperidin-3-yl)-1,3-dioxoisoindolin-5-yl) piperidine-4-carbonyl) piperazin-1-yl) phenyl) amino)-3-(piperidin-1-yl)-1,2,4-triazine-6-carboxamide **058**
5-((4-(1-(1-(2-(2,6-dioxopiperidin-3-yl)-1,3-dioxoisoindolin-5-yl) azetidine-3-carbonyl) piperidin-4-yl) phenyl) amino)-3-(piperidin-1-yl)-1,2,4-triazine-6-carboxamide **059**
5-((4-(4-(4-(2-(2,6-dioxopiperidin-3-yl)-1,3-dioxoisoindolin-5-yl) piperazine-1-carbonyl) piperidin-1-yl) phenyl) amino)-3-(piperidin-1-yl)-1,2,4-triazine-6-carboxamide **060**
5-((4-(4-(1-(2-(2,6-dioxopiperidin-3-yl)-1,3-dioxoisoindolin-5-yl) azetidine-3-carbonyl) piperazin-1-yl) phenyl) amino)-3-(piperidin-1-yl)-1,2,4-triazine-6-carboxamide **061**
(3-(5-(3-((4-(4-((6-carbamoyl-3-(piperidin-1-yl)-1,2,4-triazin-5-yl) amino) phenyl) piperidin-1-yl) methyl) pyrrolidin-1-yl)-1,3-dioxoisoindolin-2-yl)-2,6-dioxopiperidin-1-yl) methyl pivalate **062**
(3-(5-(3-((4-(4-((6-carbamoyl-3-(4-methylpiperazin-1-yl)-1,2,4-triazin-5-yl) amino) phenyl) piperidin-1-yl) methyl) pyrrolidin-1-yl)-1,3-dioxoisoindolin-2-yl)-2,6-dioxopiperidin-1-yl) methyl pivalate **063**
(3-(5-(3-((4-(4-((6-carbamoyl-3-(piperidin-1-yl)-1,2,4-triazin-5-yl) amino)-2-fluorophenyl) piperazin-1-yl) methyl) pyrrolidin-1-yl)-1,3-dioxoisoindolin-2-yl)-2,6-dioxopiperidin-1-yl) methyl pivalate **064**
(3-(5-(3-((4-(4-((5-carbamoyl-2-(piperidin-1-yl) pyrimidin-4-yl) amino) phenyl) piperidin-1-yl) methyl) pyrrolidin-1-yl)-1,3-dioxoisoindolin-2-yl)-2,6-dioxopiperidin-1-yl) methyl pivalate **065**
(3-(4-(4-((1-(4-((6-carbamoyl-3-(piperidin-1-yl)-1,2,4-triazin-5-yl) amino)-2-fluorophenyl) piperidin-4-yl) methyl) piperazin-1-yl) phenyl)-2,6-dioxotetrahydropyrimidin-1(2H)-yl) methyl pivalate **066**
5-((4-(1-((1-(2-(2,6-dioxopiperidin-3-yl)-1,3-dioxoisoindolin-5-yl) pyrrolidin-3-yl) methyl) piperidin-4-yl)-3-fluorophenyl) amino)-3-((R)-3-hydroxypiperidin-1-yl)-1,2,4-triazine-6-carboxamide **067**
(3-(5-(3-((4-(4-((6-carbamoyl-3-(piperidin-1-yl)-1,2,4-triazin-5-yl) amino)-2-fluorophenyl) piperidin-1-yl) methyl) pyrrolidin-1-yl)-1,3-dioxoisoindolin-2-yl)-2,6-dioxopiperidin-1-yl) methyl pivalate **068**
1-(3-(5-(3-((4-(4-((6-carbamoyl-3-(piperidin-1-yl)-1,2,4-triazin-5-yl) amino) phenyl) piperidin-1-yl) methyl) pyrrolidin-1-yl)-1,3-dioxoisoindolin-2-yl)-2,6-dioxopiperidin-1-yl) ethyl pivalate **069**
1-(3-(5-(3-((4-(4-((6-carbamoyl-3-(4-methylpiperazin-1-yl)-1,2,4-triazin-5-yl) amino) phenyl) piperidin-1-yl) methyl) pyrrolidin-1-yl)-1,3-dioxoisoindolin-2-yl)-2,6-dioxopiperidin-1-yl) ethyl pivalate **070**
1-(3-(5-(3-((4-(4-((6-carbamoyl-3-(piperidin-1-yl)-1,2,4-triazin-5-yl) amino)-2-fluorophenyl) piperazin-1-yl) methyl) pyrrolidin-1-yl)-1,3-dioxoisoindolin-2-yl)-2,6-dioxopiperidin-1-yl) ethyl pivalate **071**
1-(3-(4-(4-((1-(4-((6-carbamoyl-3-(piperidin-1-yl)-1,2,4-triazin-5-yl) amino)-2-fluorophenyl) piperidin-4-yl) methyl) piperazin-1-yl) phenyl)-2,6-dioxotetrahydropyrimidin-1(2H)-yl) ethyl pivalate **072**
(3-(5-(3-((4-(4-((6-carbamoyl-3-(piperidin-1-yl)-1,2,4-triazin-5-yl) amino) phenyl) piperidin-1-yl) methyl) pyrrolidin-1-yl)-1,3-dioxoisoindolin-2-yl)-2,6-dioxopiperidin-1-yl) methyl L-valinate **073**
(3-(5-(3-((4-(4-((6-carbamoyl-3-(piperidin-1-yl)-1,2,4-triazin-5-yl) amino)-2-fluorophenyl) piperazin-1-yl) methyl) pyrrolidin-1-yl)-1,3-dioxoisoindolin-2-yl)-2,6-dioxopiperidin-1-yl) methyl L-valinate **074**
5-((4-(4-((1-(4-(2,4-dioxotetrahydropyrimidin-1(2H)-yl)-3-methylphenyl) piperidin-4-yl) methyl) piperazin-1 -yl)-3-fluorophenyl)amino)-3-(piperidin-1-yl)-1,2,4-triazine-6-carboxamide **075**
5-((4-(4-((4-(4-(2,4-dioxotetrahydropyrimidin-1(2H)-yl)-3-methylphenyl) piperazin-1-yl) methyl) piperidin-1-yl)-3-fluorophenyl) amino)-3-(piperidin-1-yl)-1,2,4-triazine-6-carboxamide **076**
5-((4-(4-((1-(6-((2,6-dioxopiperidin-3-yl) amino) pyridin-3-yl) piperidin-4-yl) methyl) piperazin-1-yl)-3- fluorophenyl) amino)-3-(piperidin-1-yl)-1,2,4-triazine-6-carboxamide **077**
5-((4-(4-((1-(4-(2,4-dioxotetrahydropyrimidin-1(2H)-yl)-3-fluorophenyl)piperidin-4-yl) methyl) piperazin-1 -yl)-3-fluorophenyl) amino)-3-(piperidin-1-yl)-1,2,4-triazine-6-carboxamide **078**
5-((4-(4-((1-(6-((2,6-dioxopiperidin-3-yl) carbamoyl) pyridin-3-yl) pyrrolidin-3-yl) methyl) piperazin-1-yl)- 3-fluorophenyl) amino)-3-(piperidin-1-yl)-1,2,4-triazine-6-carboxamide **079**
5-((4-(4-((1-(4-(2,4-dioxotetrahydropyrimidin-1(2H)-yl)phenyl) piperidin-4-yl) methyl) piperazin-1-yl)-3- fluorophenyl) amino)-3-(piperidin-1-yl)-1,2,4-triazine-6-carboxamide **080**
5-((3-fluoro-4-(4-((1-(5-(6-oxo-1,6-dihydropyridazin-3-yl)-3-(trifluoromethyl) pyridin-2-yl) piperidin-4-yl) methyl) piperazin-1-yl) phenyl) amino)-3-(piperidin-1-yl)-1,2,4-triazine-6-carboxamide **081**
5-((4-(4-((1-(4-(2,4-dioxotetrahydropyrimidin-1(2H)-yl)-3-methoxyphenyl)piperidin-4-yl) methyl) piperazin-1-yl)-3-fluorophenyl) amino)-3-(piperidin-1-yl)-1,2,4-triazine-6-carboxamide **082**
5-((4-(4-((1-(4-((2,6-dioxopiperidin-3-yl) carbamoyl)-3-fluorophenyl) piperidin-4-yl) methyl) piperazin-1-yl) -3-fluorophenyl) amino)-3-(piperidin-1-yl)-1,2,4-triazine-6-carboxamide **083**
5-((4-(4-((1-(4-(2,4-dioxotetrahydropyrimidin-1(2H)-yl)-2-methylphenyl)piperidin-4-yl) methyl) piperazin-1 -yl)-3-fluorophenyl) amino)-3-(piperidin-1-yl)-1,2,4-triazine-6-carboxamide **084**
5-((4-(4-((1-(6-((2,6-dioxopiperidin-3-yl) carbamoyl) pyridin-3-yl) piperidin-4-yl) methyl) piperazin-1-yl)-3- fluorophenyl) amino)-3-(piperidin-1-yl)-1,2,4-triazine-6-carboxamide **085**
5-((4-(4-((1-(4-(2,4-dioxotetrahydropyrimidin-1(2H)-yl)-2-fluorophenyl)piperidin-4-yl) methyl) piperazin-1-yl)-3-fluorophenyl) amino)-3-(piperidin-1-yl)-1,2,4-triazine-6-carboxamide **086**
5-((3-fluoro-4-(4-((1-(4-(6-oxo-1,6-dihydropyridazin-3-yl)-2-(trifluoromethyl) phenyl) piperidin-4-yl) methyl) piperazin-1-yl) phenyl) amino)-3-(piperidin-1-yl)-1,2,4-triazine-6-carboxamide **087**
5-((4-(4-((1-(4-((2,6-dioxopiperidin-3-yl) amino)-3-fluorophenyl) piperidin-4-yl) methyl) piperazin-1-yl)-3- fluorophenyl) amino)-3-(piperidin-1-yl)-1,2,4-triazine-6-carboxamide **088**
5-((3-fluoro-4-(4-((4-(4-(6-oxo-1,6-dihydropyridazin-3-yl)-2-(trifluoromethyl) phenyl) piperazin-1-yl) methyl) piperidin-1-yl) phenyl) amino)-3-(piperidin-1-yl)-1,2,4-triazine-6-carboxamide **089**
(R)-3-(3-acetamidopiperidin-1-yl)-5-((4-(4-((1-(4-(2,4-dioxotetrahydropyrimidin-1(2H)-yl)-3-methylphenyl) piperidin-4-yl) methyl) piperazin-1-yl)-3-fluorophenyl) amino)-1,2,4-triazine-6-carboxamide **090**
(R)-3-(3-acetamidopiperidin-1-yl)-5-((4-(4-((1-(4-(2,4-dioxotetrahydropyrimidin-1(2H)-yl) phenyl) piperidin -4-yl) methyl) piperazin-1-yl)-3-fluorophenyl) amino)-1,2,4-triazine-6-carboxamide **091**
3-((R)-3-acetamidopiperidin-1-yl)-5-((4-(4-((1-(6-((2,6-dioxopiperidin-3-yl) carbamoyl) pyridin-3-yl) piperidin-4-yl) methyl) piperazin-1-yl)-3-fluorophenyl) amino)-1,2,4-triazine-6-carboxamide **092**
3-((R)-3-acetamidopiperidin-1-yl)-5-((4-(4-((1-(4-((2,6-dioxopiperidin-3-yl) carbamoyl)-3-fluorophenyl) piperidin-4-yl) methyl) piperazin-1-yl)-3-fluorophenyl) amino)-1,2,4-triazine-6-carboxamide **093**
(R)-3-(3-acetamidopiperidin-1-yl)-5-((3-fluoro-4-(4-((1-(5-(6-oxo-1,6-dihydropyridazin-3-yl)-3-(trifluoromethyl) pyridin-2-yl) piperidin-4-yl) methyl) piperazin-1-yl) phenyl) amino)-1,2,4-triazine-6-carboxamide **094**
(R)-3-(3-acetamidopiperidin-1-yl)-5-((3-fluoro-4-(4-((1-(4-(6-oxo-1,6-dihydropyridazin-3-yl)-2-(trifluoromethyl)phenyl) piperidin-4-yl) methyl) piperazin-1-yl) phenyl) amino)-1,2,4-triazine-6-carboxamide **095**
3-((R)-3-acetamidopiperidin-1-yl)-5-((4-(4-((1-(6-((2,6-dioxopiperidin-3-yl)amino) pyridin-3-yl) piperidin-4- yl) methyl) piperazin-1-yl)-3-fluorophenyl) amino)-1,2,4-triazine-6-carboxamide **096**
3-((R)-3-acetamidopiperidin-1-yl)-5-((4-(4-((1-(4-((2,6-dioxopiperidin-3-yl) amino)-3-fluorophenyl) piperidin-4-yl) methyl) piperazin-1-yl)-3-fluorophenyl) amino)-1,2,4-triazine-6-carboxamide **097**
5-((4-(4-((1-(4-(2,4-dioxotetrahydropyrimidin-1(2H)-yl)-3-methylphenyl)piperidin-4-yl) methyl) piperazin-1-yl)-3-fluorophenyl) amino)-3-morpholino-1,2,4-triazine-6-carboxamide **098**
5-((4-(4-((1-(4-(2,4-dioxotetrahydropyrimidin-1(2H)-yl)phenyl) piperidin-4-yl) methyl) piperazin-1-yl)-3- fluorophenyl) amino)-3-morpholino-1,2,4-triazine-6-carboxamide **099**
5-((4-(4-((1-(6-((2,6-dioxopiperidin-3-yl) carbamoyl) pyridin-3-yl) piperidin-4-yl) methyl) piperazin-1-yl)-3- fluorophenyl) amino)-3-morpholino-1,2,4-triazine-6-carboxamide **100**
5-((4-(4-((1-(4-((2,6-dioxopiperidin-3-yl) carbamoyl)-3-fluorophenyl) piperidin-4-yl) methyl) piperazin-1-yl) -3-fluorophenyl) amino)-3-morpholino-1,2,4-triazine-6-carboxamide **101**
5-((3-fluoro-4-(4-((1-(5-(6-oxo-1,6-dihydropyridazin-3-yl)-3-(trifluoromethyl) pyridin-2-yl) piperidin-4-yl) methyl) piperazin-1-yl) phenyl) amino)-3-morpholino-1,2,4-triazine-6-carboxamide **102**
5-((3-fluoro-4-(4-((1-(4-(6-oxo-1,6-dihydropyridazin-3-yl)-2-(trifluoromethyl) phenyl) piperidin-4-yl) methyl) piperazin-1-yl) phenyl) amino)-3-morpholino-1,2,4-triazine-6-carboxamide **103**
5-((4-(4-((1-(6-((2,6-dioxopiperidin-3-yl)amino) pyridin-3-yl) piperidin-4-yl) methyl) piperazin-1-yl)-3- fluorophenyl) amino)-3-morpholino-1,2,4-triazine-6-carboxamide **104**
5-((4-(4-((1-(4-((2,6-dioxopiperidin-3-yl) amino)-3-fluorophenyl) piperidin-4-yl) methyl) piperazin-1-yl)-3- fluorophenyl) amino)-3-morpholino-1,2,4-triazine-6-carboxamide **105**

### Description of Terminology

Unless otherwise defined, all technical and scientific terms used herein have the same meaning as commonly understood by those skilled in the art. Unless otherwise specified, all patent documents, publicly disclosed materials, etc. referred to in the present invention are incorporated herein in their entirety. If there are multiple definitions for the same term in the present invention, the definition in this section shall prevail.

It should be understood that the foregoing general description and the following detailed description are merely exemplary and explanatory, and are not interpreted as limitations on any claim. It should be noted that in the specification and the appended claims, singular references such as "a", "an" and "the" include plural references unless the context specifies otherwise. It should also be noted that "or" means "and/ or" unless specified otherwise. Furthermore, "comprise", "include", and similar terms are not limiting.

"Substituted" means that a hydrogen atom is substituted with a substituent. It should be noted that the substituent on a specific atom is constrained by its valence. In the definition part, "Cᵢ₋ⱼ" means a range including the starting point and the end point, where i and j are both integers, indicating the number of carbon atoms. For example, C₁₋₄, C₁₋₁₀, C₃₋₁₀, etc.

C, H, O, S, N, F, Cl, Br, I, *etc.* involved in the groups and compounds of the present invention all include their isotopes. At the same time, C, H, 0, S, N, F, Cl, Br, and I involved in the groups and compounds of the present invention may be optionally substituted with one or more of their corresponding isotopes, including but not limited to isotopes of carbon ²¹ C, ¹³C, and ¹⁴C, isotopes of hydrogen protium (H), deuterium (D), and tritium (T), isotopes of oxygen ¹⁶O, ¹⁷O, and ¹⁸O, isotopes of sulfur ³²S, ³³S, ³⁴S, and ³⁶S, isotopes of nitrogen ¹⁴N and ¹⁵N, isotopes of fluorine ¹⁷F and ¹⁹F, isotopes of chlorine ³⁵ Cl and ³⁷ Cl, isotopes of bromine ⁷⁹Br and⁸¹Br, *etc.*

The term "alkyl" used in the present invention means a straight or branched saturated hydrocarbon group containing 1 to 8 carbon atoms, including but not limited to methyl, ethyl, propyl, isopropyl, n-butyl, isobutyl, sec-butyl, tert-butyl, n-amyl, isoamyl, neoamyl, tert-amyl, n-hexyl, isohexyl, neohexyl, heptyl, isoheptyl, neoheptyl, octyl, isooctyl, etc. The alkyl can be substituted with one or more substituents. When there are multiple substitutions occurring, thesubstituents can be the same or different; the substituents are independently D (deuterium), oxo, halogen, cyano, nitro, hydroxy, amino, aminoalkyl, alkenyl, alkynyl, carboxyl, carboxylic ester
group, acyl, amido, methylsulfuryl, alkylamido, Ci-Cs alkyl, Ci-Cs alkoxy, Ci-Cs hydroxyalkyl, Ci-Cs alkylamino, haloC₁-C₈ alkyl, haloCi-Cs alkoxy, haloC₁-C₈hydroxyalkyl, haloCi-Cs alkylamino, C₃-C₁₂ cycloalkyl, haloC₃-C₁₂ cycloalkyl, cycloalkenyl, cycloalkynyl, alkoxycarbonyl, alkylthio, alkylsulfonyl, hydroxyalkylamido, sulfonamido, Spiralyl, C₆-C₁₂ aryl, C₅-C₁₄ heteroaryl, C₃-C₁₂heterocyclyl.

The term " alkenyl" " alkene" used in the present invention means a straight or branched hydrocarbon chain group containing 1 to 8 carbon atoms and at least one C=C double bond, including but not limited to vinyl, 1-propenyl, 2-propenyl, 1-butenyl, 2-butenyl, 3-butenyl, 1-pentenyl, 2-pentenyl, 3-pentenyl, 4-pentenyl, 1-methyl -1-butenyl, 1-methyl-2-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 1-methyl-2-hexenyl, 2-methyl-2-hexenyl, 2-methyl-3 -hexenyl, 3,5-dimethyl-2-hexenyl, 3,3-dimethyl-1-pentenyl, 3-methyl-2-ethyl-1-butenyl, 1-octenyl, 2-octenyl, *etc.* The alkenyl can be substituted with one or more substituents. When there are multiple substitutions occurring, the substituents can be the same or different; the substituents are independently D, alkyl, haloalkyl, alkoxy, haloalkoxy, hydroxyalkyl, halohydroxyalkyl, alkylamino, haloalkylamino, cycloalkyl, halocycloalkyl, heterocyclyl, aryl, heteroaryl, hydroxyl, halogen, cyano, nitro, amino, aminoalkyl, carboxyl, amido, sulfonamido, spiralyl.

The term " alkynyl" " alkyne" used in the present invention means a straight or branched hydrocarbon chain group containing 1 to 8 carbon atoms and at least one C≡C triple bond, including but not limited to ethynyl, 1-propynyl, 2-propynyl, 1-butynyl, 2-butynyl, 3-butynyl, 1-pentynyl, 2-pentynyl, 3-pentynyl, 4-pentynyl, 3-methyl -1-butynyl, 4-methyl-i-butynyl, 2-methyl-3-butynyl, 1-methyl-4-butynyl, 1-hexynyl, 2-hexynyl, 3-hexynyl, 4-hexynyl, 1-methyl-2-pentynyl, 1-methyl-3-pentynyl, 1-methyl-4-pentynyl, 2-methyl-3-pentynyl, 2,2-dimethyl-4- pentynyl, 1-heptynyl, 2-heptynyl, 3-heptynyl, 4-heptynyl, 5-heptynyl, 2-methyl-3-hexynyl, 3-methyl-1-hexynyl, 3,3-dimethyl-1-hexynyl, 4-methyl-1-hexynyl, *etc.* The alkynyl can be substituted with one or more substituents. When there are multiple substitutions occurring, the substituents can be the same or different; the substituents are independently D, alkyl, haloalkyl, alkoxy, haloalkoxy, hydroxyalkyl, halohydroxyalkyl, alkylamino, haloalkylamino, cycloalkyl, halocycloalkyl, heterocyclyl, aryl, heteroaryl, hydroxyl, halogen, cyano, nitro, amino, aminoalkyl, carboxyl, amido, sulfonamido, spiralyl.

The term "cycloalkyl""cycloalkane" used in the present invention means a monocyclic or polycyclic (two monocyclic rings connected by chemical bonds or bridged ring or spiro ring or fused ring) non-aromatic monovalent hydrocarbon group having 3 to 12 carbon atoms, and possibly one or more chemical bonds that are double or triple bonds. "Cycloalkyl" includes, but is not limited to, cyclopropyl, cyclobutyl, cyclopentyl, cyclohexyl, cycloheptyl, cyclooctyl, cyclononyl, cyclodecyl, Octahydroindene, decalin, bicyclo [1.1.0] butylyl, bicyclo[2.1.0] pentyl, bicyclo[2.2.0] exyl, bicyclo[3.2.0]heptyl, bicyclo[4.2.0]octyl, bicyclo[1.1.1] pentyl, bicyclo[2.1.1] exyl, bicyclo[3.1.1]heptyl, bicyclo[2.2.1]heptyl, bicyclo[4.1.1]octyl, bicyclo[3.2.1]octyl, bicyclo[5.1.1]nonyl, bicyclo[4.2.1]nonyl, bicyclo[4.3.1]nonyl, bicyclo[3.2.2]nonyl, bicyclo[5.2.1]decyl, bicyclo[4.2.2]decyl, spiro [2.2] penty, spiro [2.3] exyl, spiro [2.4] heptyl, spiro [2.5] octyl, spiro [2.6] nonyl, spiro [3.5] nonyl, spiro [3.4] octyl, spiro [3.3] heptyl, spiro [4.5] decyl, spiro [4. 4] nonyl, *etc.* The cycloalkyl can be substituted with one or more substituents. When there are multiple substitutions occurring, the substituents can be the same or different; the substituents are independently D (deuterium), oxo, halogen, cyano, nitro, hydroxyl, amino, aminoalkyl, alkenyl, alkynyl, carboxyl, carboxylic ester group, acyl, amido, methylsulfuryl, alkylamido, C₁-C₈ alkyl, C₁-C₈ alkoxy, C₁-C₈ hydroxyalkyl, C₁-C₈alkylamino, haloC₁-C₈ alkyl, haloCi-Cs alkoxy, haloC₁-C₈hydroxyalkyl, haloC₁-C ₈alkylamino, C₃-C₁₂ cycloalkyl, haloC₃-C₁₂ cycloalkyl, cycloalkenyl, cycloalkynyl, alkoxycarbonyl, alkylthio, alkylsulfonyl, hydroxyalkylamido, sulfonamido, spiralyl, C₆-C₁₂ aryl, C₅-C₁₄ heteroaryl, C₃-C₁₂ heterocyclyl.

The term "cycloalkenyl" " cycloalkene " used in the present invention means a monocyclic or polycyclic (two monocyclic rings connected by chemical bonds or bridged ring or spiro ring or fused ring) non-aromatic monovalent hydrocarbon group having 3 to 12 carbon atoms and at least one C=C double bond, including but not limited to cyclopropenyl, cyclobutenyl, cyclopentenyl, cyclohexenyl, cycloheptenyl, cycloalkenyl, spiro[2.2]pent -1-enyl, spiro[2.2]pent-1,4-dienyl, spiro[2.3]hex-1-enyl, spiro[2.3]hex-1,4-dienyl, spiro[3.3]hept-1-enyl, spiro[3.3] hept-1,5-dienyl, spiro[3.4]oct-1-enyl, spiro[3.4]oct-1,6-dienyl, spiro[3.4]oct-5-enyl, spiro[3.4]oct-6-enyl, spiro[3.4] oct-1-enyl, bicyclo[2.1.1]hex-1-enyl, bicyclo[2.1.1]hex-2-enyl, bicyclo[3.1.1]hept 1-enyl, bicyclo[3.1.1]hept-2- enyl, bicyclo[2.2.1]hept-1-enyl, bicyclo[2.2.1]hept-2-enyl, etc. The cycloalkene or cycloalkenyl can be substituted with one or more substituents. When there are multiple substitutions occurring, the substituents can be the same or different; the substituents are independently D (deuterium), oxo, halogen, cyano, nitro, hydroxyl, aminoalkyl, alkenyl, alkynyl, carboxyl, carboxylic ester group, acyl, amido, methylsulfuryl, alkylamido, C₁-C₈ alkyl, C₁-C₈ alkoxy, C₁-C₈ hydroxyalkyl, C₁-C₈ alkylamino, haloC₁-C₈ alkyl, haloC₁-C₈ alkoxy, haloCi-Cs hydroxyalkyl, haloCi-Cs alkylamino, C₃-C₁₂ cycloalkyl, haloC₃-C₂ cycloalkyl, cycloalkenyl, cycloalkynyl, alkoxycarbonyl, alkylthio, alkylsulfonyl, hydroxyalkylamido, sulfonamido, spirally, C₆-C₁₂ aryl, C₅-C₁₂ heteroaryl, C₃-C₁₂ heterocyclyl.

The term "cycloalkyne ""cycloalkyne group" used in the present invention means a monocyclic or polycyclic (two monocyclic rings connected by chemical bonds or bridged ring or spiro ring or fused ring) non-aromatic monovalent hydrocarbon group having 4 to 12 carbon atoms, and contains at least one triple bonds, preferably as cyclobutyylene, cyclopentynyl, cyclohexynyl, etc, one or two carbon atoms can be replaced by one oxygen atom.The cycloalkyne group can be substituted or substituted, the substituents are independently D (deuterium), oxo, halogen, cyano, nitro, hydroxyl, aminoalkyl, alkenyl, alkynyl, carboxyl, carboxylic ester group, acyl, amido, methylsulfuryl, alkylamido, C₁-C₈ alkyl, C₁-C₈ alkoxy, C₁-C₈ hydroxyalkyl, C₁-C₈ alkylamino, haloC₁-C₈ alkyl, haloCi-Cs alkoxy, haloCi-Cs hydroxyalkyl, haloCi-Cs alkylamino, C₃-C₁₂ cycloalkyl, haloC₃-C₁₂ cycloalkyl, cycloalkenyl, cycloalkynyl, alkoxycarbonyl, alkylthio, alkylsulfonyl, hydroxyalkylamido, sulfonamido, C₆-C₁₂ aryl, C₅-C₁₂ heteroaryl, C₃-C₁₂ heterocyclyl.

The term "halogen" used in the present invention means fluorine, chlorine, bromine and iodine, preferably, fluorine, chlorine and bromine.

The term "alkoxy" used in the present invention means alkyl -O-, in which the alkyl is as defined above. Examples of "alkoxy" used in the present invention include, but are not limited to, methoxy, ethoxy, n-propoxy, isopropoxy, n-butoxy, sec-butoxy, tert-butoxy, pentoxy, isopentyloxy, neopentyloxy, etc. "Alkoxy" also includes substituted alkoxy, the substituents of which may be D, halogen, oxo, amino, hydroxyl, cyano, nitro, carboxyl, amido, sulfonamido, C₁-C₈ alkyl, C₁-C₈ hydroxyalkyl, C₁-C₈ alkoxy, C₁-C₈ alkylamino, haloC₁-C₈ alkyl, haloC₁-C₈hydroxyalkyl, haloC₁-C₈ alkoxy, haloC₁-C₈ alkylamino, C₃-C₁₂ cycloalkyl, C₃-C₁₂ heterocyclyl, C₆-C₁₂aryl, C₅-C₁₄heteroaryl.

The term "hydroxyalkyl" used in the present invention means -alkyl-OH, in which the alkyl is as defined above. Examples of "hydroxyalkyl" used in the present invention include, but are not limited to, hydroxymethyl, hydroxyethyl, hydroxypropyl, hydroxyisopropyl, etc. "Hydroxyalkyl" also includes substituted hydroxyalkyl, the substituents of which may be D, halogen, oxo, amino, hydroxy, cyano, nitro, carboxyl, amido, sulfonamido, C₁-C₈ alkyl, C₁-C₈ hydroxyalkyl, C₁-C₈ alkoxy, C₁-C₈ alkylamino, haloC₁-C₈ alkyl, haloC₁-C₈hydroxyalkyl, haloC₁-C₈ alkoxy, haloC₁-C₈ alkylamino, C₃-C₁₂ cycloalkyl, C₃-C₁₂ heterocyclyl, C₆-C₁₂ aryl, Cs-Ciaheteroaryl.

The term "alkylamino" used in the present invention means -NH-alkyl, in which the alkyl is as defined above. Examples of "alkylamino" used in the present invention include, but are not limited to, methylamino, ethylamino, propylamino, isopropylamino, etc. "Alkylamino" also includes substituted alkylamino, the substituents of which may be D, halogen, oxo, amino, hydroxyl, cyano, nitro, carboxyl, amido, sulfonamido, spirally, C₁-C₈ alkyl, C₁-C₈ hydroxyalkyl, C₁-C₈ alkoxy, C₁-C₈ alkylamino, haloC₁-C₈ alkyl, haloC₁-C₈hydroxyalkyl, haloC₁-C₈ alkoxy, haloC₁-C₈ alkylamino, C₃-C₁₂ cycloalkyl, C₃-C₁₂ heterocyclyl, C₆-C₁₂ aryl, C₅-C₁₄ heteroaryl, and the substituents of which can be on alkyl or on N.

The term "aminoalkyl" used in the present invention means -alkyl-NH₂, in which the alkyl is as defined above. Examples of "aminoalkyl" used in the present invention include, but are not limited to, aminomethyl, aminoethyl, aminopropyl, aminoisopropyl, etc. "Aminoalkyl" also includes substituted aminoalkyl, the substituents of which may be D, halogen, oxo, amino, hydroxyl, cyano, nitro, carboxyl, amido, sulfonamido,spiralyl C₁-C₈ alkyl, C₁-C₈ hydroxyalkyl, C₁-C₈ alkoxy, C₁-C₈ alkylamino, haloC₁-C₈ alkyl, haloC₁-C₈hydroxyalkyl, haloC₁-C₈ alkoxy, haloCi-Cs alkylamino, C₃-C₁₂ cycloalkyl, C₃-C₁₂ heterocyclyl, C₆-C₁₂ aryl,C₅-C₁₄ heteroaryl, and the substituents of which can be on alkyl or on N.

The term "Cycloalkoxy" used in the present invention means cycloalkyll substituted with One or more carbon atom are replaced by one or more oxygen, in which the "cycloalkyl" is as defined above. Examples of the "alkylamino" used in the present invention include but are not limited to oxyheterocyclic butane, tetrahydrofuran, tetrahydropyran, dioxane, and so like.

The term "aromatic ring" "aryl" used in the present invention means an all-carbon monocyclic or fused polycyclic aromatic ring group (in the case of fused polycyclic rings, one of the fused rings can be partially saturated) having 6 to 12 carbon atoms, including but not limited to benzene ring, naphthalene ring, anthracene ring, indene ring, dihydroindenyl (indanyl), dihydronaphthalene ring, tetrahydronaphthalene ring, etc. Aryl can be unsubstituted or substituted, can be monosubstituted (such as ortho-, meta-, or para-substituted), can also be di-substituted or tri-substituted, etc. When there are multiple substitutions occurring, the substituents can be the same or different; the substituents are independently D (deuterium), halogen, cyano, nitro, hydroxyl, amino, aminoalkyl, alkenyl, alkynyl, carboxyl, acyl, amido, alkylamido, C₁-C₈ alkyl, C₁-C₈ alkoxy, C₁-C₈ hydroxyalkyl, C₁-C₈ alkylamino, haloC₁-C₈ alkyl, haloC₁-C₈ alkoxy, haloC₁-C₈ hydroxyalkyl, haloC₁-C₈ alkylamino, C3-C12 cycloalkyl, haloC₃-C₁₂ cycloalkyl, alkoxycarbonyl, alkylthio, alkylsulfonyl, hydroxyalkylamido, sulfonamido, spiralyl, C₆-C₁₂ aryl, C₅-C₁₄ heteroaryl, C₃-C₁₂ heterocyclyl.

The term "heteroaryl" used in the present invention means a monocyclic or fused polycyclic aromatic ring group (in the case of fused polycyclic rings, one of the fused rings can be partially saturated) having 5 to 14 ring atoms, which is equivalent to that one or more carbons in the above "aryl" are replaced by heteroatoms such as N, 0, S, etc. Heteroaromatic rings can bemonocyclic ring or bicyclic ring, i.e., formed by the fusion of two rings. Heteroaryl includes, but are not limited to, pyridyl, pyrimidinyl, pyrazinyl, pyridazinyl, triazinyl, isoxazolyl, isothiazolyl, pyrazolyl, thiazolyl, thienyl, furanyl, triazolyl, oxazolyl, imidazolyl, indazolyl, indolizinyl, indolyl, dihydroindolyl, isoindolinyl, quinolyl, isoquinolyl, quinoxalinyl, quinazolinyl, dihydroquinolinyl, tetrahydroquinolinyl, dihydroisoquinolinyl, dihydroisoquinolinyl, tetrahydroisoquinolinyl, pteridinyl, purinyl, benzoimidazolyl, benzofuranyl, benzothienyl, benzothiazolyl, benzotriazolyl, benzotriazinyl, benzoxadiazolyl, benzoxazolyl, benzoisoxazolyl, imidazopyridyl, imidazothiazolyl, pyrrolopyridyl, thienopyrrolyl, thienothienyl, thienopyridyl, thienopyrimidinyl, pyrazolopyrimidinyl, pyrrolopyrrolyl, pyrrolopyrimidinyl, pyrrolopyridazinyl, pyrrolopyrazinyl, imidazopyrimidinyl, imidazopyrazinyl, imidazopyridazinyl, pyrazolopyridyl, pyrazolopyridazinyl, pyrazolopyrazinyl, pyrimidopyridyl, pyrimidopyrazinyl, pyrimidopyridazinyl, pyrimidopyrimidinyl, pyridopyridyl, pyridopyrazinyl, pyridopyridazinyl, pyridazinopyridazinyl, pyridazinopyrazinyl, pyrazinopyrazinyl, etc. Heteroaryl can be unsubstituted or monosubstituted or polysubstituted. In the case of polysubstitution, the substituents can be the same or different; the substituents are independently D (deuterium), halogen, cyano, nitro, amino, aminoalkyl, hydroxyl, carboxyl, carboxylic ester group, acyl, amido, alkylamido, alkoxycarbonyl, alkylthio, alkylsulfonyl, hydroxyalkylamido, sulfonamido, Ci-Cs alkyl, Ci-Cs hydroxyalkyl, Ci-Cs alkoxy, Ci-Cs alkylamino, haloC₁-C₈ alkyl, haloC₁-C₈ hydroxyalkyl, haloC₁-C₈ alkoxy, haloC₁-C₈ alkylamino, C₃-C₁₂ cycloalkyl, haloC₃-C₁₂ cycloalkyl, C₃-C₁₂heterocyclyl, C₆-C₁₂ aryl, C₅-C₁₄ heteroaryl.

The term "heterocyclyl" used in the present invention means a monocyclic or polycyclic (two monocyclic rings connected by chemical bonds or bridged ring or spiro ring or fused ring) non-aromatic ring group having 3 to 12 ring atoms, one or more heteroatoms selected from N, O, S, and possibly one or more chemical bonds that are double or triple bonds. Heterocyclyl, includes but are not limited to, pyranyl, piperidinyl, piperazinyl, morpholinyl, dioxanyl, oxacyclopropyl, oxacyclobutyl, oxacyclohexyl, oxacycloheptyl, oxacyclooctyl, azacyclopropyl, azacyclobutyl, azacyclopentyl, azacycloheptyl, azacyclooctyl, thiocyclobutyl, thiocyclopropyl, azacyclooctane group, oxazepinyl, diazepinyl, thiazepinyl, dihydrofuranyl, dihydrothienyl, dihydropyranyl, tetrahydrofuranyl, tetrahydrothienyl, tetrahydropyranyl, tetrahydrothiopyranyl, tetrahydrothiazolyl, tetrahydroimidazolyl, hexahydropyridazinyl, hexahydropyrimidinyl, 1-azaspiro [2.2]pentyl, 1-azaspiro[2.3]hexyl, 4-azaspiro[2.3]hexyl, 5-azaspiro[2.3]hexyl, 2-azaspiro[3.3]heptyl, 2,6-diazaspiro[3.3]heptyl, 2-oxa-6-azaspiro[3.3]heptane, 1-azaspiro [2.5] octyl, 2-azaspiro[3.4]octyl, 6-azaspiro[3.4]octyl, 2,6-diazaspiro[3.4]octyl, 2-azaspiro[3.5]nonyl,6-azaspiro [3.5]nonyl, 7-azaspiro[3.5]nonyl, 2,7-diazaspiro[3.5]nonyl, 2-oxa-7-azaspiro[3.5] nonyl, 1-azaspiro[4.4]nonyl, 2-azaspiro[4.4]nonyl, 8-azaspiro[4.5]decyl, 2,8-diazaspiro[4.5] decyl, 1-oxaspiro[2.2]pentyl, 1-oxaspiro[2.3]hexyl, 4-oxaspiro[2.3]hexyl, 5-oxaspiro[2.3]hexyl, 2-oxaspiro[3.3]heptyl, 1-oxaspiro[2.5]octyl, 2-oxaspiro[3.4]octyl, 6-oxaspiro[3.4]octyl, 2-oxaspiro[3.5]nonyl, 6-oxaspiro[3.5]nonyl, 7-oxaspiro[3.5]nonyl, 1-oxaspiro[4.4]nonyl, 2-oxaspiro[4.4]nonyl, 8-oxaspiro[4.5]decyl, decahydroquinolyl, decahydroisoquinolyl, 2-azabicyclo[1.1.1]pentyl, 2-oxabicyclo[1.1.1]pentyl, azabicyclo[2.1.1]hexyl, oxabicyclo[2.1.1]hexyl, azabicyclo[3.1.1]heptyl, oxabicyclo [3.1.1]heptyl, azabicyclo[2.2.1]heptyl, azabicyclo[4.1.1]octyl, azabicyclo[3.2.1]octyl, azabicyclo[3.2.1]octyl, etc. The heterocyclyl can be substituted with one or more substituents. When there are multiple substitutions occurring, the substituents can be the same or different; the substituents are independently D (deuterium), oxo, halogen, cyano, nitro, hydroxyl, amino, aminoalkyl, alkenyl, alkynyl, carboxyl, carboxylic ester group, acyl, amido, methylsulfuryl, alkylamido, C₁-C₈ alkyl, C₁-C₈alkoxy, C₁-C₈ hydroxyalkyl, C₁-C₈ alkylamino, haloC₁-C₈ alkyl, haloC₁-C₈ alkoxy, haloC₁-C₈hydroxyalkyl, haloC₁-C₈ alkylamino, C₃-C₁₂ cycloalkyl, haloC₃-C₁₂ cycloalkyl, cycloalkenyl, cycloalkynyl, alkoxycarbonyl, alkylthio, alkylsulfonyl, hydroxyalkylamido, sulfonamido, C₆-C₁₂aryl, C₅-C₁₄heteroaryl, C₃-C₁₂heterocyclyl.

The terms "spiro ring" and "spiro ring group" used in the present invention mean a polycyclic structure, in which at least two rings share one atom (usually a C atom). In this polycyclic structure, one or more chemical bonds can be double bonds or triple bonds, and one or more heteroatoms can be present. The spiro ring group can be unsubstituted or monosubstituted or polysubstituted. In the case of polysubstitution, the substituents can be the same or different; the substituents are independently D (deuterium), oxo, halogen, cyano, nitro, hydroxyl, amino, aminoalkyl, alkenyl, alkynyl, carboxyl, carboxylic ester group, acyl, amido, methylsulfuryl, alkylamido, C₁-C₈ alkyl, C₁-C ₈alkoxy, C₁-C₈ hydroxyalkyl, C₁-C₈ alkylamino, haloC₁-C₈ alkyl, haloC₁-C₈ alkoxy, haloC₁-C₈hydroxyalkyl, haloC₁-C₈ alkylamino, C₃-C₁₂ cycloalkyl, haloC₃-C₁₂ cycloalkyl, cycloalkenyl, cycloalkynyl, alkoxycarbonyl, alkylthio, alkylsulfonyl, hydroxyalkylamido, sulfonamido, spiralyl, C₆-C₁₂ aryl, C₅-C₁₄ heteroaryl, C₃-C₁₂ heterocyclyl.

The term "bridged ring group" used in the present invention means a polycyclic structure, in which at least two rings share two or more atoms. In this polycyclic structure, one or more chemical bonds can be double bonds or triple bonds, and one or more heteroatoms can be present. The bridged ring group can be unsubstituted or monosubstituted or polysubstituted. In case of polysubstitution, the substituents can be the same or different; the substituents are independently D (deuterium), oxo, halogen, cyano, nitro, hydroxyl, amino, aminoalkyl, alkenyl, alkynyl, carboxyl, carboxylic ester group, acyl, amido, methylsulfuryl, alkylamido, C₁-C₈ alkyl, C₁-C salkoxy, C₁-C₈ hydroxyalkyl, C₁-C₈ alkylamino, haloC₁-C₈ alkyl, haloC₁-C₈ alkoxy, haloC₁-C₈hydroxyalkyl, haloC₁-C₈ alkylamino, C₃-C₁₂ cycloalkyl, haloC₃-C₁₂ cycloalkyl, cycloalkenyl, cycloalkynyl, alkoxycarbonyl, alkylthio, alkylsulfonyl, hydroxyalkylamido, sulfonamido, C₆-C₁₂aryl, C₅-C₁₄heteroaryl, C₃-C₁₂heterocyclyl.

The term "haloalkyl" used in the present invention means a straight or branched alkyl group substituted with halogens (preferably fluorine, chlorine, bromine, iodine), in which the "alkyl" is as defined above. Examples of "haloalkyl" used in the present invention include, but are not limited to, fluoromethyl, difluoromethyl, trifluoromethyl, chloromethyl, dichloromethyl, trichloromethyl, 2,2,2-trifluoroethyl, 2-fluoroethyl, 2-chloroethyl, tetrafluoroethyl, pentafluoroethyl, 1,1,1-trifluoroprop-2-yl, *etc.*

The term "halocycloalkyl" used in the present invention means halocycloalkyl substituted with One or more hydrogens are replaced by one or more halogens (preferably fluorine, chlorine, bromine, iodine), in which the "cycloalkyl" is as defined above.

The salts of the compound of the invention may be prepared by a method known to those skilled in the art.

"Pharmaceutically acceptable salt" refers to a salt formed by the compound of the present invention with an acid or a base, and is suitable for use as a dug. Pharmaceutically acceptable salts include inorganic salts and organic salts. One preferred class of salts are the salts formed by the compounds of the invention with acids. Suitable salt-forming acids include, but are not limited to: inorganic acids such as hydrochloric acid, hydrobromic acid, hydrofluoric acid, hydroiodic acid, sulfuric acid, nitric acid, phosphoric acid, carbonic acid, organic acids such as
formic acid, acetic acid, trifluoroacetic acid, propionic acid, oxalic acid, malonic acid, succinic acid, fumaric acid, maleic acid, lactic acid, malic acid, tartaric acid, citric acid, picric acid, methanesulfonic acid, p-toluenesulfonic acid, ethanesulfonic acid, benzenesulfonic acid, camphorsulfonic acid, 2-O-β-D-glucopyranosyl-L-ascorbic acid, isonicotinic acid, salicylic acid, ascorbic acid, gentisic acid, gluconic acid, pyruvic acid, naphthalenesulfonic acid, stearic acid, phenylacetic acid, p-aminobenzenesulfonic acid, isethionic acid, pamoic acid, tannic acid; and acidic amino acids such as aspartic acid,glutamic acid. One preferred class of salts are the salts formed by the compounds of the invention with bases. Suitable salt-forming bases include, but are not limited to: inorganic bases such as sodium hydroxide, potassium hydroxide, sodium carbonate, sodium bicarbonate, and sodium phosphate, and organic bases such as ammonia, riethylamine, diethylamine, piperazine, guanidine, and diethanolamine.

The second object of the present invention is to provide a pharmaceutical composition including one or more of the compounds described in any of the above technical solutions. The pharmaceutical composition of the present invention can be composed of one or more of the compounds described in any of the above technical solutions and other compounds, or composed of one or more of the compounds described in any of the above technical solutions.

The present invention provides a pharmaceutical preparation, which comprises at least one active component, and the active component is one or more of the compounds described in any of the above technical solutions. The pharmaceutical preparation comprises at least one active component and one or more pharmaceutically acceptable carriers or excipients. The active component can be any one or more of the BTK inhibitor compound of the present invention, an optical isomer of the compound, a pharmaceutically acceptable salt of the compound or its optical isomer, a solvate of the compound or its optical isomer.

The carriers include conventional diluents, excipients, fillers, binders, wetting agents, disintegrants, absorption accelerators, surfactants, adsorption carriers, lubricants, etc. in the pharmaceutical field, and when necessary, flavoring agents, sweeteners, etc. can also be added.

The drug of the present invention can be formulated into various forms such as tablets, powders, granules, capsules, oral liquids, and injections. The drugs in each of the above dosage forms can be formulated according to conventional methods in the pharmaceutical field.

On the other hand, the present invention provides the use of the compound of the general formula I to formula III disclosed herein and its optical isomers or pharmaceutically acceptable salts or solvates thereof to inhibit Bruton's tyrosine kinase (Btk) activity, or to treat diseases, disorders, or conditions that would benefit from the inhibition of Bruton's tyrosine kinase (Btk) activity.

In a further preferred embodiment, the present invention provides a method for inhibiting the Bruton's tyrosine kinase activity in a subject in need by administering a composition containing a therapeutically effective amount of at least one compound to the subject, wherein the structural formula of the compound is set forth in the general formula I to formula III. In some embodiments, the subject in need thereof suffers from an autoimmune disease, such as inflammatory bowel disease, arthritis, lupus, rheumatoid arthritis, psoriatic arthritis, osteoarthritis, Still's disease, juvenile arthritis, diabetes, myasthenia gravis, Hashimoto's thyroiditis, Ord's thyroiditis, Graves' disease, Sgögren's syndrome, multiple sclerosis, Guillain-Barré syndrome, acute disseminated encephalomyelitis, Addison's disease, optic opsoclonus-myoclonus syndrome, ankylosing spondylitis, antiphospholipid antibody syndrome, aplastic anemia, autoimmune hepatitis, coeliac disease, Goodpasture's syndrome, immune thrombocytopenic purpura (ITP), optic neuritis, scleroderma, primary biliary cirrhosis, Reiter's syndrome, Takayasu's arteritis, temporal arteritis, warm autoimmune hemolytic anemia, Wegener's granulomatosis, psoriasis, alopecia universalis, Behçet's disease, chronic fatigue, familial dysautonomia, endometriosis, interstitial cystitis, neuromyotonia, scleroderma or vulvodynia and chronic graft-versus-host disease (cGvHD).

In a further embodiment, the subject in need suffers from a cancer. In one embodiment, the cancer is a B cell or plasma cell proliferative disease, such as diffuse large B cell lymphoma, follicular lymphoma, chronic lymphocytic lymphoma, chronic lymphocytic leukemia, B cell prolymphocytic leukemia, lymphoplasmacytic lymphoma/Waldenström macroglobulinemia, splenic marginal zone lymphoma, plasma cell myeloma, plasmacytoma, extranodal marginal zone B cell lymphoma, lymph node marginal zone B cell lymphoma, mantle cell lymphoma, mediastinum (thymus) large B cell lymphoma, intravascular large B cell lymphoma, primary effusion lymphoma, Burkitt lymphoma/leukemia, multiple myeloma, or lymphomatoid granulomatosis.

The present invention also provides the use of the compounds of the present invention or pharmaceutically acceptable salts thereof in the preparation of BTK inhibitors, especially their use in the treatment of cell proliferation diseases. The cell proliferative diseases include cancers. In other words, the invention also provides the use of the compound of the general formula I to formula III, its optical isomers or pharmaceutically acceptable salts or solvates thereof alone or in combination with other drugs in the treatment of proliferative diseases (such as cancers). Antitumor drugs that can be used in combination with the compounds provided by the present invention or their pharmaceutically acceptable salts thereof include, but are not limited to, at least one of the following types: mitotic inhibitors (such as vinblastine, vindesine, and vinorelbine); tubulin breakdown inhibitors (such as Taxol)); alkylating reagents (such as cisplatin, carboplatin and cyclophosphamide); antimetabolites (such as 5-fluorouracil, tegafur, methotrexate, cytarabine, and hydroxyurea); antibiotics can be inserted (such as doxorubicin, mitomycin and bleomycin)); enzymes (such as aspartase); topoisomerase inhibitors (such as etoposide and camptothecin); biological response modifiers (such as interferons); immune checkpoint inhibitors (such as PD-1 inhibitors, PD-L inhibitors, CTLA-4 inhibitors); CD20 monoclonal antibody; BCL2 inhibitors.

The inventors of the present invention have confirmed through experiments that the compounds of the present invention have a degradation activity against BTK.

The inventors of the present invention have confirmed through experiments that the compounds of the present invention have anti-proliferation inhibitory effects on Mino, OCI-LY10 tumor cell lines.

The inventors of the present invention have confirmed through experiments that the compounds of the present invention are metabolically stable in the liver microparticles.

The inventors of the present invention have confirmed through experiments that the compounds of the present invention may be taken orally.

The inventors of the present invention have confirmed through experiments that the compounds of the present invention have a better effect on tumor growth inhibition in the in vivo efficacy model of OCI-LY10.

### DETAILED DESCRIPTION OF EMBODIMENTS

### Intermediate 1: 4-(4-((6-carbamoyl-3-(methylthio)-1,2,4-triazin-5-yl) amino)-2-fluorophenyl) piperidine-1-carbox late tert-butyl ester

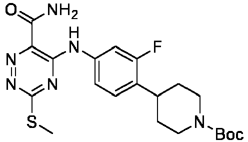

Ethyl 5-chloro-3-(methylthio)-1,2,4-triazine-6-carboxylic acid ethyl ester (760 mg, 3.40 mmol) was dissolved in 10 mL of acetonitrile, and 4-(4-amino-2-fluorophenyl)piperidin-1-carboxylic acid tert-butyl ester (1.0g, 3.40 mmol) and N,N-diisopropylethylamine (1.2mL, 6.80mmol) were added to the system for a reaction of 0.5h at room temperature, and then methanol solution of ammonia (25mL, 1mmol/mL) was added to the system. React at room temperature for 0.5h. After the end of the reaction, water was added and filtered, and 1.3g of intermediate 1 solid was obtained by reducing pressure and concentration, with a yield of 86%. ESI-MS(M+H)⁺=463.

### Intermediate 2: 4-(4-((6-carbamoyl-3-(methylthio)-1,2,4-triazin-5-yl) amino) phenyl) piperidin-1-carboxylate tert-butyl ester

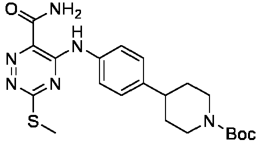

Referring to the synthesis route and method of intermediate 1, the tert-butyl ester of 4-(4-amino-2- fluorophenyl) piperidin-1-carboxylic acid was replaced with 4-(4-aminophenyl) piperidin-1-carboxylic acid tert-butyl ester to synthesize intermediate 2. ESI-MS(M+H) ⁺=445.

### Intermediate 3: 4-(4-((5-carbamoyl-2-(methylthio) pyrimidin-4-yl) amino)-2- fluorophenyl) piperidine-1- carboxylate tert-butyl ester

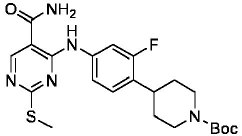

Referring to the synthesis route and method of intermediate 1, ethyl 5-chloro-3-( methylthio)-1,2,4- triazine -6-carboxylic acid was replaced with ethyl 4-chloro-2-methylthiopyrimidine-5-carboxylic acid to synthesize intermediate 3. ESI-MS(M+H) ⁺=462.

**Referring to the synthesis route and method of intermediate 1, the following intermediate structure is obtained:**

| Intermediate No. | Intermediate structure | LC-MS (ESI-MS) *m*/*z* [M+H] ⁺ |
|---|---|---|
| 4 | 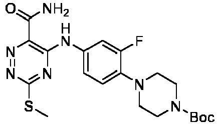 | 464 |
| 5 | 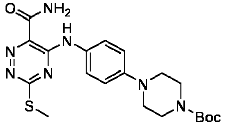 | 446 |
| 6 | 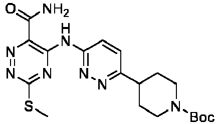 | 447 |
| 7 | 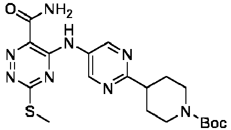 | 447 |
| 8 | 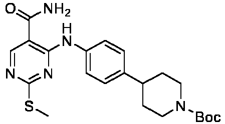 | 444 |
| 9 | 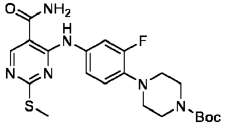 | 463 |
| 10 | 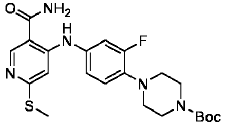 | 462 |
| 11 | 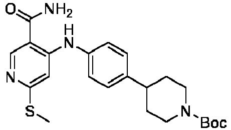 | 443 |
| 12 | 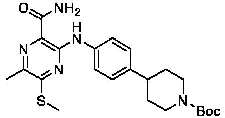 | 458 |
| 13 | 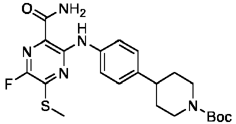 | 462 |
| 14 | 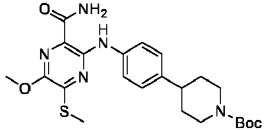 | 474 |
| 15 | 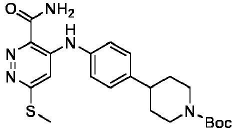 | 444 |

### Intermediate 16: 5-((3-fluoro-4-(4-formylpiperidin-1-yl)phenyl) amino)-3-(piperidin-1-yl)- 1, 2, 4 - triazine-6-carboxamide

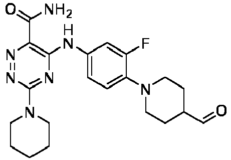

### Synthesis step 1: 1-(2-fluoro-4-nitrophenyl) piperidin-4-carboxaldehyde (intermediate 16-1)

Tert-butyl 4-formipperidine-1-carboxylic acid (7.3 g, 34.27 mmol) was dissolved in 20 mL of DCM, trifluoroacetic acid (30 mL) was added to the system, and stirred for one hour at room temperature. At the end of the reaction, it was concentrated under reduced pressure to obtain yellow liquid piperidinine-4-carboxaldehyde. The above products were dissolved in 50 mL of DMF, and cesium carbonate (3.0 g, 9.21 mmol) and 2,4- difluoro -1-nitrobenzene (5.5 g, 31.44 mmol) were added to the system for 2 h at 90°C. At the end of the reaction, water was added to quench it, EA was extracted three times, organic phases were combined, anhydrous sodium sulfate was dried, and concentrated under reduced pressure to obtain 7.5g of yellow liquid 1-(2-fluoro-4-nitrophenyl) piperidin -4-carboxaldehyde with a yield of 94%. ESI-MS(M+H)⁺ =253.

### Synthesis step 2: 4-(1,3-dioxanecyclo-2-yl)-1-(2-fluoro-4-nitrophenyl) piperidine (intermediate 16-2)

Intermediate 16-1 (7.5 g, 29.64 mmol) was dissolved in 50 mL of toluene, and p-toluenesulfonic acid (563 mg, 2.96 mmol) and ethylene glycol (18 mL, 296.40 mmol) were added to the system for 12 h at 120°C. End of reaction. Water was added to quench, concentrated under reduced pressure, solid precipitation was precipitated by adding water, and filtration was obtained to obtain 7 g of brown-black solid 4-(1,3-dioxanecyclo-2-yl) -1-(2- fluoro-4-nitrophenyl) piperidine in a yield of 80%. ESI-MS(M+H)⁺=297.

### Synthesis step 3: 4-(4-(1,3-dioxanecyclo-2-yl) piperidin-1-yl)-3-fluoroaniline (intermediate 16-3)

The intermediate 16-2 (7 g, 23.64 mmol) was dissolved in 70 mL of tetrahydrofuran, and ammonium chloride (13 g, 236.48 mmol), water (10 mL) and iron powder (12 g, 236.48 mmol) were added to the system for 12 h at 70°C. At the end of the reaction, EA was extracted three times, organic phases were combined, anhydrous sodium sulfate was dried, concentrated under reduced pressure, and 3.4 g of yellow solid 4-(4-(1,3-dioxanecyclo-2-yl) piperidin-1-yl)-3-fluoroaniline was obtained by column chromatography (ethyl acetate/petroleum ether) in a yield of 54%. ESI-MS(M+H)⁺ =267.

### Step 4: 5-((4-(4-(1,3-dioxanecyclo-2-yl) piperidin-1-yl)-3-fluorophenyl) amino)-3-(methylthio)-1,2,4-triazine-6-carboxamide (intermediate 16-4)

The intermediate 16-3 (1.5 g, 5.63 mmol) was dissolved in 15 mL of acetonitrile, and DIPEA (1.81 g, 14.07 mmol) and 5-chloro-3-(methylthio)-1,2,4-triazine-6-carboxylic acid ethyl ester (1.3 g, 5.63 mmol) were added to the system sequentially, and after 30 min of reaction at room temperature, methanol solution of ammonia (7 mol/L, 15 mL) was added to the system, and the system became turbid within 10 min, and the reaction continued at room temperature for 2 h. At the end of the reaction, the petroleum ether was pulped and filtered to obtain 2.1 g of yellow solid 5-((4-(4-(1,3-dioxanecyclo-2-yl) piperidin-1-yl)-3-fluorophenyl) amino)-3-( methylthio)-1, 2, 4- triazine-6-carboxamide in a yield of 83%. ESI-MS(M+H)⁺=435.

### Step 5: 5-((4-(4-(1,3-dioxancyclo-2-yl) piperidin-1-yl)-3- fluorophenyl) amino)-3-(piperidin-1-yl)-1,2,4-triazine-6-carboxamide (intermediate 16-5)

Intermediate 16-4 (1 g, 2.00 mmol) was dissolved in 10 mL of NMP, mCPBA (85%, 1.8 g, 10.00 mmol) was added to the system, and stirred at room temperature for 2 h. At the end of the reaction, DIPEA (1.1 mL, 6.00 mmol) and piperidine (653 mg, 3.00 mmol) were added to the system for 2 h at 80°C. At the end of the reaction, water was added to quench, EA was extracted five times, the organic phases were combined, the organic phases were washed twice with saturated sodium chloride aqueous solution, the anhydrous sodium sulfate was dried, and the crude products were concentrated under reduced pressure, and the obtained crude product was subjected to column chromatography (dichloromethane/methanol elution) to obtain 690 mg of yellow solid 5-((4-(4-(1,3- dioxanecyclo-2-yl) piperidin-1-yl)-3-fluorophenyl)-3-(piperidin-1-yl)-1,2,4-triazine-6-carboxamide in a yield of 73%. ESI-MS(M+H)⁺=472.

### Synthesis step 6: 5-((3-fluoro-4-(4-formylpiperidin-1-yl) phenyl) amino)-3-(piperidin-1-yl)-1,2, 4 triazin-6-carboxamide (intermediate 16)

The intermediate 16-5 (690 mg, 1.46 mmol) was dissolved in 3 mL of tetrahydrofuran, and 3N HCl (7 mL) was added to the system for 3 h at 80°C. At the end of the reaction, saturated sodium bicarbonate aqueous solution was added to the system to adjust to alkaline, DCM extracted three times, organic phases were combined, anhydrous sodium sulfate was dried, concentrated under reduced pressure, and 380 mg of intermediate 16 yellow solid was obtained by column chromatography (methanol/dichloromethane system) with a yield of 57%. ESI-MS(M+H)⁺ =428.

**Referring to the synthesis route and method of intermediate 16, the following intermediate structure is obtained:**

| Intermediate No. | Intermediate structure | LC-MS (ESI-MS) *m*/*z* [M+H] ⁺ |
|---|---|---|
| 17 | 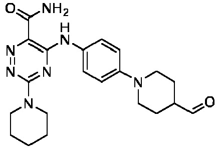 | 410 |
| 18 | 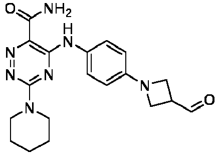 | 382 |
| 19 | 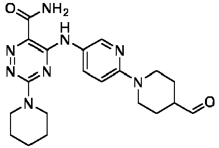 | 411 |
| 20 | 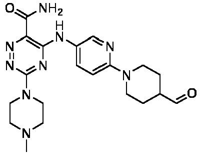 | 426 |
| 21 | 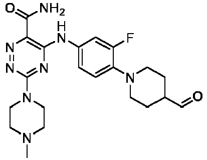 | 443 |
| 22 | 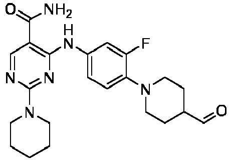 | 427 |

### Intermediate 23: tert-butyl ester of 4-(4-(2,4- dioxotetrahydropyrimidin-1 (2H)-yl) phenyl) piperazine -1-carboxylate

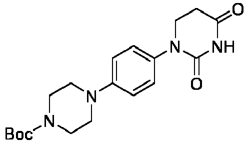

### Synthesis step 1: 3-((4-bromophenyl) amino) propionic acid (intermediate 23-1)

4-Bromoaniline (5.00 g, 29.06 mmol) was dissolved in 40 mL of toluene, and acrylic acid (2.50 g, 34.87 mmol) was added to the system for 12 h at 100°C. At the end of the reaction, water was added to quench, concentrated under reduced pressure, and 2.8 g of yellow solid 3-((4-bromophenyl) amino) propionic acid was obtained by column chromatography (PE/EA elution) with a yield of 39%. ESI-MS: m/z =244[M+H]⁺.

### Synthesis step 2: 1-(4-bromophenyl) dihydropyrimidine-2,4(1H,3H)-dione (intermediate 23-2)

Intermediate 23-1 (2.80 g, 11.52 mmol) was dissolved in 30 mL of acetic acid, urea (1.40 g, 23.04 mmol) was added to the system, and the reaction was carried out at 120°C for 12 h. At the end of the reaction, the solid precipitation was precipitated by adding water, and the yellow solid was filtered, dissolved by adding DCM, dried with anhydrous sodium sulfate, concentrated under reduced pressure, and 1.1 g of solid 1-(4-bromophenyl) dihydropyrimidine-2,4(1H,3H)-dione was obtained by column chromatography (DCM/MeOH elution) with a yield of 35%. ESI-MS: m/z = 269[M+H]⁺.

### Synthesis step 3: 4-(4-(2,4-dioxotetrahydropyrimidine-1(2H)-yl)phenyl) piperazine-1-carboxylate tert-butyl ester (intermediate 23)

The intermediate 23-2 (150 mg, 0.55 mmol) was dissolved in 3 mL of 1,4-dioxane solution, and tert-butyl piperazine-1-carboxylic acid (156 mg, 0.83 mmol), sodium tert-butoxide (162 mg, 1.65 mmol), cesium carbonate (536 mg, 1.65 mmol), palladium acetate (12 mg, 0.05 mmol), tris(dibenzylacetone) palladium (45 mg, 0.05 mmol), X-phos (52 mg, 0.11 mmol), reacted at 90°C for 12 h. At the end of the reaction, water was added and quenched, extracted with EA for 3 times, organic phases were combined, anhydrous sodium sulfate was dried, and 80 mg of 4-(4-(2,4-dioxotetrahydropyrimidine-1(2H)-yl) phenyl) piperazine-1-carboxylic acid tert-butyl ester was obtained by column chromatography (PE/EA elution) in a yield of 38%. ESI-MS: m/z = 375 [M+H]⁺.

### Intermediate 24: 1-(2-(2,6-dioxoppiperidin-3-yl)-1,3-dioxoisoindolin -5- yl) pyrrolidin-3-carboxylic acid

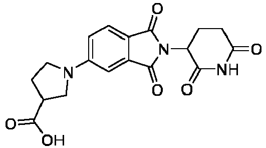

2-(2,6-dioxoppiperidin-3-yl)-5-fluoroisoindoline-1,3-dione (500 mg, 1.81 mmol) was dissolved in 8 mL DMSO, and pyrrolidin-3-carboxylic acid (312 mg, 2.72 mmol) and DIPEA (701 mg, 5.43 mmol) were added to the system for 12 h at 90°C. At the end of the reaction, water was added, DCM extracted three times, the organic phase was combined, the organic phase was washed with saturated ammonium chloride aqueous solution, the anhydrous sodium sulfate was dried, and the intermediate was concentrated under reduced pressure, 550 mg of intermediate 24 was obtained by column chromatography with a yield of 81%. ESI-MS(M+H) ⁺=372.

### Intermediate 25: 4-(2-(2,6-dioxopridin-3-yl)-1,3-dioxoisoindolin-5-yl) piperazine-1-carboxylic acid tert-butyl ester

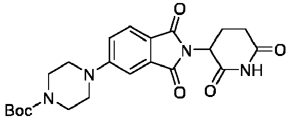

Referring to the synthesis route and method of intermediate 24, pyrrolidine-3-carboxylic acid was replaced with tert-butyl piperazine-1-carboxylic acid to synthesize intermediate 25. ESI-MS(M+H)⁺ =443.

### Intermediate 26: 4-(2-(2,6-dioxopridin-3-yl)-6-fluoro-1,3-dioxoisoindolin-5-yl) piperazine-1-carboxylic acid tert-butyl ester

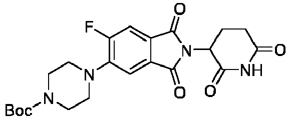

Referring to the synthesis route and method of intermediate 25, 2-(2,6-dioxopridin-3-yl)-5- fluoroisoindolin -1,3- dione was replaced with 2-(2,6-dioxopperidine-3-yl)-5,6-difluoroisoindolin-1,3-dione, and the intermediate 26 was synthesized. ESI-MS(M+H) ⁺ =461.

### Intermediate 27: 1-(4-(2,4-dioxotetrahydropyrimidine-1(2H)-yl)-3- fluorophenyl) piperidin-4- carboxaldehyde

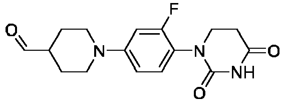

### Synthesis step 1: 5-(4-(1,3-dioxopentacyclo-2-yl) piperidin-1-yl)-2-nitroaniline (intermediate 27-1)

5-fluoro-2-nitroaniline (1 g, 6.41 mmol) was dissolved in 10 mL of DMF, and 4-(1,3-dioxanecyclo-2-yl) piperidine (1.2 g, 7.69 mmol) and cesium carbonate (6.2 g, 19.23 mmol) were added to the system for 3 hours at 80°C. At the end of the reaction, water was added to quench, DCM extracted three times, organic phases were combined, anhydrous sodium sulfate was dried, concentrated under reduced pressure, and 5-(4-(1,3-dioxanecyclo-2-yl) piperidin-1-yl)-2-nitroaniline 1.5 g was obtained by column chromatography with a yield of 79% and ESI-MS(M+H)⁺=294.

### Synthesis step 2: 4-(1,3-dioxanecyclo-2-yl)-1-(3-fluoro-4-nitrophenyl) piperidine (intermediate 27-2)

The intermediate 27-1 (1.5 g, 5.11 mmol) was dissolved in 15 mL of concentrated hydrochloric acid, HF (70%, 30 mL) was added to the system at 0 °C, the reaction was carried out at room temperature until the reaction was completely dissolved, NaNO₂ (423 mg, 6.13 mmol) was added to the system at -78 °C, and the reaction was heated to 60 °C for 15-30 min after the reaction was heated to 60 °C for 30 min. At the end of the reaction, water was added to quench, DCM was extracted three times, organic phases were combined, anhydrous sodium sulfate was dried, concentrated under reduced pressure, and 1 g of 4-(1,3-dioxancyclo-2-yl)-1-(3-fluoro-4-nitrophenyl) piperidine was obtained by column chromatography with a yield of 66%. ESI-MS(M+H) ⁺ =297.

### Synthesis step 3: 4-(4-(1,3-dioxancyclo-2-yl) piperidin-1-yl)-2-fluoroaniline (intermediate 27-3)

The intermediate 27-2 (1 g, 3.37 mmol) was dissolved in 10mL of tetrahydrofuran, and ammonium chloride (2 g, 33.78 mmol), water (10 mL) and iron powder (1.9 g, 33.78 mmol) were added to the system for 12 h at 70°C. At the end of the reaction, the iron powder was removed by diatomaceous earth filtration, the filtrate was extracted three times with DCM, the organic phase was combined, the anhydrous sodium sulfate was dried, concentrated under reduced pressure, and 4-(4-(1,3-dioxancyclo-2-yl) piperidin-1-yl)-2-fluoroaniline 750 mg was purified by column chromatography (EA/PE elution) with a yield of 83%. ESI-MS(M+H) ⁺ =267.

### Synthesis step 4: 3-(4-(4-(1,3-dioxanecyclo-2-yl) piperidin-1-yl)-2-fluorophenyl) amino) propionic acid (intermediate 27-4)

The intermediate 27-3 (750 mg, 2.81 mmol) was dissolved in 10 mL of toluene, and acrylic acid (243 mg, 3.38 mmol) was added to the system for 12 h at 100°C, and the reaction was completed. DCM extraction was carried out five times, organic phases were combined, anhydrous sodium sulfate was dried, concentrated under reduced pressure, and 860 mg of yellow liquid 3-(4-(4-(1,3-dioxocyclo-2-yl) piperidin-1-yl)-2-fluorophenyl) amino) propionic acid was obtained by column chromatography with a yield of 90%. ESI-MS(M+H)⁺ =339.

### Synthesis step 5: 1-(4-(4-(1,3-dioxancyclo-2-yl) piperidin-1-yl)-2-fluorophenyl) dihydropyrimidine -2,4(1H,3H)-dione (intermediate 27-5)

The intermediate 27-4 (860 mg, 2.54 mmol) was dissolved in 10 mL of acetic acid, urea (315 mg, 5.08 mmol) was added to the system for 12h at 120°C. At the end of the reaction, DCM was extracted three times, organic phases were combined, anhydrous sodium sulfate was dried, concentrated under reduced pressure, and 540 mg of brown-yellow solid 1-(4-(4-(4-(1,3-dioxanecyclo-2-yl) piperidin-1-yl)-2- fluorophenyl) dihydropyrimidine-2,4 (1H,3H)-dione was obtained by column chromatography (DCM/MeOH elution) in a yield of 58%. ESI-MS (M+H)⁺ =364.

### Synthesis step 6: 1-(4-(2,4-dioxotetrahydropyrimidine-1(2H)-yl)-3-fluorophenyl) piperidine- 4- carboxaldehyde (intermediate 27)

The intermediate 27-5 (540 mg, 1.68 mmol) was dissolved in 6 mL of TFA, reacted at 80°C for 1 h, and then added 0.5 mL of concentrated hydrochloric acid to the system for 3 h at 80 °C. At the end of the reaction, the saturated sodium carbonate aqueous solution was adjusted to alkaline, MeOH/DCM was extracted five times, the organic phases were combined, the anhydrous sodium sulfate was dried, concentrated under reduced pressure, and the 490 mg of the intermediate 27 was obtained by column chromatography with a yield of 91%. ESI-MS(M+H)⁺ =320.

**Referring to the synthesis route and method of intermediate 27, the following intermediate structure is obtained:**

| Intermediate No. | Intermediate structure | LC-MS (ESI-MS) *m*/*z* [M+H] ⁺ |
|---|---|---|
| 28 | 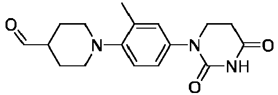 | 316 |
| 29 | 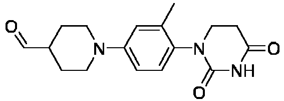 | 316 |
| 30 | 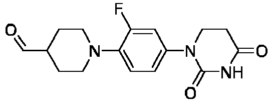 | 320 |
| 31 | 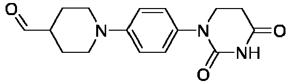 | 302 |
| 32 | 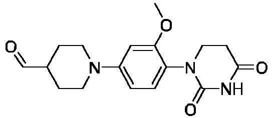 | 332 |

### Intermediate 33: N-(2,6-dioxopridin-3-yl)-5-(4-formylpiperidin-1-yl) pyridinamide

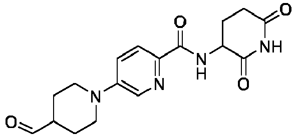

### Synthesis step 1: 5-(4-(1,3-dioxopentacyclo-2-yl) piperidine-1-yl) methyl picolinate (intermediate 33-1)

Methyl 5-fluoropyridine-2-carboxylic acid (2.2 g, 14.18 mmol) was dissolved in 20 mL of DMF, and 4-(1,3-dioxanecyclo-2-yl) piperidine (2.7 g, 17.02 mmol) and potassium carbonate (5.8 g, 42.54 mmol) were added to the system for 2 h at 90°C. At the end of the reaction, water was added to quench, DCM was extracted three times, organic phases were combined, anhydrous sodium sulfate was dried, concentrated under reduced pressure, and 3.2g of yellow solid methyl 5-(4-(1,3-dioxopentacyclo-2-yl) piperidin-1-yl) pyridin-2-carboxylic acid was obtained by column chromatography with a yield of 78%. ESI-MS(M+H)⁺ =293.

### Synthesis step 2: 5-(4-(1,3-dioxopentacyclo-2-yl) piperidin-1-yl) picolinic acid (intermediate 33-2)

The intermediate 33-1 (1.5 g, 5.13 mmol) was dissolved in 20 mL of tetrahydrofuran, lithium hydroxide (615 mg, 25.67 mmol) and water (7.5 mL) were added to the system, the reaction was carried out at room temperature for 2h, the reaction was completed, concentrated to solvent-free, DCM beating, filtration, and concentrated filtrate to obtain 1.3g of white solid 5-(4-(1,3-dioxancyclo-2-yl)piperidin-1-yl)pyridine-2-carboxylic acid in a yield of 93%. ESI-MS(M+H)⁺ =279.

### Step 3: 5-(4-(1,3-dioxancyclo-2-yl) piperidin-1-yl)-N-(2,6-dioxopridin-3-yl) picolimide (intermediate 33-3)

The intermediate 33-2 (500 mg, 1.08 mmol) was dissolved in 10 mL of DMF, and 3-aminopiperidin-2,6-dione (255 mg, 1.97 mmol), HATU (752 mg, 1.97 mmol) and triethylamine (0.75 mL, 5.39 mmol) were added to the system for 1 h at room temperature. At the end of the reaction, DCM was extracted three times, organic phases were combined, anhydrous sodium sulfate was dried, concentrated under reduced pressure, and 350 g of gray solid 5-(4-(1,3-dioxopentacyclo-2-yl) piperidin-1-yl)-N-(2,6-dioxopridin-3-yl) pyridinamide was obtained by column chromatography (DCM/MeOH elution) in a yield of 51%. ESI-MS(M+H)⁺ =389.

### Step 4: N-(2,6-dioxopridin-3-yl)-5-(4-formylpiperidin-1-yl) pyridinamide (intermediate 33)

The intermediate 33-3 (100 mg, 0.26 mmol) was dissolved in 2 mL of TFA and reacted at 80°C for 1 h, and then 1 mL of concentrated hydrochloric acid was added to the system for 3 h at 80°C. At the end of the reaction, saturated sodium carbonate aqueous solution was added to adjust the system to alkaline, DCM extraction was carried out five times, organic phases were combined, anhydrous sodium sulfate was dried, and concentrated under reduced pressure to obtain 75 mg of intermediate 33 yellow solid with a yield of 84%. ESI-MS(M+H)⁺ =345.

**Referring to the synthesis route and method of intermediate 33, the following intermediate structure is synthesized:**

| Intermediate No. | Intermediate structure | LC-MS (ESI-MS) *m*/*z* [M+H] ⁺ |
|---|---|---|
| 34 | 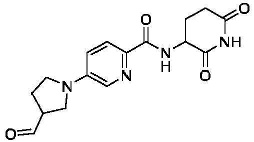 | 331 |
| 35 | 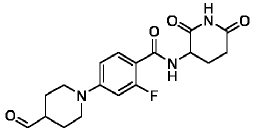 | 362 |

### Intermediate 36: 1-(6-((2,6-dioxopridin-3-yl) amino) pyridin-3-yl) piperidin-4-carboxaldehyde

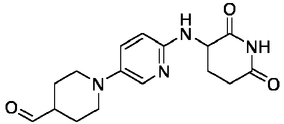

### Synthesis step 1: 5-(4-(1,3-dioxancyclo-2-yl) piperidine-1-yl)-2-nitropyridine (intermediate 36-1)

5-fluoro-2-nitropyridine (1.5 g, 10.56 mmol) was dissolved in 20 mL of DMF, and 4-(1,3-dioxanecyclo-2-yl) piperidine (1.8 g, 12.67 mmol) and cesium carbonate (10 g, 31.68 mmol) were added to the system for 2 h at 80°C. At the end of the reaction, water was added to quench and DCM extracted three times, organic phases were combined, anhydrous sodium sulfate was dried, concentrated under reduced pressure, and solid 5-(4-(1,3-dioxanecyclo-2-yl) piperidin-1-yl)-2-nitropyridine 2 g was obtained by column chromatography with a yield of 69%. ESI-MS(M+H)⁺ = 280.

### Synthesis step 2: 5-(4-(1,3-dioxopentyl-2-yl) piperidin-1-yl) pyridin-2-amine (intermediate 36-2)

The intermediate 36-1 (2 g, 7.31 mmol) was dissolved in 25 mL of tetrahydrofuran, and ammonium chloride (3.9 g, 73.12 mmol), water (5 mL) and iron powder (4.1 g, 73.12 mmol) were added to the system for 12 h at 70°C. At the end of the reaction, a large amount of iron powder was filtered out while it was hot, DCM extracted three times, organic phases were combined, anhydrous sodium sulfate was dried, concentrated under reduced pressure, and 1.6 g of 5-(4-(1,3-dioxanecyclo-2-yl) piperidin-1-yl) pyridin-2-amine was obtained by column chromatography with a yield of 89%. ESI-MS(M+H)⁺ =250.

### Step 3: 3-((5-(4-(1,3-dioxanecyclo-2-yl) piperidin-1-yl) pyridin-2-yl) amino) piperidin-2,6-dione (intermediate 36-3)

The intermediate 36-2 (1.6 g, 6.49 mmol) was dissolved in 20 mL of DMF, and 3-bromopiperidine-2, 6-dione (1.9 g, 9.74 mmol), Pd(dppf) Cl₂ (472 mg, 0.65 mmol), and Na₂CO₃ (2.1 g, 19.47 mmol) were added to the system for 3 h at 100°C. At the end of the reaction, water was added and quenched, DCM extracted three times, organic phases were combined, anhydrous sodium sulfate was dried, concentrated under reduced pressure, and 1.9 g of 3-((5-(4-(1,3-dioxanecyclo-2-yl) piperidin-1-yl) pyridin-2-yl) amino) piperidin-2,6-dione was obtained by column chromatography with a yield of 82%. ESI-MS(M+H)⁺ =361.

### Synthesis step 4: 1-(6-((2,6-dioxopperidine-3-yl) amino) pyridin-3-yl) piperidine -4-carboxaldehyde (intermediate 36)

The intermediate 36-3 (1.9 g, 5.35 mmol) was dissolved in 10 mL of TFA and reacted at 80°C for 1h. Then 2 mL of concentrated hydrochloric acid was added to the system and reacted at 80°C for 3 h. At the end of the reaction, the saturated sodium carbonate aqueous solution was adjusted to alkaline, DCM extracted five times, the organic phase was combined, the anhydrous sodium sulfate was dried, and the intermediate 36 solid 1.4 g was obtained under reduced pressure with a yield of 8 3%. ESI-MS(M+H) ⁺ =317.

**Referring to the synthesis route and method of intermediate 36, the following intermediate structure is obtained:**

| Intermediate No. | Intermediate structure | LC-MS (ESI-MS) *m*/*z* [M+H]⁺ |
|---|---|---|
| 37 | 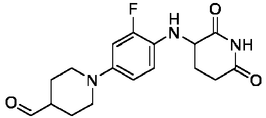 | 334 |
| 38 | 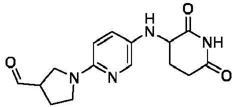 | 303 |

### Intermediate 39: 1-(5-(6-oxo-1,6-dihydropyridazine-3-yl)-3-(trifluoromethyl) pyridin-2-yl) piperidin-4-carboxaldehyde

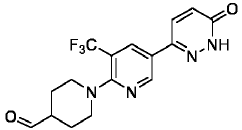

### Synthesis step 1: (6-chloro-5-(trifluoromethyl) pyridin-3-yl) boric acid (intermediate 39-1)

5-bromo-2-chloro-3-(trifluoromethyl) pyridine (5 g, 19.20 mmol) was dissolved in 50 mL of 1,4-dioxane, and bis(pinacolato)diboron (4.8 g, 19.20 mmol), potassium acetate (4.7 g, 48.12 mmol), and Pd(dppt)Cl₂ (1.39 g, 1.92 mmol) were added to the system, and the reaction was completed at 100°C for 6 h in an argon atmosphere. DCM was extracted three times, organic phases were combined, anhydrous sodium sulfate was dried, and concentrated under reduced pressure to obtain 4 g of (6-chloro-5-(trifluoromethyl) pyridin-3-yl) boric acid in a yield of 92%, ESI-MS(M+H)⁺=226.

### Synthesis step 2: 3-chloro-6-(6-chloro-5-(trifluoromethyl) pyridine-3-yl) pyridazine (intermediate 39-2)

The intermediate 39-1 (2 g, 8.88 mmol) was dissolved in 20 mL acetonitrile, and 3,6-dichloropyridazine (1.7 g, 11.54 mmol), potassium carbonate (3.4 g, 26.64 mmol), water (2 mL), and PdCl₂(pph₃)₂ (616 mg, 0.88 mmol) were added to the system for 4 h at 80°C. After the reaction, it was concentrated under reduced pressure to obtain 700 mg of 3-chloro-6-(6-chloro-5-(trifluoromethyl) pyridin-3-yl) pyridazine in yield of 26% and ESI-MS (M+H)⁺=294.

### Synthesis step 3: 6-(6-chloro-5-(trifluoromethyl) pyridin-3-yl) pyridazine-3-ol (intermediate 39-3)

The intermediate 39-2 (700 mg, 2.38 mmol) was dissolved in 9 mL of 1,4-dioxane, 3M HCl was added to the system, and the reaction was completed at 110°C for 4h. DCM was extracted three times, organic phases were combined, anhydrous sodium sulfate was dried, and concentrated under reduced pressure to obtain 6-(6-chloro -5-(trifluoromethyl) pyridin-3-yl) pyridazine-3-ol 440 mg in yield 67%, ESI-MS(M+H)⁺=276.

### Synthesis step 4: 6-(6-(4-(1,3-dioxancyclo-2-yl) piperidin-1-yl)-5-(trifluoromethyl) pyridin-3-yl) pyridazine-3(2H)-one (intermediate 39-4)

The intermediate 39-3 (440 mg, 1.60 mmol) was dissolved in 5 mL of DMF, and 4-(1,3-dioxanecyclo-2-yl) piperidine (376 mg, 2.40 mmol) and cesium carbonate (1.5 g, 4.80 mmol) were added to the system, and the reaction was completed at 120°C for 12 h. 6-(6-(4-(1,3-dioxanecyclo-2-yl)piperidin-1-yl)-5-(trifluoromethyl) pyridine-3-yl)pyridazine-3(2H)-one was obtained with a yield of 54% and ESI-MS(M+H)⁺=397.

### Synthesis step 5: 1-(5-(6-oxo-1,6-dihydropyridazine-3-yl)-3-(trifluoromethyl) pyridin-2-yl) piperidin-4-carboxaldehyde (intermediate 39)

The intermediate 39-4 (140 mg, 0.35 mmol) was dissolved in 2 mL of TFA, and after the reaction was at 80°C for 1h, 0.1 mL of concentrated hydrochloric acid was added to the system, and the reaction was continued at 80°C for 1h, and the reaction was completed. Saturated sodium carbonate aqueous solution was adjusted to neutral or alkaline, DCM extracted five times, organic phases were combined, anhydrous sodium sulfate was dried, and concentrated under reduced pressure to obtain 100 mg of 1-(5-(6-oxo-1,6- dihydropyridazine -3-yl)-3- (trifluoromethyl) pyridin-2-yl) piperidin-4-carboxaldehyde in a yield of 81%. ESI-MS(M+H) ⁺ =353.

**Referring to the synthesis route and method of intermediate 39, the following intermediate structure is synthesized:**

| Intermediate No. | Intermediate structure | LC-MS (ESI-MS) *m*/*z* [M+H] ⁺ |
|---|---|---|
| 40 | 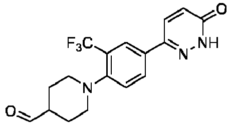 | 352 |

### Example 41: tert-butyl ester of 4-(5-(2,6-dioxopridin-3-yl) pyridin-2-yl) piperazine-1-carboxylate

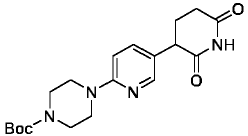

### Synthesis step 1: (6-(4-(tert-butoxycarbonyl) piperazine-1-yl) pyridine-3-yl) boronic acid (intermediate 40-1)

4-(5-bromopyridine-2-yl) piperazine-1-carboxylic acid tert-butyl ester (1.4 g, 4.09 mmol) was dissolved in 15 mL of 1,4-dioxane, and bis(pinacolato)diboron (1.0 g, 4.09 mmol), potassium acetate (1.2 g, 12.27 mmol) and Pd(dppf)Cl₂ (232 mg, 0.32 mmol) were added to the system in a argon atmosphere at 100°C for 6 h, and the reaction was completed. After filtration, the filtrate was added with water, extracted by DCM for three times, the organic phase was combined, the anhydrous sodium sulfate was dried, concentrated under reduced pressure, and 1.0 g of (6-(4-(tert-butoxycarbonyl) piperazine-1-yl) pyridin-3-yl) boric acid was obtained by column chromatography with a yield of 79%. ESI-MS(M+H)⁺ =308.

### Synthesis step 2: 4-(2',6'-bis(benzyloxy)-[3,3'-bipyridine]-6-yl) piperazine-1-carboxylic acid tert-butyl ester (intermediate 40-2)

5-The intermediate 40-1 (950 mg, 3.09 mmol) was dissolved in 15 mL of 1,4-dioxane, and 2,6-bis(benzyloxy) -3-bromopyridine (2.29 g, 6.19 mmol), cesium acetate (3.0 g, 9.28 mmol), Pd(dppf)Cl₂ (224 mg, 0.30 mmol) were added to the system, and the reaction was completed at 100°C for 12 h in argon atmosphere. After filtration, the filtrate was added with water, extracted by DCM for three times, combined with organic phases, dried with anhydrous sodium sulfate, concentrated under reduced pressure, and obtained 1.2 g of 4-(2',6'-bis(benzyloxy)- [3,3'-bipyridine]-6-yl) piperazine-1-carboxylic acid tert-butyl ester by column chromatography with a yield of 70%. ESI-MS(M+H)⁺ =553.

### Synthesis step 3: 4-(5-(2,6-dioxopridin-3-yl) pyridin-2-yl) piperazine-1-carboxylate tert-butyl ester (intermediate 40)

The intermediate 40-2 (1.0 g, 1.81 mmol) was dissolved in 20 mL of methanol, Pd/C (400 mg) was added to the system sequentially, and the reaction was carried out at room temperature in a hydrogen environment for 24 h. After the reaction, 320 mg of tert-butyl piperazine-1-carboxylic acid was obtained by filtration, concentration under reduced pressure, and column chromatography to obtain 320 mg of tert-butyl ester of 4-(5-(2,6- dioxopridin -3-yl) pyridine-2-yl) piperazine-1-carboxylate in yield of 47%, ESI-MS(M+H)⁺=375.
**Referring to the synthesis route and method of intermediate 41, the following intermediate structure is obtained:**

| Intermediate No. | Intermediate structure | LC-MS (ESI-MS)*m*/*z* [M+H]⁺ |
|---|---|---|
| 42 | 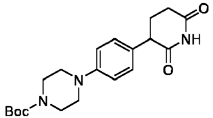 | 374 |

### Example 1:5-((4-(1-((1-(2-(2,6-dioxopiperidin-3-yl)-1,3-dioxoisoindolin-5-yl) pyrrolidin-3-yl) methyl) piperidin-4-yl)-3-fluorophenyl) amino)-3-(piperidin-1-yl)-1,2,4-triazine-6-carboxamide (002)

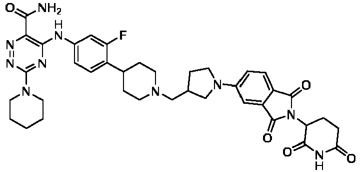

### Step 1: 4-(4-((6-carbamoyl-3-hydroxy-1,2,4-triazine-5-yl) amino)-2-fluorophenylpiperidin-1- carboxylate tert-butyl ester (002-1)

Intermediate 1 (1.3 g, 2.81 mmol) was dissolved in 20 mL of NMP, mCPBA (85%, 5.0 g, 28.93 mmol) was added to the system, stirred at room temperature for 2 h, and then DIPEA (4.5 g, 34.82 mmol) and water (10 mL) were added to the system to continue the reaction at room temperature for 2 h. At the end of the reaction, 25 mL of water was added, filtered, and the filter cake was subjected to column chromatography (dichloromethane/methanol elution) to obtain 350 mg of orange-yellow solid 4-(4-((6-carbamoyl-3-hydroxy-1,2,4-triazine-5-yl) amino) -2-fluorophenyl) piperidin-1-carboxylic acid tert-butyl ester in a yield of 30%. ESI-MS(M+H)⁺ =433.

### Synthesis step 2: tert-butyl (4-(4-((6-carbamoyl-3-hydroxy-1,2,4-triazine-5-yl) amino)-2- fluorophenyl) piperidine-1-yl) methyl) pyrrolidine-1-carboxylate (002-2)

002-1 (350 mg, 0.81 mmol) was dissolved in 5 mL DCM, trifluoroacetic acid (3 mL) was added to the system, and stirred for 1 h at room temperature. At the end of the reaction, it is concentrated under reduced pressure to obtain a yellow liquid. The above products were dissolved in 10 mL DCM, DIPEA (314 mg, 2.43 mmol) and 3-formylpyrrolidin-1-carboxylic acid tert-butyl ester (323 mg, 1.62 mmol) were added sequentially, stirred at room temperature for 10 min, acetic acid (0.5 mL) and sodium cyanoborohydride (101 mg, 1.62 mmol) were added to the system, and the reaction was continued at room temperature for 1 h. At the end of the reaction, saturated sodium bicarbonate aqueous solution was quenched, DCM extracted three times, organic phases were combined, anhydrous sodium sulfate was dried, concentrated under reduced pressure, and 210 mg of yellow solid 3-((4-(4-((6-carbamoyl-3-hydroxy-1,2,4-triazin-5-yl)amino)-2-fluorophenyl)piperidin-1-yl)methyl)pyrrolidin-1-carboxylic acid tert-butyl ester was obtained by column chromatography (DCM/MeOH elution) in yield of 50%. ESI-MS(M+H)⁺=516.

### Step 3: 5-((4-(1-((1-(2-(2-(2,6-dioxopridin-3-yl)-1,3-dioxoisoindolin-5-yl)pyrrolidin-3-yl) methyl) piperidin -4-yl) -3-fluorophenyl)amino)-3-hydroxy-1,2,4-triazine-6-carboxamide (002-3)

002-2 (210 mg, 0.41 mmol) was dissolved in 5 mL of DCM, trifluoroacetic acid (2 mL) was added to the system, and stirred for 1h at room temperature. At the end of the reaction, it is concentrated under reduced pressure to obtain a yellow liquid. The above products were dissolved in 5 mL of DMSO, and DIPEA (1.48 g, 11.44 mmol), 2-(2,6-dioxopiidin-3-yl)-5-fluoroisoindoline-1,3-dione (280 mg, 1.02 mmol) were added to the system for 12 h at 95°C. At the end of the reaction, the solid precipitation is precipitated by adding water, the yellow solid is filtered, the DCM is added to dissolve, the anhydrous sodium sulfate is dried, and the concentration is reduced pressure. The yellow solid 5-((4-(1-((1-(2-(2,6-dioxopridin-3-yl)-1,3-dioxoisoindolin-5-yl) pyrrolidin-3-yl) methyl) piperidin -4-yl)-3-fluorophenyl) amino)-3-hydroxy-1,2,4-triazine-6-carboxamide 200 mg was obtained by column chromatography (DCM/MeOH elution, yield: 73%. ESI-MS(M+H) ⁺=672.

### Step 4: 3-chloro-5-((4-(1-(1-(2-(2-(2,6-dioxopridin-3-yl)-1,3-dioxoisoindolin-5-yl) pyrrolidin-3-yl) methyl) piperidin-4-yl)-3-fluorophenyl) amino)-1,2,4-triazin-6-carboxamide (002-4)

002-3 (200 mg, 0.29 mmol) was dissolved in 10 mL of 1,4-dioxane, and N, N-diisopropylethylamine (1.5 g, 11.50 mmol) and phosphorus oxychloride (2 mL) were added to the system for 0.2 h at 105°C. After the reaction, slowly pour 20 mL of water, add sodium bicarbonate to adjust to pH value greater than 7, filter, and filter cake, the crude product 3-chloro-5-((4-(1-(1-(2-(2-(2,6-dioxopiperidin-3-yl)-1,3-dioxoisoindolin-5-yl) pyrrolidin-3-yl) methyl) piperidin-4-yl)-3-fluorophenyl) amino)-1,2,4-triazine-6-carboxamide 200 mg, yield: 99%. ESI-MS (M+H)⁺=690.

### Synthesis step 5: 5-((4-(1-((1-(2-(2,6-dioxopiperidin-3-yl)-1,3-dioxoisoindolin-5-yl) pyrrolidin-3-yl) methyl) piperidin-4-yl)-3-fluorophenyl) amino)-3-(piperidin-1-yl)-1,2,4-triazine-6-carboxamide (002)

002-4 (100 mg, 0.14 mmol) was dissolved in 3 mL of DMF, piperidine (18 mg, 0.20 mmol) and N, N-diisopropylethylamine (40 mg, 0.31 mmol) were added to the system for 0.5 h at 75°C. After the end of the reaction, water was added until there was no longer yellow solid precipitation in the system, and the solid was obtained by column chromatography to obtain 54 mg of compound 002 solid in a yield of 53%. ESI-MS(M+H) ⁺ =739. ¹H-NMR (400 MHz, CCl₃D) δ 11.00 (brs, 1H), 8.20 (brs, 1H), 7.57 - 7.72 (m, 3H), 7.22 - 7.23 (m, 2H), 6.96 - 6.97 (m, 1H), 6.68 - 6.71 (m, 1H), 5.45 (brs, 1H), 4.92 - 4.96 (m, 1H), 3.70 - 4.01 (m, 4H), 3.40 - 3.61 (m, 3H), 3.17 - 3.22 (m, 1H), 2.60 - 3.19 (m, 6H), 2.41 - 2.48 (m, 2H), 2.08 - 2.25 (m, 3H), 1.73 - 1.89 (m, 10H), 1.25 - 1.29 (m, 3H).

### Example 2:5-((4-(1-((1-(2-(2,6-dioxopiperidin-3-yl)-1,3-dioxoisoindolin-5-yl)pyrrolidin-3-yl) methyl) piperidin-4-yl) phenyl) amino)-3-(piperidin-1-yl)-1,2,4-triazine-6-carboxamide (001)

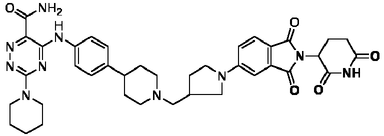

Referring to the synthesis route and method of embodiment 1, the intermediate 1 in the synthesis step 1 is replaced with intermediate 2, and the target compound 001 is synthesized to obtain ESI-MS(M+H) ⁺=721. ¹H-NMR (400 MHz, CCl₃D) δ 10.89 (brs, 1H), 8.02 (brs, 1H), 7.60 - 7.71 (m, 4H), 7.23 - 7.25 (m, 2H), 6.97 - 6.98 (m, 1H), 6.68 - 6.71 (m, 1H), 5.36 - 5.38 (m, 1H), 4.91 - 4.96 (m, 1H), 3.77 - 4.02 (m, 4H), 3. 38 - 3.62 (m, 3H), 3.19 - 3.24 (m, 1H), 2.94 - 3.08 (m, 2H), 2.64 - 2.92 (m, 4H), 2.41 - 2.51 (m, 3H), 2.00 - 2.23 (m, 7H), 1.71 - 1.86 (m, 8H).

### Example 3:5-((4-(4-((1-(2-(2,6-dioxopiperidin-3-yl)-1,3-dioxoisoindolin-5-yl)pyrrolidin-3-yl) methyl) piperazin-1-yl)-3-fluorophenyl) amino)-3-(piperidin-1-yl)-1,2,4-triazine-6-carboxamide (003)

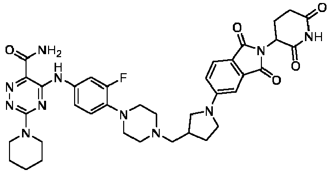

Referring to the synthesis route and method of embodiment 1, intermediate 1 in synthesis step 1 is replaced with intermediate 4, and the target compound 003 is synthesized to obtain ESI-MS(M+H)⁺=740. ¹H-NMR (400 MHz, CDCl₃) δ 10.88 (brs, 1H), 8.37 (s, 1H), 7.60 -7.71 (m, 3H), 7.18 - 7.22 (m, 1H), 6.92 - 6.96 (m, 2H), 6.68 - 6.70 (m, 1H), 5.53 (brs, 1H), 4.91 - 4.96 (m, 1H), 3.38 - 3.91 (m, 6H), 3.09 - 3.23 (m, 5H), 2.61 - 2.91 (m, 6H), 2.40 - 2.53 (m, 2H), 1.99 - 2.26 (m, 2H), 1.68 - 1.89 (m, 7H), 1.24 - 1.33 (m, 3H).

### Example 4:5-((4-(1-((1-(2-(2,6-dioxopiperidin-3-yl)-1,3-dioxoisoindolin-5-yl)piperidin-4-yl) methyl) piperidin-4-yl)-3-fluorophenyl) amino)-3-(piperidin-1-yl)-1,2,4-triazine-6-carboxamide 007

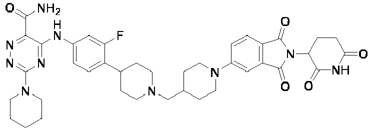

Referring to the synthesis route and method of embodiment 1, the tert-butyl ester of 3-formylpyrrolidine-1- carboxylic acid in the synthesis step 2 was replaced with 4-formylpiperidin-1-carboxylate tert-butyl ester, and the target compound 007 was synthesized, ESI-MS(M+H) ⁺=753. ¹H-NMR (400 MHz, CDCl₃) δ 11.00 (brs, 1H), 8.34 (s, 1H), 7.60 - 7.72 (m, 3H), 7.28 - 7.29 (m, 1H), 7.20 - 7.23 (m, 2H), 7.03 - 7.05 (m, 1H), 5.51 (brs, 1H), 4.92 - 4.96 (m, 1H), 3.94 - 3.96 (m, 4H), 2.77 - 3. 01 (m, 9H), 1.91 - 2.27 (m, 6H), 1.68 - 1.81 (m, 11H), 1.28 - 1.35 (m, 4H).

### Example 5:5-((4-(1-((1-(1-(2,6-dioxopiperidin-3-yl)-3-methyl-2-oxo-2,3-dihydro-1H-benzo[d] imidazol-5-yl) pyrrolidin-3-yl) methyl) piperidin-4-yl) phenyl) amino)-3- (piperidin-1-yl)-1,2,4-triazine-6- carboxamide (011)

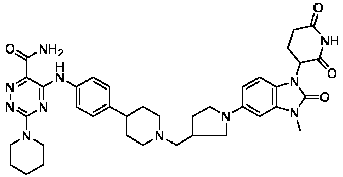

Referring to the synthesis route and method of embodiment 1, 2-(2,6-dioxopridin-3-yl)-5- fluoroisoindoline -1,3-dione in the synthesis step 3 was replaced with 3-(5-fluoro-3-methyl-2-oxo-2,3-dihydro-1H-benzo [d]imidazo-1-yl) piperidin-2,6-dione, and the target compound 011 was synthesized, ESI-MS(M+H)⁺=722.

### Example 6:3-((R)-3-acetamidopiperidin-1-yl)-5-((4-(1-((1-(2-(2,6- dioxopiperidin-3-yl)-1,3- dioxoisoindolin-5-yl) pyrrolidin-3-yl) methyl) piperidin-4-yl) phenyl) amino)-1,2,4-triazine-6-carboxamide (016)

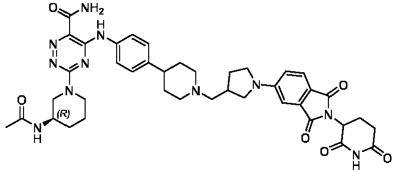

### Synthesis step 1:(R)-3-acetamidopiperidin-1-carboxylic acid tert-butyl ester (016-1)

(R)-3-aminopiperidine-1-carboxylic acid tert-butyl ester (100 mg, 0.50 mmol) was dissolved in 3 mL DCM, and acetic anhydride (76 mg, 0.75 mmol) and triethylamine (86 mg, 0.85 mmol) were added to the system for 0.5 h at room temperature. At the end of the reaction, DCM extraction, combined organic phases, saturated sodium bicarbonate aqueous solution washing, saturated ammonium chloride aqueous solution washing, saturated sodium chloride aqueous solution washing, anhydrous sodium sulfate drying, reduced pressure concentration, colorless liquid (R)-3-acetamidopiperidine-1-carboxylic acid tert-butyl ester 110 mg was obtained, the yield was 90%. ESI-MS(M+H)⁺ =243.

### Synthesis step 2: 3-chloro-5-((4-(1-(1-(2-(2-(2,6-dioxoppiperidin-3-yl)-1,3- dioxoisoindolin-5-yl) pyrrolidin- 3-yl) methyl) piperidin-4-yl) phenyl) amino)-1,2,4-triazine-6- carboxamide (016-2)

Referring to the synthesis route and method of the synthesis step 1-4 in embodiment 1, the intermediate 1 in the synthesis step 1 is replaced with the intermediate 2, and 3-chloro-5-((4-(1-(1-(2-(2-(2,6-dioxopridin-3-yl)-1,3- dioxoisoindolin-5-yl) pyrrolidin-3-yl) methyl)piperidin-4-yl)phenyl)amino)-1,2,4-triazine-6-carboxamide, ESI-MS(M+H)⁺=672.

### Synthesis step 3:3-((R)-3-acetamidopiperidin-1-yl)-5-((4-(1-((1-(2-(2,6- dioxopiperidin-3-yl)-1,3- dioxoisoindolin-5-yl) pyrrolidin-3-yl) methyl) piperidin-4-yl) phenyl) amino)-1,2,4-triazine-6-carboxamide (016)

Compound 016-1 was dissolved in 5 mL DCM, trifluoroacetic acid (1 mL) was added to the system, the reaction was carried out at room temperature for 1h, and concentrated under reduced pressure to obtain a colorless liquid (R)-N-(piperidin-3-yl) acetamide, which was directly used in the next step of the reaction. 016-2 (50 mg, 0.07 mmol) was dissolved in 3 mL of DMF, and (R)-N-(piperidin-3-yl) acetamide (50 mg, 0.21 mmol) and N, N-diisopropylethylamine (40 mg, 0.31 mmol) were added to the system for 0.5 h at room temperature. After the end of the reaction, water was added until there was no longer yellow solid precipitation in the system, and the solid was obtained by column chromatography to obtain 35 mg of compound 016 solid in 60% yield. ESI-MS(M+H)⁺=778. ¹H-NMR (400 MHz, CCl₃D) δ 10.95 (brs, 1H), 8.37 (brs, 1H), 7.73 (brs, 1H), 7.58 - 7.66 (m, 3H), 7.24 - 7.27 (m, 2H), 6.96 - 6.97 (m, 1H), 6.68 - 6.70 (m, 1H), 5.71 - 5.73 (m, 1H), 5.57 (brs, 1H), 4.92 - 4.96 (m, 1H), 3.78 - 4.13 (m, 4H), 3.38 - 3.61 (m, 3H), 2.63 - 3.23 (m, 6H), 2.38 - 2.51 (m, 3H), 2.03 - 2.23 (m, 4H), 1.93 (s, 3H), 1.77 - 1.85 (m, 8H), 1.27 - 1.31 (m, 3H).

### Example 7:3-((R)-3-aminopiperidin-1-yl)-5-((4-(1-((1-(2-(2,6-dioxopiperidin-3-yl)-1,3- dioxoisoindolin -5-yl) pyrrolidin-3-yl) methyl) piperidin-4-yl) phenyl) amino)-1,2,4-triazine-6-carboxamide (029)

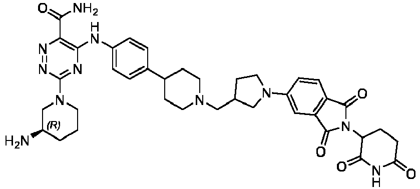

016-2 (145 mg, 0.22 mmol) was dissolved in 5mL DCM, and (R)-piperidine-3-ylcarbamate tert-butyl ester (65 mg, 0.32 mmol) and N, N-diisopropylethylamine (40 mg, 0.31 mmol) were added to the system for 0.5 h at room temperature. After the reaction, water was added to quench, DCM extracted, saturated ammonium chloride aqueous solution washed, concentrated and purified by column chromatography to obtain purified solids. The above solids were dissolved in 3 mL DCM, and 1,4-dioxane solution of hydrogen chloride (0.3 mL, 4.0 mol/L) was added dropwise, and stirred for 0.5h at room temperature. At the end of the reaction, it was concentrated under reduced pressure to obtain 35 mg of compound 029 yellow solid with a yield of 97%. ESI-MS(M+H)⁺=736. ¹H-NMR (400 MHz, DMSO-d₆) δ 11.52 (brs, 1H), 11.07 (brs, 1H), 8.31 - 8.39 (m, 3H), 7.85 (s, 1H), 7.65 - 7.70 (m, 3H), 7.32 - 7.33 (m, 2H), 6.95 (s, 1H), 6.83 - 6.85 (m, 1H), 5.03 - 5.08 (m, 1H), 3.78 - 3.82 (m, 1H), 3.60 - 3.68 (m, 4H), 3.05 - 3.24 (m, 6H), 2.83 - 2.92 (m, 3H), 2.54 - 2.63 (m, 2H), 2.16 - 2.32 (m, 2H), 1.62 - 2.02 (m, 10H), 1.23 - 1.27 (m, 3H). **Referring to the synthesis route and method of embodiment 1-7, the following target compounds are synthesized:**

| Example | Compound No. | Structural formula | Characterization data |
|---|---|---|---|
| 8 | 004 | 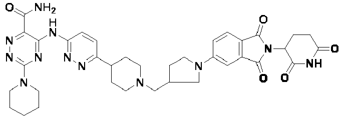 | ESI-MS(M+H) ⁺=723 |
| 9 | 005 | 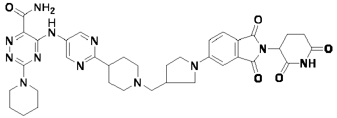 | ESI-MS(M+H) ⁺=723 |
| 10 | 006 | 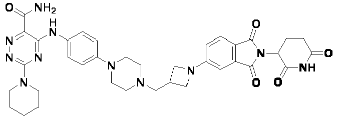 | ESI-MS(M+H) ⁺=708 |
| 11 | 008 | 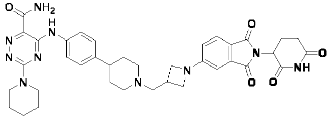 | ESI-MS(M+H) ⁺=707 |
| 12 | 009 | 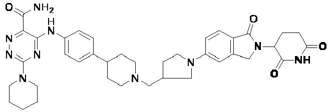 | ESI-MS(M+H) ⁺=707 |
| 13 | 010 | 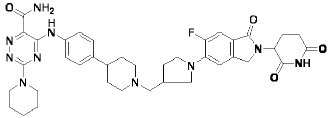 | ESI-MS(M+H) ⁺=725 |
| 14 | 012 | 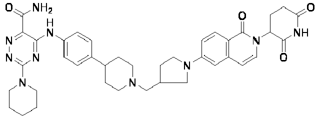 | ESI-MS(M+H) ⁺=719 |
| 15 | 013 | 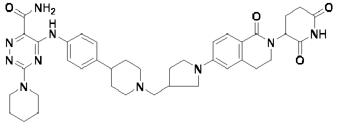 | ESI-MS(M+H) ⁺=721 |
| 16 | 015 | 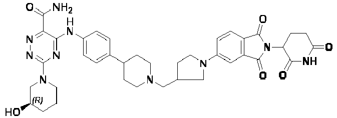 | ¹H-NMR (400 MHz, CDCl₃) δ 10.92 (brs, 1H), 8.01 (s, 1H), 7.59 -7.71 (m, 4H), 7.24 - 7.25 (m, 2H), 6.97 - 6.98 (m, 1H), 6.68 - 6.71 (m, 1H), 5.40 (brs, 1H), 4.91 - 4.96 (m, 1H), 3.89 - 3.91 (m, 1H), 3.41 - 3.72 (m, 6H), 2.68 - 3.24 (m, 4H), 2.42 - 2.51 (m, 3H), 1.98 - 2.23 (m, 5H), 1.66 -1.87 (m, 9H), 1.29 - 1.32 (m, 4H). ESI-MS(M+H) ⁺=737 |
| 17 | 017 | 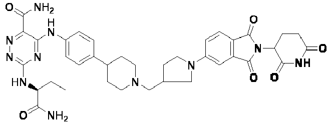 | ESI-MS(M+H) ⁺=738 |
| 18 | 018 | 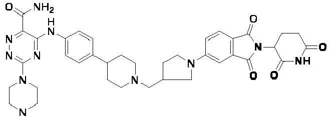 | ¹H-NMR (400 MHz, CDCl₃) δ 10.93 (brs, 1H), 8.12 - 8.16 (m, 1H), 7.58 -7.71 (m, 4H), 7.22 - 7.24 (m, 2H), 6.97 (s, 1H), 6.69 - 6.71 (m, 1H), 5.40 (brs, 1H), 4.91 - 4.96 (m, 1H), 3.93 - 4.01 (m, 4H), 3.41 - 3.63 (m, 3H), 2.69 - 3.25 (m, 6H), 2.52 - 2.55 (m, 4H), 2.37 (s, 3H), 2.21 - 2.23 (m, 4H), 1.83 - 2.02 (m, 6H), 1.29 - 1.33 (m, 3H). ESI-MS(M+H) ⁺=736 |
| 19 | 019 | 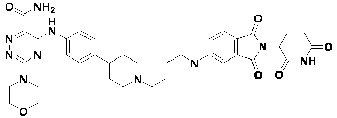 | ¹H-NMR (400 MHz, CDCl₃) δ 10.96 (brs, 1H), 8.13 (s, 1H), 7.56 -7.73 (m, 4H), 7.24 - 7.25 (m, 2H), 6.97 - 6.98 (m, 1H), 6.67 - 6.70 (m, 1H), 5.46 (brs, 1H), 4.91 - 4.96 (m, 1H), 3.79 - 3.94 (m, 8H), 3.39 - 3.61 (m, 2H), 3.19 - 3.24 (m, 1H), 2.66 - 3.10 (m, 6H), 2.42 - 2.51 (m, 2H), 2.07 - 2.24 (m, 2H), 1.83 - 1.88 (m, 6H), 1.27 - 1.34 (m, 3H). ESI-MS(M+H) ⁺=723 |
| 20 | 020 | 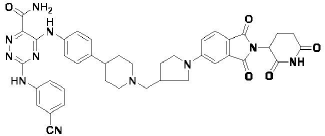 | ESI-MS(M+H) ⁺=754 |
| 21 | 021 | 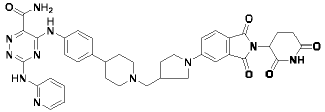 | ESI-MS(M+H) ⁺=730 |
| 22 | 022 | 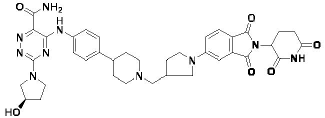 | ESI-MS(M+H) ⁺=723 |
| 23 | 023 | 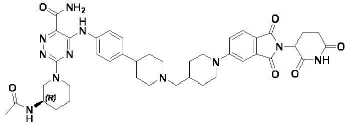 | ¹H-NMR (400 MHz, CDCl₃) δ 10.88 (brs, 1H), 8.33(s, 1H), 7.65 - 7.66 (m, 1H), 7.59 - 7.62 (m, 1H), 7.51 - 7.53 (m, 2H), 7.20 - 7.21 (m, 2H), 7.16 - 7.17 (m, 1H), 6.96 - 6.98 (m, 1H), 5.59 - 5.61 (m, 1H), 5.47 (brs, 1H), 4.84 - 4.89 (m, 1H), 3.86 - 4.05 (m, 4H), 3.24 - 3.26 (m, 2H), 2.64 - 2.94 (m, 6H), 2.46 - 2.53 (m, 3H), 2.24 - 2.30 (m, 4H), 1.91 - 2.18 (m, 4H), 1.62 - 1.74 (m, 8H), 1.25 - 1.27 (m, 5H). ESI-MS(M+H) ⁺=792 |
| 24 | 030 | 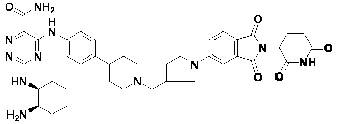 | ESI-MS(M+H) ⁺=750 |
| 25 | 031 | 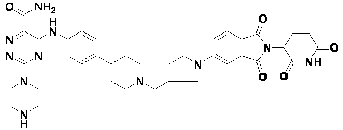 | ¹H-NMR (400 MHz, DMSO-d6) δ 11.54 (brs, 1H), 11.08 (brs, 1H), 9.38 (s, 1H), 8.46 (s, 1H), 7.90 (s, 1H), 7.63 - 7.69 (m, 3H), 7.28 - 7.31 (m, 2H), 6.95 - 6.96 (m, 1H), 6.82 - 6.85 (m, 1H), 5.04 - 5.08 (m, 1H), 4.06 - 4.08 (m, 4H), 3.24 - 3.32 (m, 8H), 3.06 - 3.10 (m, 2H), 2.81 - 2.93 (m, 3H), 2.55 - 2.66 (m, 2H), 2.18 - 2.33 (m, 3H), 1.87 - 2.02 (m, 5H), 1.24 - 1.30 (m, 3H).ESI-MS(M+H)⁺=722 |
| 26 | 032 | 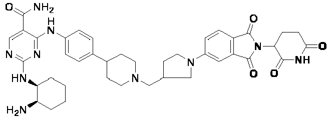 | ESI-MS(M+H) ⁺=749 |
| 27 | 033 | 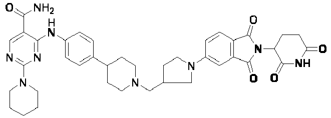 | ¹H-NMR (400 MHz, CCl₃D) δ 10.89 (brs, 1H), 8.32 (s, 1H), 7.95 (brs, 1H), 7.59 - 7.66 (m, 3H), 7.18 - 7.22 (m, 2H), 6.96 - 6.97 (m, 1H), 6.68 - 6.70 (m, 1H), 5.33 - 5.50 (m, 2H), 4.92 - 4.95 (m, 1H), 3.80 - 3.84 (m, 4H), 3.39 - 3.61 (m, 3H), 3.17 - 3.22 (m, 1H), 2.64 - 3.09 (m, 4H), 2.38 - 2.49 (m, 3H), 2.01 - 2.23 (m, 4H), 1.62 - 1.87 (m, 10H), 1.30 - 1.32 (m, 3H). ESI-MS(M+H) ⁺=720 |
| 28 | 034 | 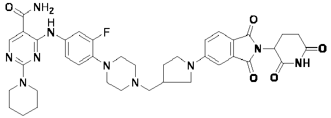 | ESI-MS(M+H) ⁺=739 |
| 29 | 035 | 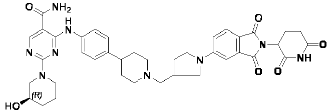 | ESI-MS(M+H) ⁺=736 |
| 30 | 036 | 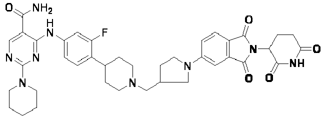 | ESI-MS(M+H) ⁺=738 |
| 31 | 037 | 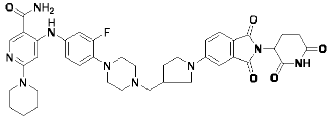 | ESI-MS(M+H) ⁺=738 |
| 32 | 038 | 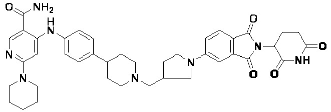 | ESI-MS(M+H) ⁺=719 |
| 33 | 039 | 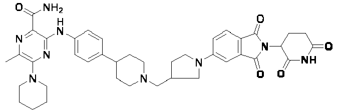 | ESI-MS(M+H) ⁺=734 |
| 34 | 040 | 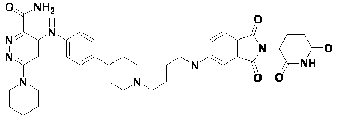 | ESI-MS(M+H) ⁺=720 |
| 35 | 041 | 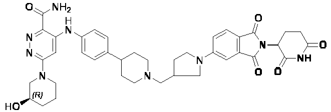 | ESI-MS(M+H) ⁺=736 |
| 36 | 042 | 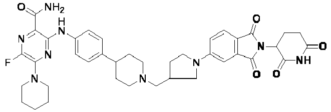 | ESI-MS(M+H) ⁺=738 |
| 37 | 043 | 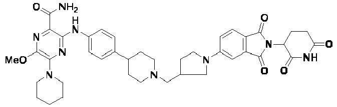 | ESI-MS(M+H) ⁺=750 |
| 38 | 067 | 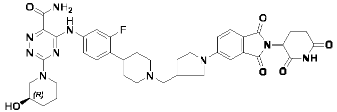 | ESI-MS(M+H) ⁺=755 |

### Example 39: 5-((4-(1-((1-(2-(2,6-dioxopiperidin-3-yl)-1,3-dioxoisoindolin-5-yl) pyrrolidin-3-yl) methyl) piperidin-4-yl) phenyl) amino)-3-(thiazol-2-yl)-1,2,4-triazine-6-carboxamide (024)

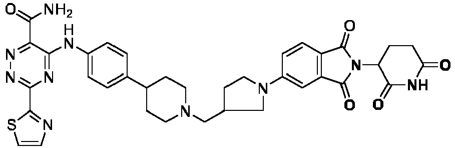

A mixture of 016-2 (50 mg, 0.07 mmol), 2-(tributyl tinyl) thiazole (45 mg, 0.12 mmol), sodium carbonate (22 mg, 0.21 mmol), tetras(triphenylphosphine)palladium (0) (25 mg), and 1,4-dioxane solution (1 mL) were stirred overnight at 50°C for the reaction. At the end of the reaction, water and DCM were added to extract, the organic phase was separated, the anhydrous magnesium sulfate was dried, filtered and concentrated, and the compound 024 solid 21 mg was purified by column chromatography in a yield of 43%. ESI-MS(M+H) ⁺= 721.

Referring to the synthesis route and method of embodiment 39, the following target compounds are synthesized and obtained:

| Example | Compound No. | Structural formula | Characterization data |
|---|---|---|---|
| 40 | 025 | 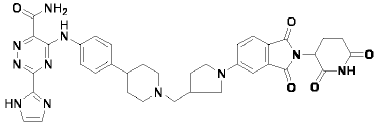 | ESI-MS(M+H) ⁺=704 |
| 41 | 026 | 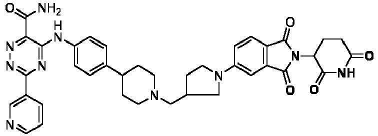 | ESI-MS(M+H) ⁺=715 |

### Example 42: 5-((4-(1-((1-(2-(2,6-dioxopiperidin-3-yl)-1,3-dioxoisoindolin-5-yl) pyrrolidin-3-yl) methyl) piperidin-4-yl) phenyl) amino)-3-(3-hydroxyphenoxy)-1,2,4-triazine-6-carboxamide (027)

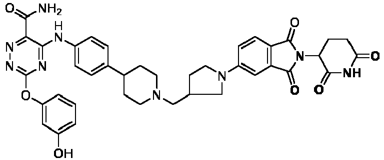

016-2 (50 mg, 0.07 mmol) was dissolved in 2 mL of DMSO, and resorcinol (13 mg, 0.12 mmol) and cesium carbonate (39 mg, 0.12 mmol) were added to the system for 3 h at 50°C. At the end of the reaction, water and DCM were added to extract, the organic phase was separated, the anhydrous magnesium sulfate was dried, filtered and concentrated, and the compound 027 solid 37 mg was purified by column chromatography in a yield of 70%. ESI-MS(M+H) ⁺= 746.

### Example 43: 3-(2,3-difluorophenoxy)-5-((4-(1-((1-(2-(2,6-dioxopiperidin-3-yl)-1,3- dioxoisoindolin-5-yl) pyrrolidin-3-yl)methyl)piperidin-4-yl)phenyl)amino)-1,2,4-triazine-6-carboxamide (028)

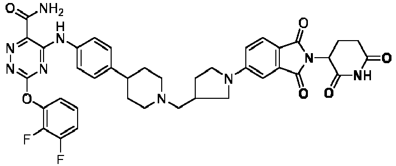

Referring to the synthesis method and route of embodiment 42, resorcinol is replaced with 2,3-difluorophenol, and compound 028 can be synthesized to obtain ESI-MS(M+H) ⁺=766.

### Example 44: 5-((4-(4-((1-(4-(2,4-dioxotetrahydropyrimidin-1(2H)-yl)-3-methylphenyl) piperidin-4-yl) methyl) piperazin-1-yl)-3-fluorophenyl) amino)-3-(piperidin-1-yl)-1,2,4-triazine-6-carboxamide (075)

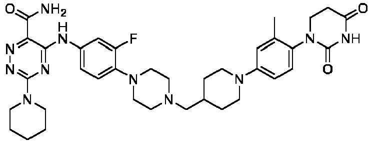

### Step 1: 4-(4-((6-carbamoyl-3-(piperidin-1-yl)-1,2,4-triazine-5-yl) amino)-2-fluorophenyl) piperazine -1-carboxylate tert-butyl ester (075-1)

Intermediate 4 (1.6 g, 3.45 mmol) was dissolved in 25 mL of NMP, mCPBA (85%, 2.9 g, 17.25 mmol) was added to the system, and stirred for 2 h at room temperature. At the end of the reaction, DIPEA (1.3 g, 10.35 mmol) and piperidine (439 mg, 5.17 mmol) were added to the system for 2 h at 80°C. At the end of the reaction, the solid precipitation is precipitated by adding water, the yellow solid is filtered, the DCM is added to dissolve, the anhydrous sodium sulfate is dried, and the concentration is reduced pressure. Tert-butyl piperazine-1-carboxylic acid 1.7 g was obtained by column chromatography (DCM/MeOH elution) in yellow solid 4-(4-(6-carbamoyl- 3-(piperidin-1-yl)-1,2,4-triazine-5-yl) amino)-2-fluorophenyl) piperazine-1-carboxylic acid in a yield of 98%. ESI-MS(M+H) ⁺ =501.5.

### Step 2: 5-((4-(4-((1-(4-(2,4-dioxotetrahydropyrimidin-1(2H)-yl)-3-methylphenyl) piperidin-4-yl) methyl) piperazin-1-yl)-3-fluorophenyl) amino)-3-(piperidin-1-yl)-1,2,4-triazine-6-carboxamide (075)

Intermediate 075-1 (100 mg, 0.25 mmol) was dissolved in 3 mL DCM, 1.5 mL trifluoroacetic acid was added to the system, and the reaction was carried out at room temperature for 30 min. At the end of the reaction, 5-((3- fluoro-4- (piperazine-1-yl) phenyl) amino)-3-(pipeiidin-1-yl)-1,2,4-triazine-6-carboxamide was obtained under reduced pressure. The obtained product was dissolved in 2mL DCM, intermediate 29 (118mg, 0.37mmol) was added to the system, stirred at room temperature for 10min, acetic acid (0.1mL) and sodium cyanoboron hydride (47mg, 0.75mmol) were added to the system, and the reaction was carried out at room temperature for 0.5h. At the end of the reaction, water was added to quench, DCM extraction, organic phases were combined, anhydrous sodium sulfate was dried, and concentrated under reduced pressure. Compound 075 was obtained by column chromatography (DCM/MeOH elution) with a yield of 28%. ESI-MS(M+H)⁺ =700. ¹H-NMR (400 MHz, DMSO-d₆) δ 11.30 (brs, 1H), 10.25 (s, 1H), 8.35 (s, 1H), 7.62 - 7.72 (m, 2H), 7.25 - 7.28 (m, 1H), 7.01 - 7.06 (m, 2H), 6.76 - 6.80 (m, 2H), 3.62 - 3.83 (m, 7H), 3.32 - 3.48 (m, 1H), 2.98 - 3. 00 (m, 4H), 2.62 - 2.71 (m, 4H), 2.49 - 2.52 (m, 4H), 2.20 - 2.22 (m, 2H), 2.11 (s, 3H), 1.77 - 1.81 (m, 2H), 1.59 - 1.67 (m, 6H), 1.16 - 1.23 (m, 3H).

### Example 45: 5-((4-(4-((1-(6-((2,6-dioxopiperidin-3-yl) amino) pyridin-3-yl) piperidin-4-yl) methyl) piperazin-1-yl)-3-fluorophenyl) amino)-3-(piperidin-1-yl)-1,2,4-triazine-6-carboxamide (077)

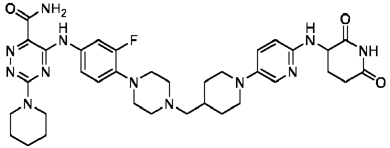

Referring to the synthesis route and method of embodiment 44, the intermediate 29 in the synthesis step 2 is replaced with the intermediate 36, and the target compound 077 is synthesized and ESI-MS(M+H) ⁺=701. ¹H-NMR (400 MHz, DMSO-d₆) δ 11.30 (brs, 1H), 10.73 (s, 1H), 8.35 (s, 1H), 7.61 - 7.72 (m, 3H), 7.19 - 7.28 (m, 2H), 7.01 - 7.04 (m, 1H), 6.51 - 6.53 (m, 1H), 6.37 - 6.39 (m, 1H), 4.60 - 4.66 (m, 1H), 3.79 - 3. 83 (m, 4H), 3.34 - 3.36 (m, 3H), 2.97 - 3.00 (m, 4H), 2.68 - 2.77 (m, 1H), 2.51 - 2.54 (m, 3H), 2.20 - 2.22 (m, 2H), 1.93 - 2.10 (m, 3H), 1.75 - 1.81 (m, 2H), 1.58 - 1.67 (m, 6H), 1.22 - 1.26 (m, 5H).

### Example 46:5-((4-(4-((4-(2-(2,6-dioxopiperidin-3-yl)-1,3-dioxoisoindolin-5-yl) piperazin-1-yl) methyl) piperidin-1-yl)-3- fluorophenyl) amino)-3-(piperidin-1-yl)-1,2,4-triazine-6-carboxamide (044)

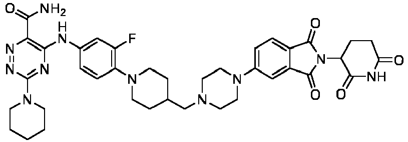

Referring to the synthesis route and method of the synthesis step 2 of embodiment 44, 075-1 is replaced with intermediate 16, intermediate 29 is replaced with intermediate 25, and compound 044 yellow solid 40 mg is synthesized in a yield of 15%. ESI-MS(M+H) ⁺ =754. 1H-NMR (400 MHz, CCl3D) δ 10.85 (brs, 1H), 8.04 (brs, 1H), 7.69 - 7.70 (m, 2H), 7.60 - 7.65 (m, 1H), 7.28 - 7.29 (m, 1H), 7.16 - 7.19 (m, 1H), 7.05 - 7.08 (m, 1H), 6.92 - 6.96 (m, 1H), 5.38 (brs, 1H), 4.92 - 4.97 (m, 1H), 3.41 - 3.47 (m, 6H), 2.59 - 2.92 (m, 9H), 2.29 - 2.31 (m, 2H), 2.11 - 2.17 (m, 3H), 1.87 - 2.01 (m, 2H), 1.65 - 1.74 (m, 8H), 1.29 - 1.46 (m, 3H).
**Referring to the synthesis route and method of embodiment 44-46, the following target compounds are synthesized:**

| Example | Compound No. | Structural formula | Characterization data |
|---|---|---|---|
| 47 | 014 | 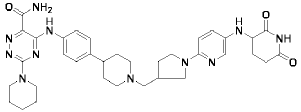 | ESI-MS(M+H)⁺=668 |
| 48 | 045 | 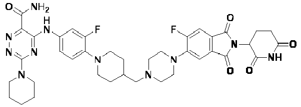 | ¹H-NMR (400 MHz, CCl₃D) δ 10.85 (brs, 1H), 7.98 (brs, 1H), 7.60 - 7.69 (m, 2H), 7.46 - 7.48 (m, 1H), 7.38 - 7.39 (m, 1H), 7.16 - 7.19 (m, 1H), 6.92 - 6.99 (m, 1H), 5.34 - 5.35 (m, 1H), 4.91 - 4.95 (m, 1H), 3.43 - 3.46 (m, 2H), 3.28 - 3.31 (m, 5H), 2.59 - 2.93 (m, 11H), 2.31 - 2.32 (m, 2H), 2.11 - 2.16 (m, 1H), 1.86-2.04 (m, 3H), 1.66 - 1.72 (m, 9H).ESI-MS(M+H)⁺=772 |
| 49 | 046 | 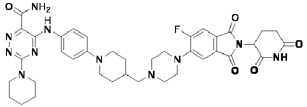 | ¹H-NMR (400 MHz, CDCl₃) δ 10.72 (brs, 1H), 8.04 (s, 1H), 7.68 - 7.70 (m, 1H), 7.52 - 7.55 (m, 2H), 7.46 - 7.49 (m, 1H), 7.38 - 7.39 (m, 1H), 6.93 - 6.96 (m, 2H), 5.34 (brs, 1H), 4.91 - 4.96 (m, 1H), 3.85 - 3.93 (m, 4H), 3.66 - 3.69 (m, 2H), 3.30 (s, 4H), 2.62 - 2.92 (m, 7H), 2.29 - 2.34 (m, 2H), 2.11 - 2.16 (m, 1H), 1.88 - 1.92 (m, 2H), 1.65 - 1.72 (m, 8H), 1.29 - 1.40 (m, 3H). ESI-MS(M+H)⁺=754 |
| 50 | 047 | 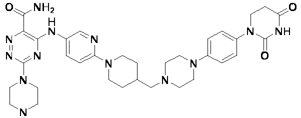 | ESI-MS(M+H)⁺=684 |
| 51 | 048 | 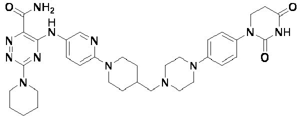 | ESI-MS(M+H)⁺=669 |
| 52 | 049 | 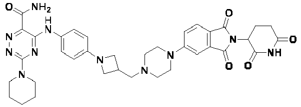 | ESI-MS(M+H)⁺=708 |
| 53 | 050 | 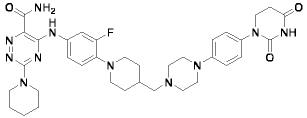 | ¹H-NMR (400 MHz, DMSO-d₆) δ 11.30 (brs, 1H), 10.26 (s, 1H), 8.32 (s, 1H), 7.59 - 7.70 (m, 2H), 7.23 - 7.26 (m, 1H), 7.12 - 7.15 (m, 2H), 7.01 - 7.05 (m, 1H), 6.91 - 6.93 (m, 2H), 3.66 - 3.83 (m, 6H), 3.00 - 3.12 (m, 6H), 2.60 - 2.68 (m, 4H), 2.21 - 2.31 (m, 3H), 1.93 - 2.02 (m, 1H), 1.78 - 1.83 (m, 2H),1.56 - 1.66 (m, 7H), 1.24 - 1.29 (m, 4H).ESI-MS(M+H)⁺=686 |
| 54 | 051 | 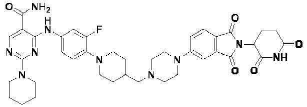 | ESI-MS(M+H)⁺=753 |
| 55 | 052 | 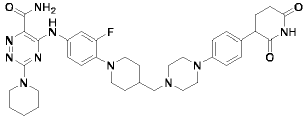 | ESI-MS(M+H)⁺=685 |
| 56 | 053 | 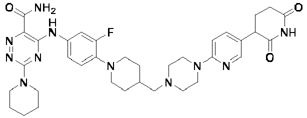 | ESI-MS(M+H)⁺=686 |
| 57 | 054 | 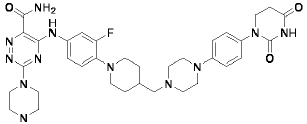 | ESI-MS(M+H)⁺=701 |
| 58 | 076 | 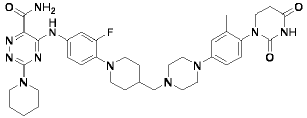 | ESI-MS(M+H)⁺=700 |
| 59 | 078 | 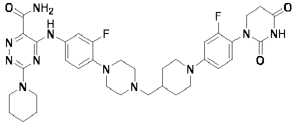 | ESI-MS(M+H)⁺=704 |
| 60 | 079 | 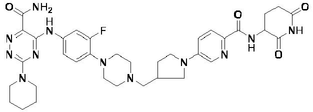 | ¹H-NMR (400 MHz, DMSO-d₆) δ 11.30 (brs, 1H), 10.84 (s, 1H), 8.61 - 8.63 (m, 1H), 8.34 (s, 1H), 7.90 - 7.91 (m, 1H), 7.81 - 7.83 (m, 1H), 7.61 - 7.70 (m, 2H), 7.26 - 7.29 (m, 1H), 6.95 - 7.06 (m, 2H), 4.68 - 4.76 (m, 1H), 3.82 - 3.83 (m, 3H), 3.44 - 3.52 (m, 2H), 3.00 - 3.10 (m, 5H), 2.72 - 2.82 (m, 1H), 2.53 - 2.65 (m, 5H), 2.39 - 2.41 (m, 2H), 1.96 - 2.17 (m, 3H), 1.56 - 1.76 (m, 8H), 1.21 - 1.24 (m, 2H).ESI-MS(M+H)⁺=715 |
| 61 | 080 | 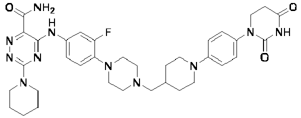 | ESI-MS M+H)⁺=686 |
| 62 | 081 | 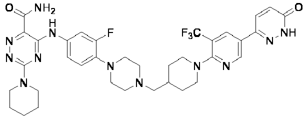 | ¹H-NMR (400 MHz, DMSO-d₆) δ 11.35 (brs, 1H), 8.81 - 8.91 (m, 2H), 8.35 - 8.37 (m, 2H), 8.09 - 8.12 (m, 1H), 7.65 - 7.72 (m, 2H), 7.31 - 7.34 (m, 1H), 6.99 - 7.11 (m, 2H), 4.09 - 4.11 (m, 2H), 3.69 - 3.86 (m, 6H), 3.56 - 3.61 (m, 3H), 2.92 - 3.11 (m, 7H), 1.89 - 1.99 (m, 2H), 1.57 - 1.65 (m, 7H), 1.27 - 1.29 (m, 2H).ESI-MS(M+H)⁺=737 |
| 63 | 082 | 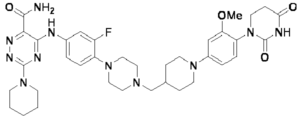 | ESI-MS(M+H)⁺=716 |
| 64 | 083 | 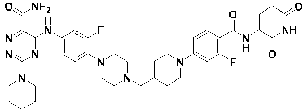 | ESI-MS(M+H)⁺=746 |
| 65 | 084 | 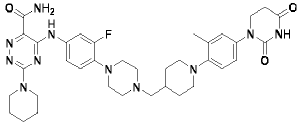 | ESI-MS(M+H)⁺=700 |
| 66 | 085 | 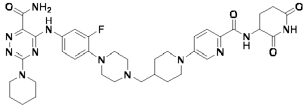 | ESI-MS(M+H)⁺=729 |
| 67 | 086 | 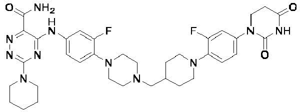 | ESI-MS(M+H)⁺=704 |
| 68 | 087 | 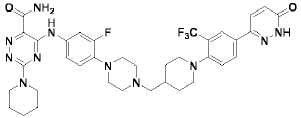 | ESI-MS(M+H)⁺=736 |
| 69 | 088 | 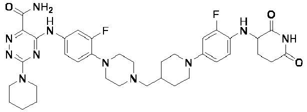 | ESI-MS(M+H)⁺=718 |
| 70 | 089 | 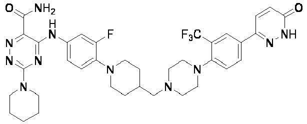 | ESI-MS(M+H)⁺=736 |
| 71 | 090 | 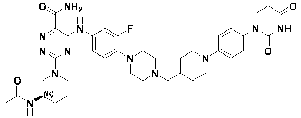 | ESI-MS(M+H)⁺=757 |
| 72 | 091 | 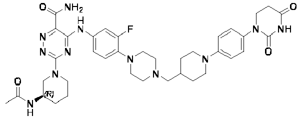 | ESI-MS(M+H)⁺=743 |
| 73 | 092 | 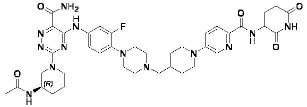 | ESI-MS(M+H)⁺=786 |
| 74 | 093 | 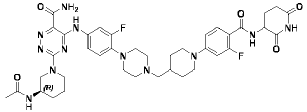 | ESI-MS(M+H)⁺=803 |
| 75 | 094 | 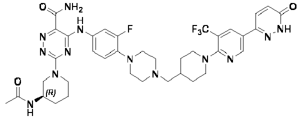 | ESI-MS(M+H)⁺=794 |
| 76 | 095 | 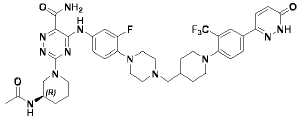 | ESI-MS(M+H)⁺=793 |
| 77 | 096 | 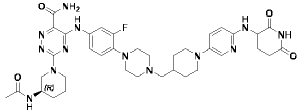 | ESI-MS(M+H)⁺=758 |
| 78 | 097 | 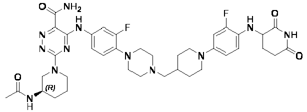 | ESI-MS(M+H)⁺=775 |
| 79 | 098 | 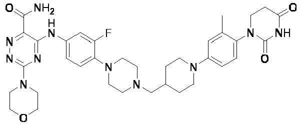 | ESI-MS(M+H)⁺=702 |
| 80 | 099 | 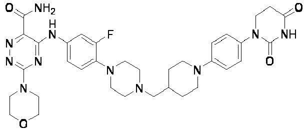 | ESI-MS(M+H)⁺=688 |
| 81 | 100 | 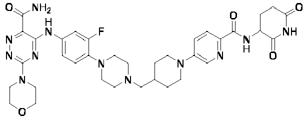 | ESI-MS(M+H)⁺=731 |
| 82 | 101 | 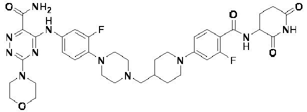 | ESI-MS(M+H)⁺=748 |
| 83 | 102 | 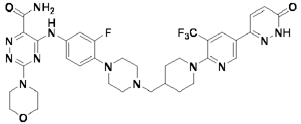 | ESI-MS(M+H)⁺=739 |
| 84 | 103 | 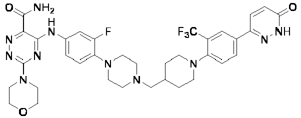 | ESI-MS(M+H)⁺=738 |
| 85 | 104 | 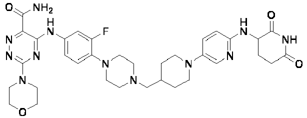 | ESI-MS(M+H)⁺=703 |
| 86 | 105 | 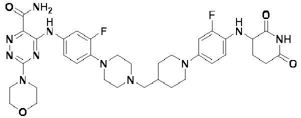 | ESI-MS(M+H)⁺=720 |

### Example 87: 5-((4-(1-(1-(2-(2,6-dioxopiperidin-3-yl)-1,3-dioxoisoindolin-5-yl) pyrrolidine-3-carbonyl) piperidin-4-yl) phenyl) amino)-3-(piperidin-1-yl)-1,2,4-triazine-6-carboxamide (055)

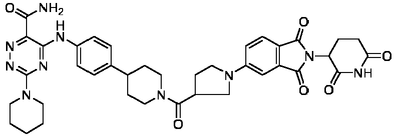

### Step 1: 4-(4-((6-carbamoyl-3-(piperidin-1-yl)-1,2,4-triazine-5-yl) amino) phenyl) piperidin-1-carboxylic acid tert-butyl ester (055-1)

Referring to the synthesis route and method of the synthesis step 1 of embodiment 44, the intermediate 4 is replaced with intermediate 2, and the tert-butyl ester of 4-(4-((6-carbamoyl-3-(piperidin-1-yl)-1,2,4-triazin-5-yl) amino) phenyl) piperidin-1-carboxylic acid is synthesized. ESI-MS(M+H) ⁺=482.

### Step 2: 5-((4-(1-(1-(2-(2,6-dioxopiperidin-3-yl)-1,3-dioxoisoindolin-5-yl) pyrrolidine-3- carbonyl) piperidin-4-yl) phenyl) amino) -3-(piperidin-1-yl)-1,2,4- triazine-6- carboxamide (055)

055-1 was dissolved in 3 mL of DCM, 1.5 mL of trifluoroacetic acid was added to the system, and the reaction was carried out at room temperature for 30 min. At the end of the reaction, the obtained product (102mg, 0.27mmol) was dissolved in 5 mL of DMF, and intermediates 24 (100mg, 0.27mmol), HATU (112mg, 0.29mmol) and TEA (81mg, 0.80mmol) were added to the system in turn, and the reaction was completed at 30°C for 2h. Water was added to quench, DCM was extracted three times, the organic phase was combined, the organic phase was washed with saturated ammonium chloride aqueous solution, the anhydrous sodium sulfate was dried, and the compound 055 solid was 60 mg by column chromatography with a yield of 30%, ESI-MS(M+H)⁺=735.
**Referring to the synthesis route and method of embodiment 87, the following compounds are synthesized and obtained:**

| Example | Compound No. | Structural formula | Characterization data |
|---|---|---|---|
| 88 | 056 | 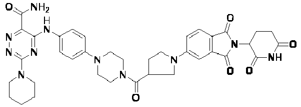 | ESI-MS(M+H)⁺=736 |
| 89 | 057 | 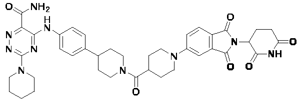 | ESI-MS(M+H)⁺=749 |
| 90 | 058 | 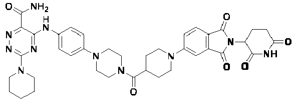 | ESI-MS(M+H)⁺=750 |
| 91 | 059 | 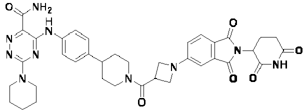 | ESI-MS(M+H)⁺=721 |
| 92 | 060 | 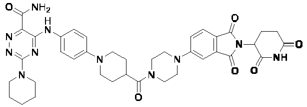 | ESI-MS(M+H)⁺=750 |
| 93 | 061 | 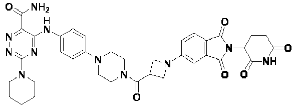 | ESI-MS(M+H)⁺=722 |

### Example 94: (3-(5-(3-((4-(4-((6-carbamoyl-3-(piperidin-1-yl)-1,2,4-triazin-5-yl) amino) phenyl) piperidin -1-yl) methyl) pyrrolidin-1-yl)-1,3-dioxoisoindolin-2-yl)-2,6-dioxopiperidin-1-yl) methyl pivalate (062)

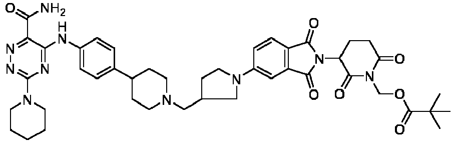

Compound 001 (100 mg, 0.12 mmol) was dissolved in 2 mL of DMF, and cesium carbonate (46 mg, 0.14 mmol) and chloromethyl neovalerate (18 mg, 0.12 mmol) were added to the system for 12 h at room temperature. At the end of the reaction, water was added to quench, EA was extracted three times, organic phases were combined, anhydrous sodium sulfate was dried, and concentrated under reduced pressure to obtain compound 062 yellow solid 30 mg, yield: 26%. ESI-MS(M+H) ⁺= 835. ¹H-NMR (400 MHz, CDCl₃) δ 10.89 (brs, 1H), 7.60 - 7.70 (m, 4H), 7.23 - 7.24 (m, 2H), 6.96 - 6.97 (m, 1H), 6.68 - 6.71 (m, 1H), 5.79 - 5.84 (m, 2H), 5.40 (brs, 1H), 4.97 - 5.01 (m, 1H), 3.86 - 3.95 (m, 4H), 3. 44 - 3.61 (m, 2H), 3.19 - 3.23 (m, 1H), 2.99 - 3.03 (m, 2H), 2.79 - 2.86 (m, 2H), 2.44 - 2.51 (m, 3H), 2.03 - 2.24 (m, 5H), 1.70 - 1.85 (m, 10H), 1.25 - 1.32 (m, 3H), 1.18 (s, 9H).
**Referring to the synthesis route and method of embodiment 94, the following target compound is synthesized and obtained:**

| Example | Compound No. | Structural formula | Characterization data |
|---|---|---|---|
| 95 | 063 | 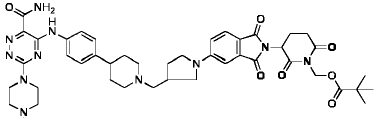 | ESI-MS(M+H)⁺=850 |
| 96 | 064 | 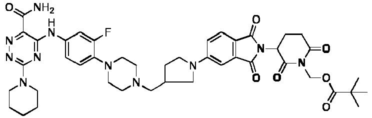 | ESI-MS(M+H)⁺=854 |
| 97 | 065 | 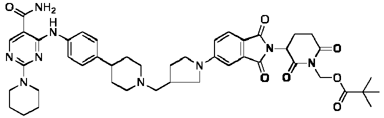 | ESI-MS(M+H)⁺=834 |
| 98 | 066 | 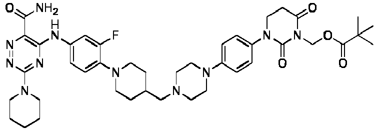 | ESI-MS(M+H)⁺=800 |
| 99 | 068 | 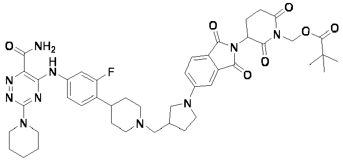 | ESI-MS(M+H)⁺=853 |
| 100 | 069 | 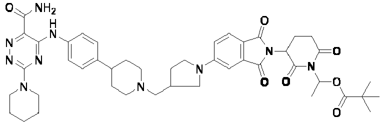 | ESI-MS(M+H)⁺=849 |
| 101 | 070 | 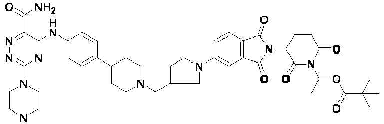 | ESI-MS(M+H)⁺=864 |
| 102 | 071 | 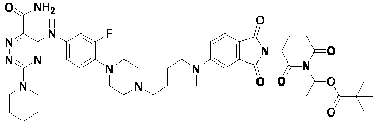 | ESI-MS(M+H)⁺=868 |
| 103 | 072 | 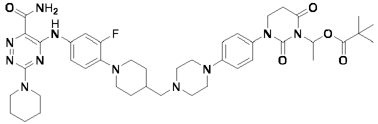 | ESI-MS(M+H)⁺=814 |
| 104 | 073 | 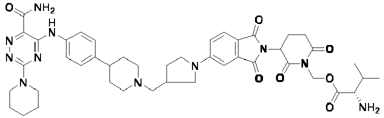 | ESI-MS(M+H)⁺=850 |
| 105 | 074 | 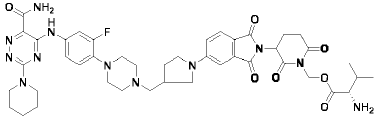 | ESI-MS(M+H)⁺=869 |

### Example 106: BTK degradation activity test

The Mino cell line cells are 0.5~1 million/well in 6-well plates. The compounds are diluted to different concentrations, added to the plate, and after 24-72 h of incubation, the cells are collected. After the cells were lysed, the total protein concentration in each well was determined with a BCA kit. The amount of BTK is determined using the Westemblot or Elisa method.

**Table 1 BTK degradation activity**

| **Compound No.** | **DC₅₀** | **Compound** No. | **DC₅₀** | **Compound** No. | **DC₅₀** |
|---|---|---|---|---|---|
| 001 | A | 002 | A | 003 | A |
| 004 | A | 005 | A | 006 | A |
| 007 | A | 008 | A | 009 | A |
| 010 | A | 011 | A | 012 | A |
| 013 | A | 014 | A | 015 | A |
| 016 | A | 017 | A | 018 | A |
| 019 | A | 020 | A | 021 | A |
| 022 | A | 023 | A | 024 | A |
| 025 | A | 026 | A | 027 | A |
| 028 | A | 029 | A | 030 | A |
| 031 | A | 032 | A | 033 | A |
| 034 | A | 035 | A | 036 | A |
| 037 | A | 038 | A | 039 | A |
| 040 | A | 041 | A | 042 | A |
| 043 | A | 044 | A | 045 | A |
| 046 | A | 047 | A | 048 | A |
| 049 | A | 050 | A | 051 | A |
| 052 | A | 053 | A | 054 | A |
| 055 | A | 056 | A | 057 | A |
| 058 | A | 059 | A | 060 | A |
| 061 | A | 062 | A | 063 | A |
| 064 | A | 065 | A | 066 | A |
| 067 | A | 068 | A | 069 | A |
| 070 | A | 071 | A | 072 | A |
| 073 | A | 074 | A | 075 | A |
| 076 | A | 077 | A | 078 | A |
| 079 | A | 080 | A | 081 | A |
| 082 | A | 083 | A | 084 | A |
| 085 | A | 086 | A | 087 | A |
| 088 | A | 089 | A | 090 | A |
| 091 | A | 092 | A | 093 | A |
| 094 | A | 095 | A | 096 | A |
| 097 | A | 098 | A | 099 | A |
| 100 | A | 101 | A | 102 | A |
| 103 | A | 104 | A | Compound a | A |
| Compound b | A | | | | |

| | | | | | |
|---|---|---|---|---|---|
| Note: A: DC₅₀ < 10 nM, | | | | | |

Compound a is derived from Compound 149 in the patent WO2021113557A1;
Compound b is derived from Compound 44 in the patent WO2021113557A1:
   The compounds of the present invention have a degrading effect on BTK.

### Example 107. Test of inhibitory activity on tumor cell proliferation

The anti-tumor efficacy of the compounds was tested by measuring their inhibitory effects on OCI-LY10 and Mino cell proliferation. OCI-LY10 and Mino cells were cultured in RPMI-1640 medium containing 10% fetal calf serum. OCI-LY10 and Mino cells were digested, inoculated in a 96-well plate at a cell concentration of 10000 cells/well, and incubated overnight at 37°C, 5% CO₂. Compounds of different concentrations (1000 nM, 4-fold dilution, 8 points) were added to the 96-well plate and incubated for 72 h or 168 h at 37°C and 5% CO₂, and then 20 uL of MTS was added to each well. After incubation for 2 h, 25 µl of 10% SDS was added to each well to terminate the reaction. The absorbance at 490 nm and 650 nm were measured with a microplate reader. IC₅₀ was calculated using GraphPad Prism 5.0.

**Table 2 Inhibitory activity on Mino cell proliferation**

| **Compound No.** | **Mino IC₅₀ (nM)** | **Compound No.** | **Mino IC₅₀ (nM)** |
|---|---|---|---|
| 001 | 5.50 | 044 | 3.46 |
| 004 | 1.15 | 045 | 3.91 |
| 005 | 2.47 | 050 | 6.05 |
| 007 | 5.31 | 075 | 1.42 |
| 011 | 4.20 | 082 | 2.30 |
| 012 | 1.22 | 084 | 3.30 |
| 013 | 3.37 | 087 | 4.31 |
| 014 | 4.14 | 090 | 1.94 |
| 016 | 1.45 | 092 | 3.28 |
| 019 | 2.13 | 098 | 3.11 |
| 023 | 6.75 | 100 | 3.83 |
| 033 | 9.10 | Compound a | 10.56 |

**Table 3 Inhibitory activity on OCI-LY10 cell proliferation**

| **Compound No.** | **OCI-1y10 IC₅₀ (nM)** | **Compound No.** | **OCI-1y10 IC₅₀ (nM)** |
|---|---|---|---|
| 001 | 3.00 | 077 | 1.59 |
| 004 | 0.18 | 078 | 0.70 |
| 005 | 0.41 | 079 | 1.15 |
| 014 | 0.67 | 082 | 0.64 |
| 016 | 0.32 | 083 | 1.24 |
| 019 | 1.10 | 085 | 0.57 |
| 023 | 0.69 | 086 | 0.70 |
| 048 | 0.61 | 088 | 0.66 |
| 052 | 1.39 | 090 | 0.89 |
| 053 | 1.73 | 092 | 0.24 |
| 075 | 0.51 | 100 | 0.39 |
| 076 | 1.09 | Compound a | 10.96 |

Tables 2 and 3 show that the compounds of the present invention have obvious proliferation inhibitory activity on tumor cells.

### Example 108. Liver microsomal metabolic stability

Under the condition of ice bath, 1 mg of human or Mouse liver microparticles was add to PBS (1 mL) buffer, then NADPH (20 µL) was added, and incubated at 37°C for 5 min. 10 µL of the compound to be tested was added, then suck several times with a pipette and divide into 3 aliquots of 330 µL;eachand then samples were taken at 0, 15, 30, 60 and 120 min respectively. 10µL of the solution was taken with a pipette each time and added to 490µL of the solution containing the internal standard compound. The resulting solution was vortexed and then placed on ice, and then sampling at another time point continues. All samples were centrifuged at 10,000 rpm for 10 min, and the supernatant was taken for LC/MS testing. The metabolism of the compound in liver microsomes was calculated based on the peak area.

**Table 4 Tests of the metabolic stability of compounds on liver particles**

| **Compound No.** | **Thalf (min)** | |
|---|---|---|
| | **Human** | **Mouse** |
| 001 | 71.37 | 47.13 |
| 002 | 120 | 54 |
| 016 | 37 | 49 |
| 023 | 20 | 59 |
| 045 | 38 | 120 |
| 077 | 57 | 79 |
| 090 | 78 | 63 |
| 092 | 41 | 90 |
| Compound b | 12.4 | 47.1 |

It can be seen from Table 4 that the compound of the present invention has good metabolic stability.

### Example 109. Mouse absorption test

Experimental method: mice were used as experimental animals, and drugs were administered intragastrically at 10 mg/kg (5% DMSO + 5% Solutol + 90% saline). The blood collection time points for intragastric administration were 0.25, 0.5, 1, 2, 4, 6, 8, and 24 h. 0.3ml of whole blood was taken, and 0.1ml of plasma was taken after centrifugation and analyzed by LC-MS.

**Table 5 Mice absorption results of compounds of the present invention**

| **Compound No.** | **DNAUC** |
|---|---|
| 001 | 2300 |
| 002 | 2223 |
| 003 | 1648 |
| 007 | 1955 |
| 044 | 1941 |
| 045 | 1686 |
| 046 | 1867 |
| Compound b | 1088 |

| | |
|---|---|
| Note: DNAUC indicates AUC (h*ng/mL)/dose (mg/kg). | |

It can be seen from Table 5, the compounds of the present invention have good oral absorption in mice.

### Example 110. Rat absorption test

SD Rats were used as experimental animals, and drugs were administered intragastrically at 10 mg/kg (5% DMSO + 5% Solutol + 90% normal saline). The blood collection time points for intragastric administration were 0.25, 0.5, 1, 2, 4, 6, 8, and 24 h. 0.3ml of whole blood was taken, and 0. 1ml of plasma was taken after centrifugation and analyzed by LC-MS.

**Table 6 Rat absorption results of compounds of the present invention**

| **Compound No.** | **DNAUC** |
|---|---|
| 002 | 252 |
| 003 | 456 |
| 045 | 716 |
| 075 | 1125 |
| 077 | 285 |
| 079 | 6611 |
| 081 | 372 |
| 085 | 579 |
| Compound a | 164 |

| | |
|---|---|
| Note: DNAUC indicates AUC (h*ng/mL)/dose (mg/kg). | |

It can be seen from Table6 that the compounds of the present invention have good oral absorption in rats.

### Example 111. Antitumor efficacy in vivo

NOD/SCID mice were inoculated subcutaneously with OCL-LY10 cells to establish a tumor model, and when the tumor grew to about 100 mm³, it was administered in groups as follows: the control group of group A was gavaged with the corresponding volume of CMC-Na solution; Group B was given 3mg/kg of the test compound by gavage; Group C was given 10mg/kg of the test compound by gavage; Group D was given 30 mg/kg of the test compound by gavage. The weight and tumor size changes of the mice were weighed twice a week, and after 3 weeks, the mice were sacrificed with cervical dislocation method, and the efficacy of the compound on the xenograft of OCL-LY10 mice was investigated.

## Claims

1. A compound, **characterized in that**, the compound has a structure shown in the general formula **(I):** 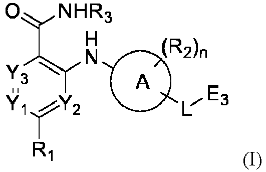
or its stereoisomer thereof, or its stereoisomer mixture thereof, or its pharmaceutically acceptable salt thereof, prodrugs;
wherein:
L is selected from: a chemical group or a chemical bond connecting the ring A and the E3;
E3 is selected from: E3 ubiquitin ligase ligand;
ring A is selected from: 3-12 membered heterocyclic ring, 6-10 membered aromatic ring, 5-12 membered heteroaromatic ring, 3-12 membered cycloalkane, 4-12 membered cycloalkyne, 3-12 membered cycloalkene, wherein the heterocyclic ring, heteroaromatic ring have 1-3 heteroatoms independently selected from N, O or S;
R₂ is selected from: H, halogen, cyano, Ci-Ce alkyl, Ci-Ce haloalkyl, C₃-C₈ cycloalkyl, C₁-C₄ alkoxy, C₃-C₈ cycloalkoxy, C₃-C₈ heterocyclyl, C₁-C₄ alkylamino, -COOH, -NH₂, OH, NO₂;
n is selected from: 0, 1, 2, 3, 4;
Y₁ is selected from: N, CR₄;
Y₂ is selected from: N, CR₅,
Y₃ is selected from: N, CR₅,
R₄ is selected from: halogen, C₁-C₄ alkyl, C₃-C₈ cycloalkyl, C₁-C₄ haloalkyl, C₃-C₈ halocycloalkyl, C₁-C₄ alkoxy, amino, carboxy, hydroxyl, cyano;
R₅ is selected from: H, halogen, C₁-C₄ alkyl, C₃-C₈ cycloalkyl, C₁-C₄ haloalkyl, C₃-C₈ halocycloalkyl, C₁-C₄ alkoxy, amino, carboxy, hydroxyl, cyano;
R₃ is selected from: H, C₁-C₈ alkyl, C₁-C₆ haloalkyl, C₃-C₈ alkenyl, C₃-C₈ alkynyl, cycloalkyl, heterocyclyl, aryl;
R₁ is selected from: NR₆R₇, OR₆, SR₆, -C(O)R₆, -C(O)OR₆ , -C(O)NR₆R₇, OC(O)R₆, OC(O)NR₆R₇, S(O)R₆, S(OX=NH)R₆, S(O)2NR₆R₇, N(R₆)C(O)R₇, N(R₆)C(O)OR₇, Ci-Cs alkyl, 3-12 membered heterocyclic ring, 5-6 membered heteroaromatic ring, C₃-C₈ cycloalkyl, aryl, where in the alkyl, heterocyclyl, heteroaromatic ring, cycloalkyl, aryl can be further substituted with one or more substituents selected from Rs;
R₈ is selected from: H, halogen, hydroxyl, amino, cyano, oxo, carboxyl, C₁-C₈ alkyl, C₁-C₈ haloalkyl, Ci-Cs alkoxy, C₃-C₈ cycloalkyl, -C(O)Ra, -C(O)ORa, -C(O)NRaRb, ORa, OC(O)Ra, OC(O)NRaRb, SRa, S(O)Ra, S(O)(=NH)Ra, S(O)₂NRaRb, NRaRb, N(Ra)C(O)Rb, N(Ra)C(O)ORb, N(Rc)C(O)NRaRb, -N(Ra)S(O)₂Rb, -N(Ra) S(O)₂NRbRc; wherein the alkyl, alkoxy and cycloalkyl can be further substituted with one or more substituents selected from: H, halogen, hydroxyl, amino, cyano, oxo, carboxyl, Ci-Cs alkyl, Ci-Cs haloalkyl, Ci-Cs alkoxy, C₃-C₈ cycloalkyl;
R₆ and R₇ are each independently selected from: H, C₁-C₈ alkyl, aminoalkyl, hydroxyalkyl, C₃-C₈ cycloalkyl, heterocyclyl, aryl, heteroaryl; wherein the alkyl, aminoalky, hydroxyalkyl, cycloalkyl, heterocyclyl, aryl, heteroaryl can be further substituted with one or more substituents selected from: H, halogen, hydroxyl, amino, cyano, oxo, carboxyl, Ci-Cs alkyl, Ci-Cs haloalkyl, Ci-Cs alkoxy, C₃-C₈ cycloalkyl, -C(O)Ra, -C(O)ORa, -C(O)NRaRb, ORa, OC(O)Ra, OC(O)NRaRb, SRa, S(O)Ra, S(O)(=NH)Ra, S(O)₂NRaRb, NRaRb, N(Ra)C(O)Rb, N(Ra)C(O)ORb, N(Rc)C(O)NRaRb, -N(Ra)S(O) ₂Rb, -N(Ra)S(O)₂NRbRc;
Ra, Rb and Rc are each independently selected from: H, halogen, alkyl, amino, hydroxyl, cyano, C₃-C₈ cycloalkyl, alkylamino, alkoxy.

2. The compound of claim 1, **characterized in that**:
Y₁ is selected from: N;
Y₂ is selected from: N;
Y₃ is selected from: N;

3. The compound of claim 2, **characterized in that**:
ring A is selected from: 6-10 membered aromatic ring, 5-12 membered heteroaromatic ring, wherein the heteroaromatic ring have 1-3 heteroatoms independently selected from N, O or S, wherein:
ring A is selected from:

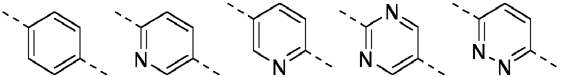

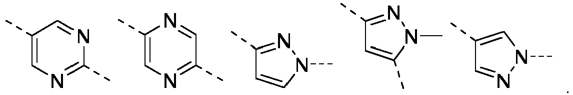

4. The compound of claim 3, **characterized in that**, the compound has a structure shown in the general formula II(a)~II(i): 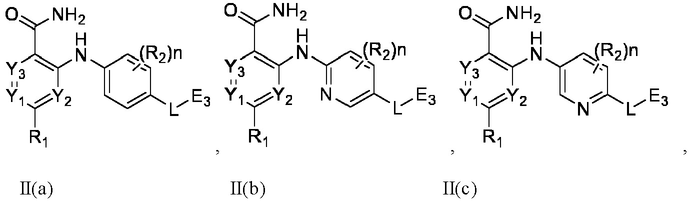 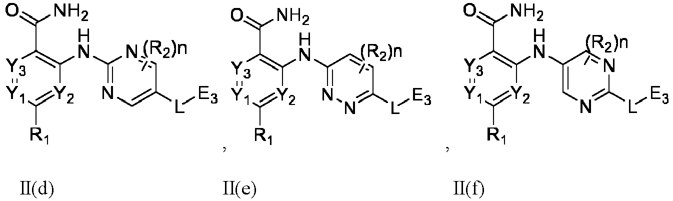 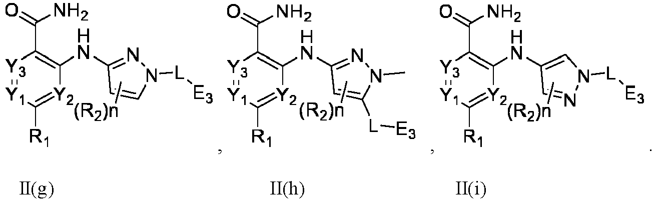 or its stereoisomer thereof, or its stereoisomer mixture thereof, or its pharmaceutically acceptable salt thereof, prodrugs;

5. The compound of claim 4, **characterized in that**, the compound has a structure shown in the general formula III(a)-III(c): 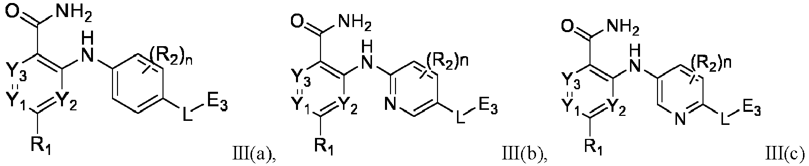
or its stereoisomer thereof, or its stereoisomer mixture thereof, or its pharmaceutically acceptable salt thereof, prodrugs;
R₁ is selected from: NR₆R₇, OR₆, C₁-C₈ alkyl, 3-12 membered heterocyclic ring, 5-6 membered heteroaromatic ring, C₃-C₈ cycloalkyl, aryl, where in the alkyl, heterocyclyl, heteroaromatic ring, cycloalkyl, aryl can be further substituted with one or more substituents selected from Rs;

6. The compound of claim 1-5, **characterized in that**:
L is selected from: 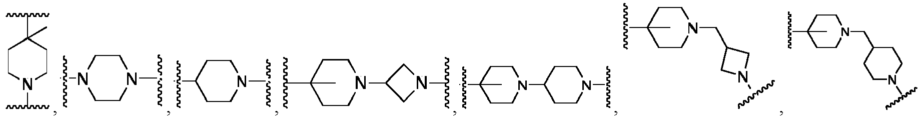 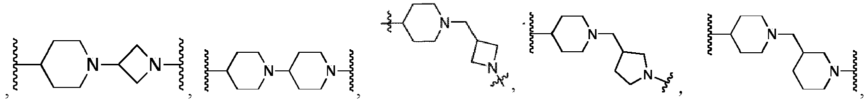 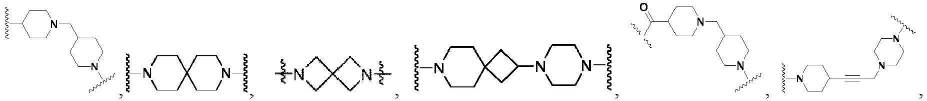 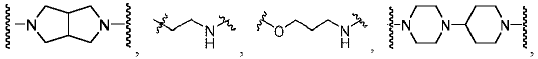 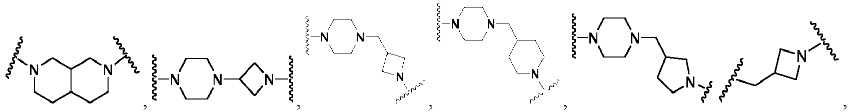 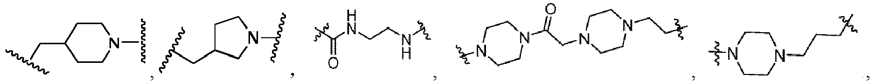 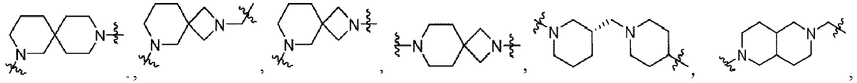 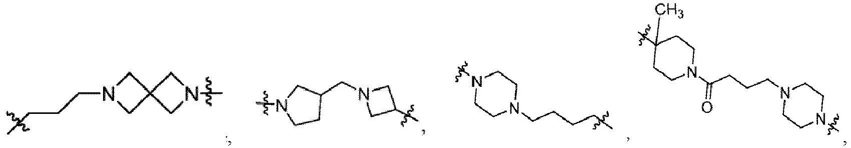 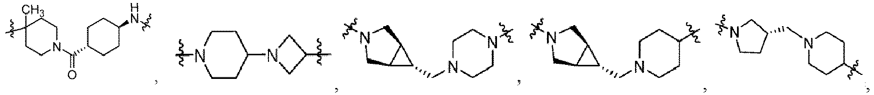 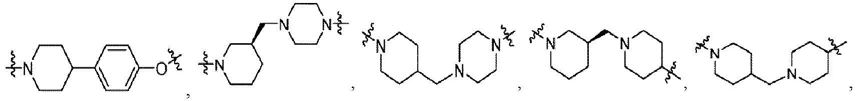 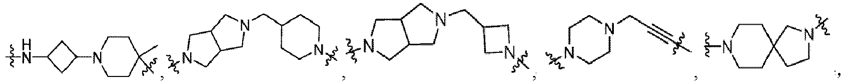 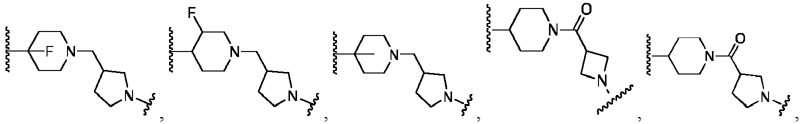 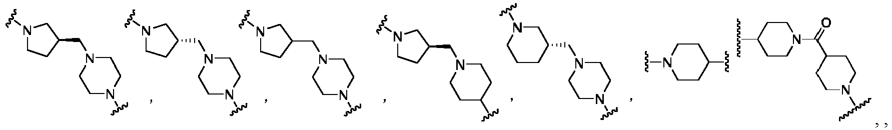 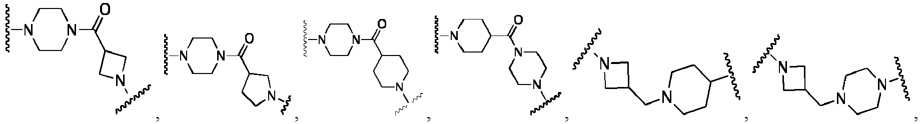 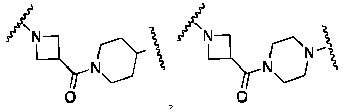
E3 is selected from: 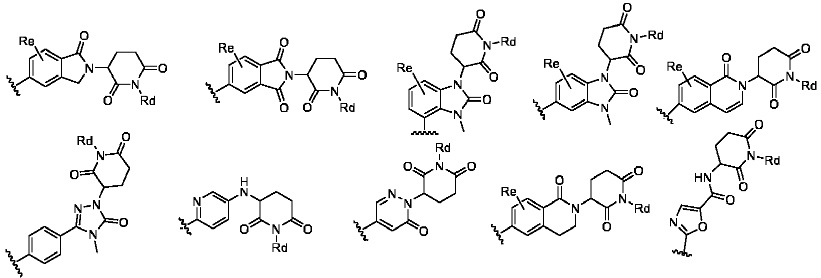 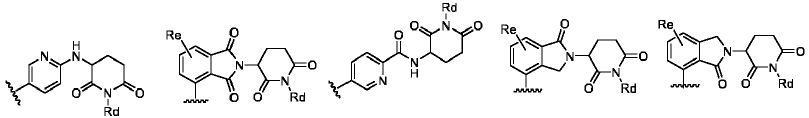 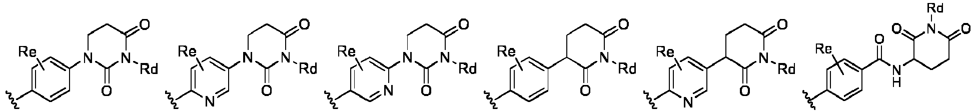 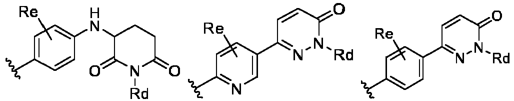
Re is selected from: H, F, Cl, -CH₃, -OMe, -CN, -CF3;
Rd is selected from: H, L₂R_{g};
L₂ is selected from: 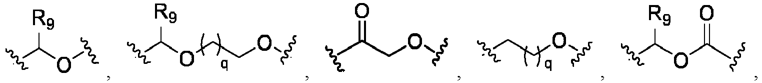 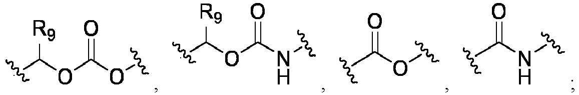
R₉ is selected from: H, C₁-C₅ alkyl;
q is selected from: 1, 2, 3, 4 or 5;
R_{g} is selected from: H, substituted or not substituted C₁-C₈ alkyl; substituents selected from: amino, hydroxyl, cyano, halogen.

7. The compound of claim 1, **characterized in that**, the compound is selected from the following compounds: 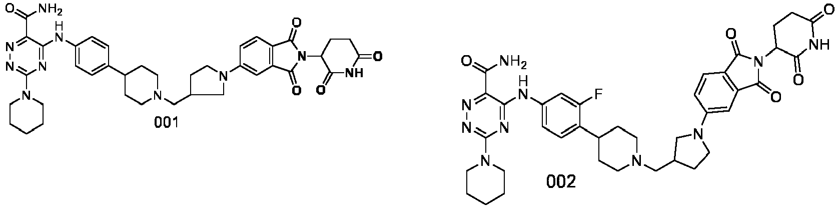 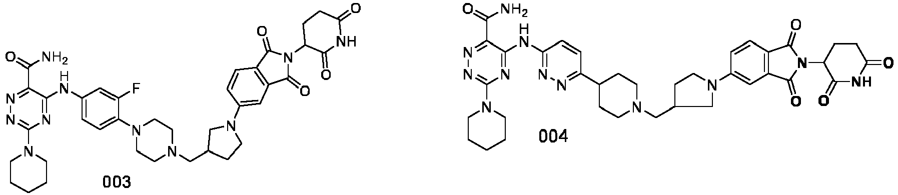 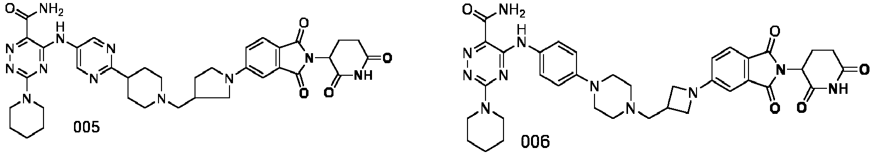 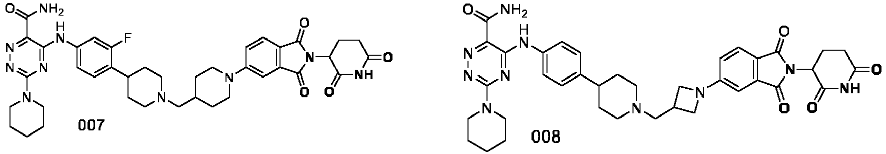 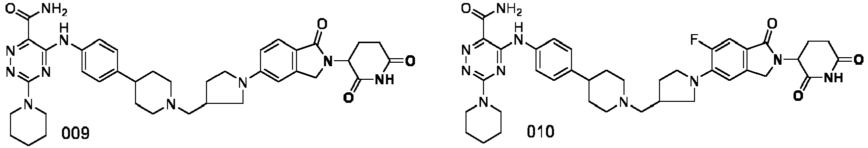 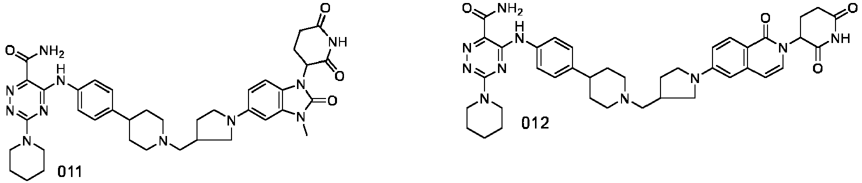 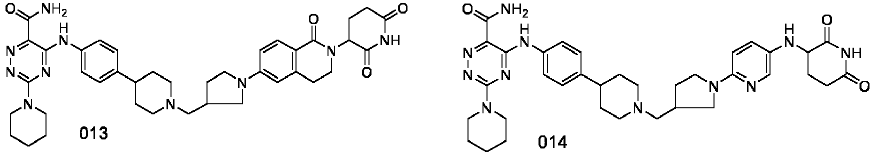 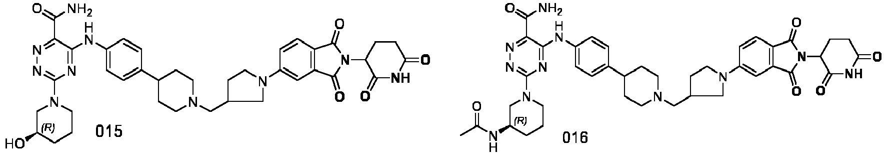 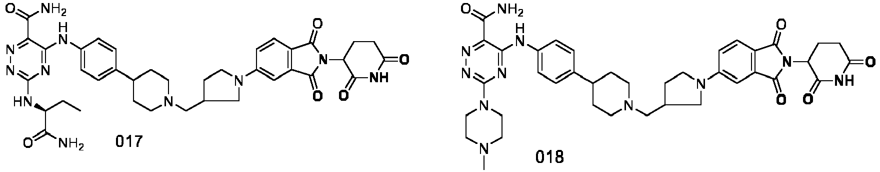 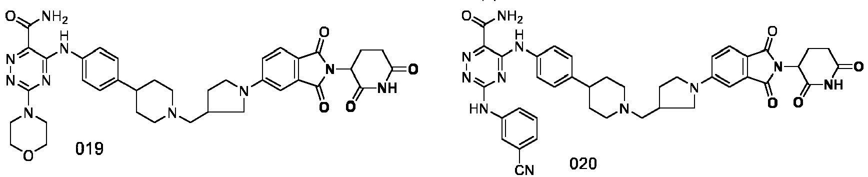 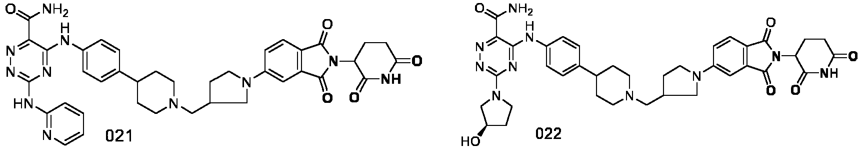 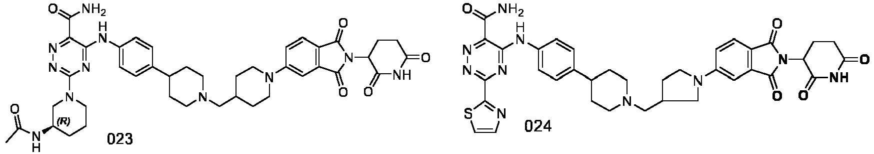 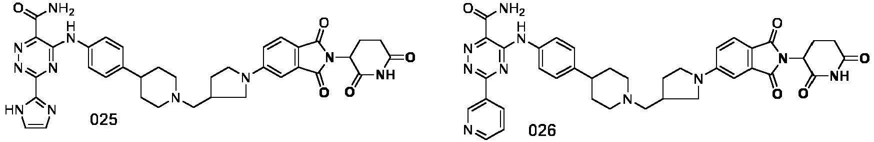 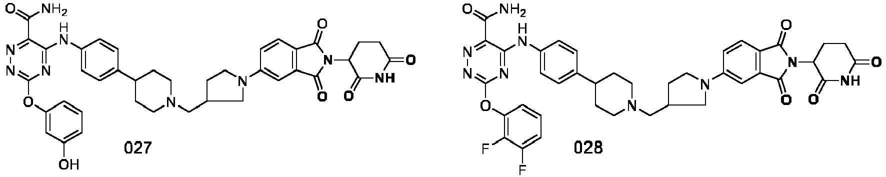 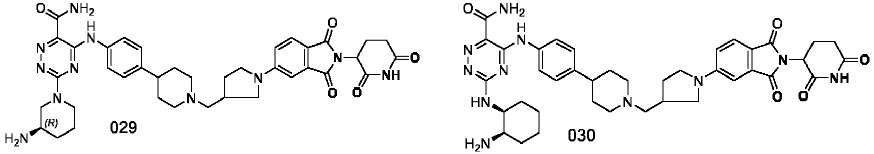 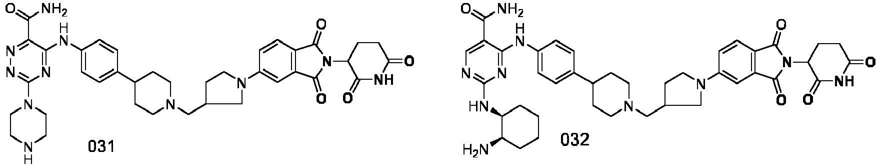 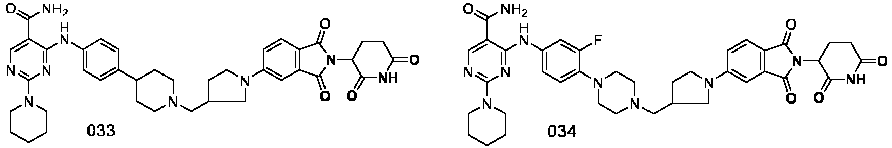 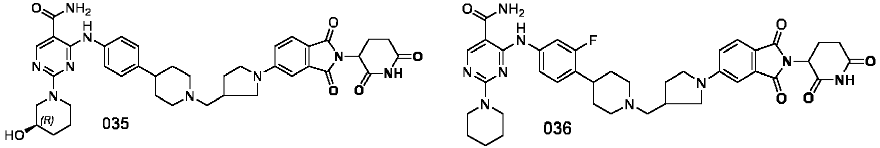 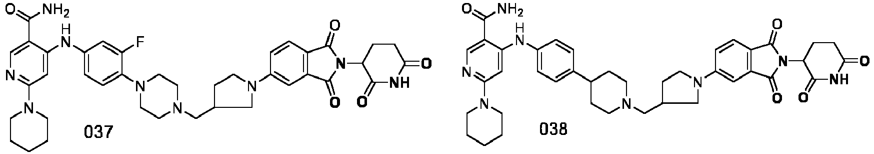 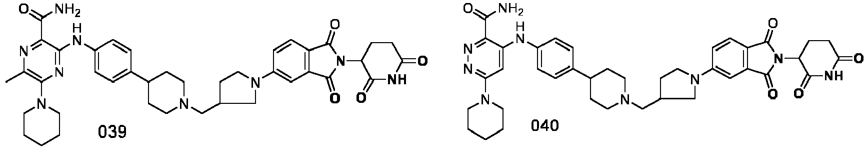 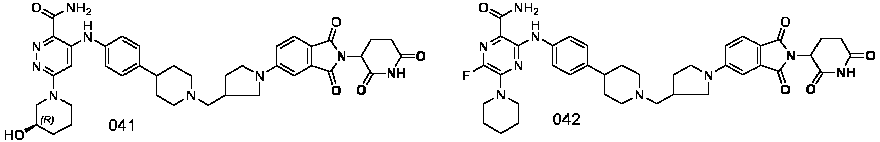 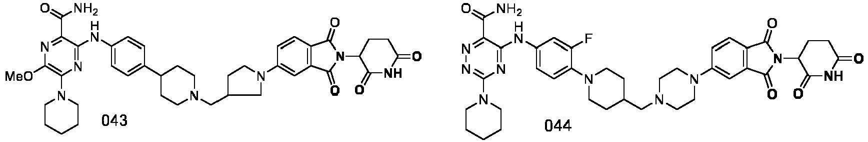 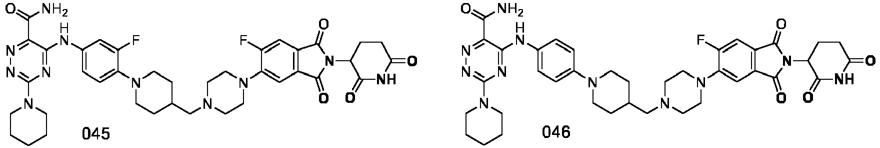 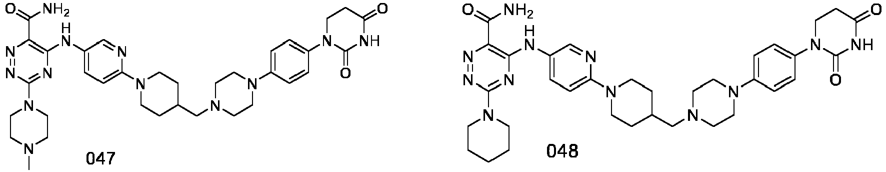 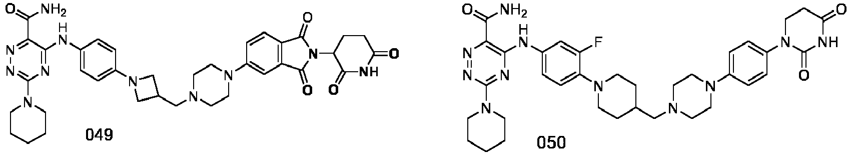 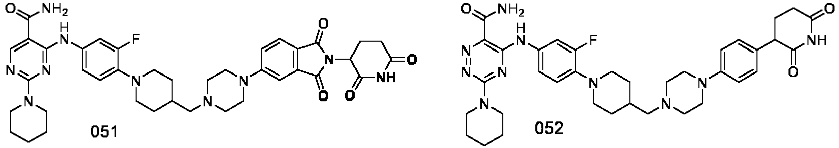 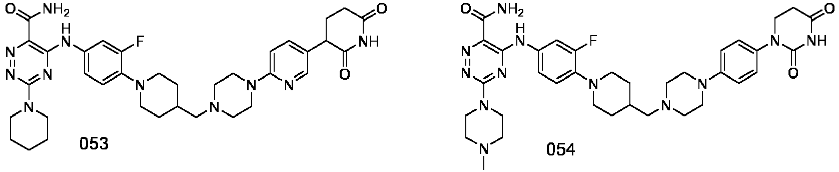 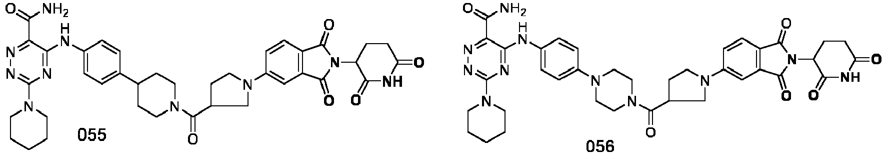 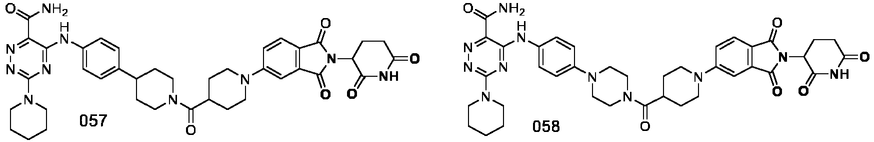 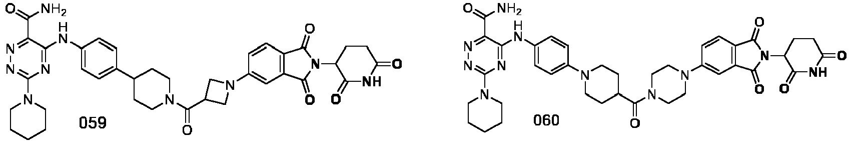 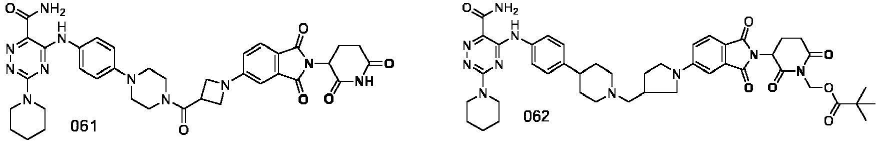 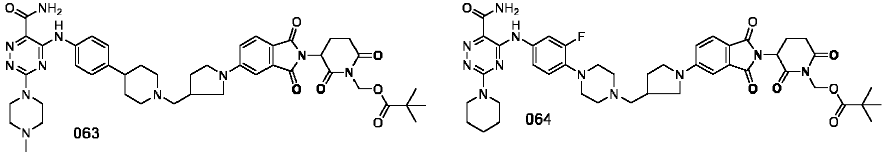 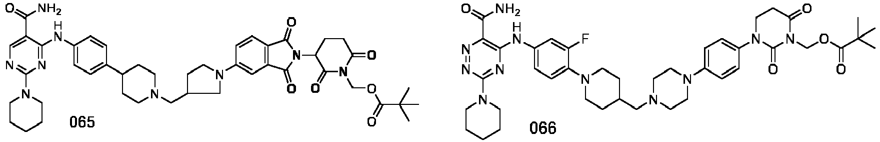 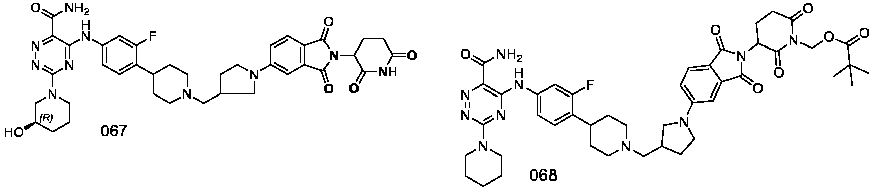 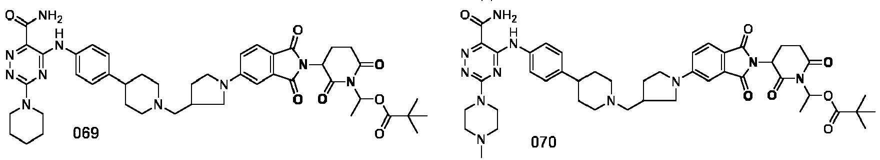 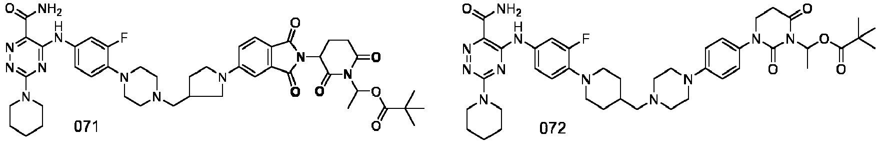 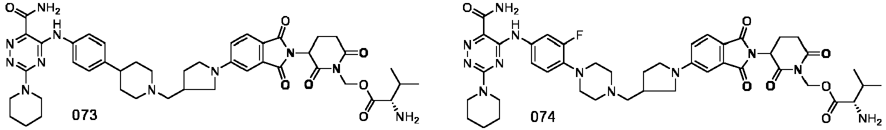 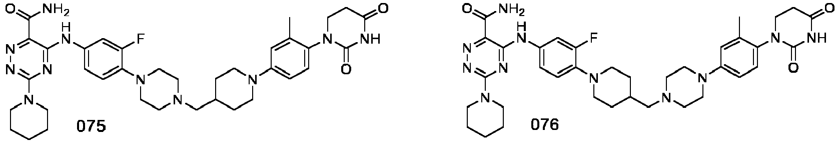 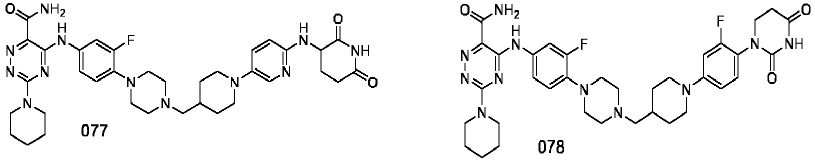 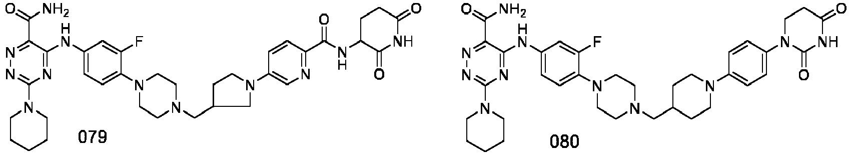 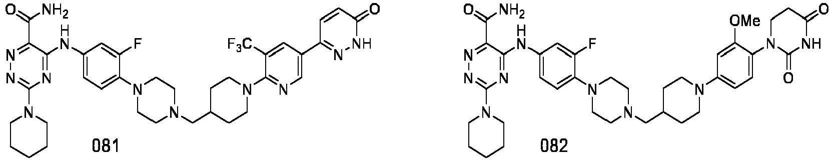 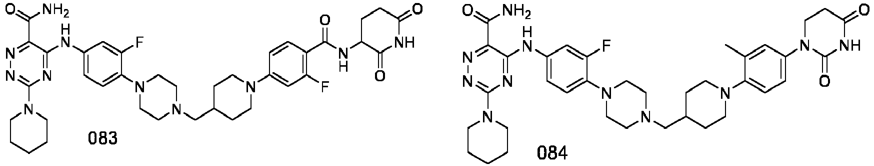 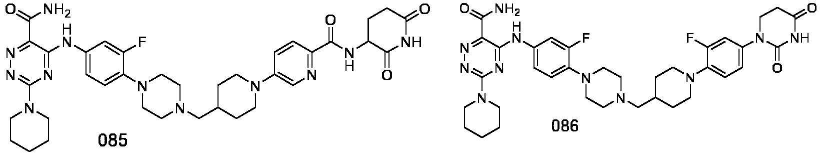 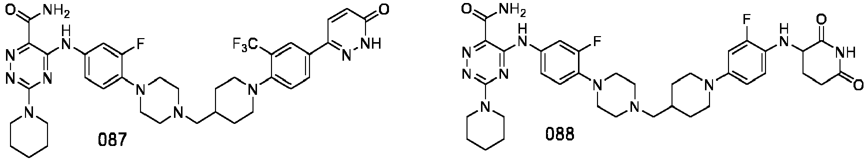 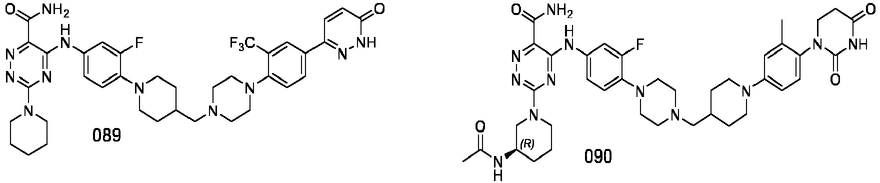 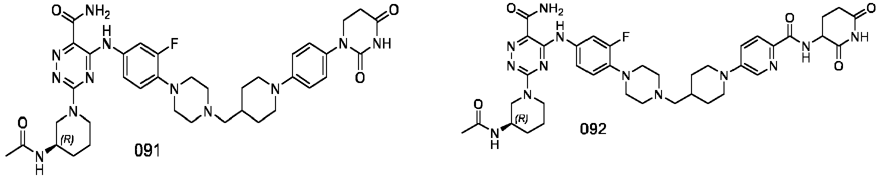 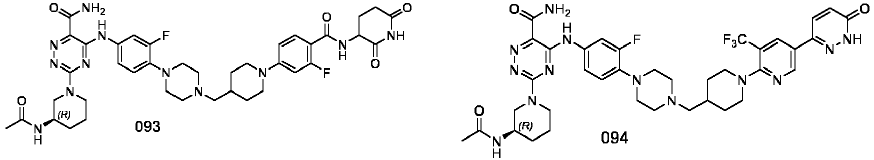 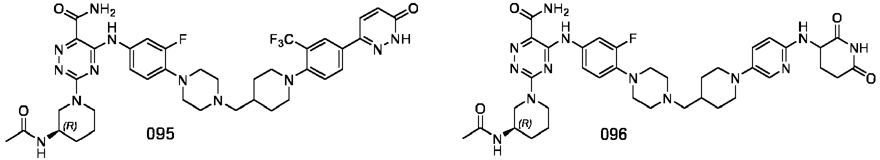 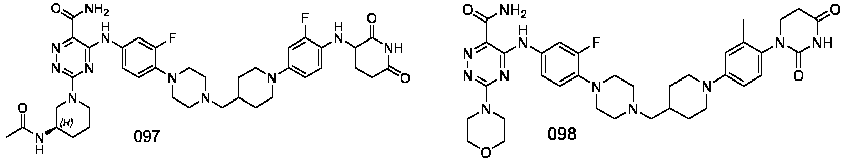 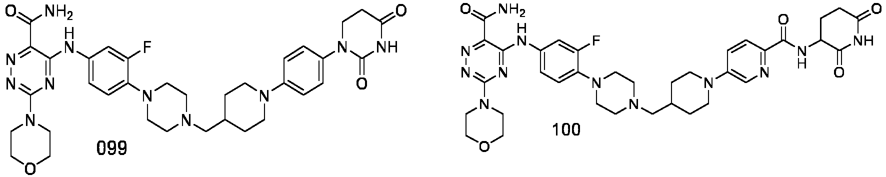 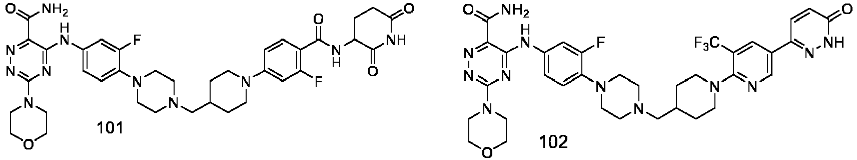 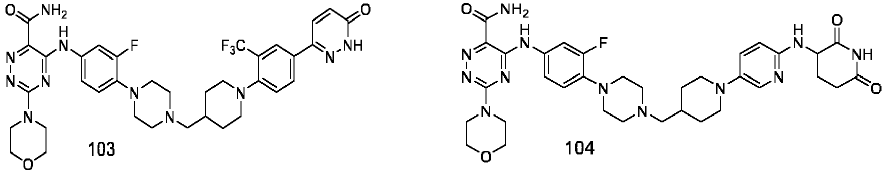 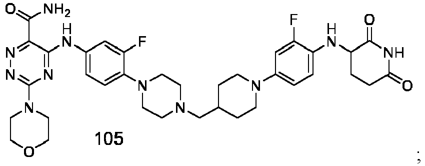 or its stereoisomer thereof, or its stereoisomer mixture thereof, or its pharmaceutically acceptable salt thereof, prodrugs.

8. A pharmaceutical composition, **characterized in that**, the pharmaceutical composition comprises one or more of the compounds of any one of claims 1 to 7.

9. Use of the compound of any one of claims 1 to 7 or claim 8 in the preparation of a drug alone or a drug that is used in combination with other drugs for treating a disease, a disorder or a condition that would benefit from the inhibition or degradation of Bruton's tyrosine kinase activity.

10. The use of claim 9, **characterized in that**, the disease, disorder or condition is that one that would benefit from the inhibition or degradation of Bruton's tyrosine kinase mutation.

11. The use of claim 9, **characterized in that**, the disease is selected from one or more of B cell or plasma cell proliferative diseases, and autoimmune diseases.

12. The use of claim 11, **characterized in that**, the B cell or plasma cell proliferative diseases include diffuse large B cell lymphoma, follicular lymphoma, chronic lymphocytic lymphoma, chronic lymphocytic leukemia, B cell prolymphocytic leukemia, lymphoplasmacytic lymphoma/Waldenström macroglobulinemia, splenic marginal zone lymphoma, plasma cell myeloma, plasmacytoma, extranodal marginal zone B cell lymphoma, lymph node marginal zone B cell lymphoma, mantle cell lymphoma, mediastinum (thymus) large B cell lymphoma, intravascular large B cell lymphoma, primary effusion lymphoma, Burkitt lymphoma/leukemia or lymphomatoid granulomatosis, multiple myeloma.

13. The use of claim 11, **characterized in that**, the autoimmune diseases include inflammatory bowel disease, arthritis, lupus, rheumatoid arthritis, psoriatic arthritis, osteoarthritis, Still's disease, juvenile arthritis, diabetes, myasthenia gravis, Hashimoto's thyroiditis, Ord's thyroiditis, Graves' disease, Sjögren's syndrome, multiple sclerosis, Guillain-Barré syndrome, acute disseminated encephalomyelitis, Addison's disease, optic opsoclonus-myoclonus syndrome, ankylosing spondylitis, antiphospholipid antibody syndrome, aplastic anemia, autoimmune hepatitis, coeliac disease, Goodpasture's syndrome, immune thrombocytopenic purpura, optic neuritis, scleroderma, primary biliary cirrhosis, Reiter's syndrome, Takayasu's arteritis, temporal arteritis, warm autoimmune hemolytic anemia, Wegener's granulomatosis, psoriasis, alopecia universalis, Behçet's disease, chronic fatigue, familial dysautonomia, endometriosis, interstitial cystitis, neuromyotonia, scleroderma or vulvodynia, and chronic graft versus host.
